(19) Europäisches Patentamt
European Patent Office
Office européen des brevets

(11) EP 4 092 024 A1

(12) **EUROPEAN PATENT APPLICATION**
published in accordance with Art. 153(4) EPC

(43) Date of publication:
**23.11.2022 Bulletin 2022/47**

(21) Application number: **21741216.2**

(22) Date of filing: **13.01.2021**

(51) International Patent Classification (IPC):
***C07D 471/04** (2006.01)* ***C07D 471/10** (2006.01)*
***C07D 401/14** (2006.01)* ***A61K 31/506** (2006.01)*
***A61P 35/00** (2006.01)*

(52) Cooperative Patent Classification (CPC):
**A61K 31/506; A61P 35/00; A61P 35/02; A61P 43/00; C07D 401/14; C07D 471/04; C07D 471/10; C07D 491/107; C07D 519/00**

(86) International application number:
**PCT/CN2021/071375**

(87) International publication number:
**WO 2021/143701 (22.07.2021 Gazette 2021/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.01.2020 CN 202010056202**

(71) Applicant: **Beijing InnoCare Pharma Tech Co., Ltd.**
**Beijing 102206 (CN)**

(72) Inventors:
- **CHEN, Xiangyang**
**Beijing 102206 (CN)**
- **GAO, Yingxiang**
**Beijing 102206 (CN)**

(74) Representative: **Patentanwälte Magenbauer & Kollegen**
**Partnerschaft mbB**
**Plochinger Straße 109**
**73730 Esslingen (DE)**

# (54) PYRIMIDINE-4(3H)-KETONE HETEROCYCLIC COMPOUND, PREPARATION METHOD THEREFOR AND USE THEREOF IN MEDICINE AND PHARMACOLOGY

(57) The present invention relates to pyrimidin-4(3*H*)-one heterocyclic compounds for inhibiting or regulating SHP2, preparation methods thereof, and pharmaceutical use thereof. Specifically, the present invention relates to compounds represented by general formula (I) and pharmaceutically acceptable salts thereof, pharmaceutical compositions containing the compounds or pharmaceutically acceptable salts thereof, a method of applying the compounds or pharmaceutically acceptable salts thereof for treating and/or preventing SHP2-mediated diseases, particularly cancers, and preparation methods for the compounds or pharmaceutically acceptable salts thereof. The present invention further relates to use of the compounds or pharmaceutically acceptable salts thereof, or the pharmaceutical compositions containing the compounds or pharmaceutically acceptable salts thereof in the preparation of medicaments for treating and/or preventing SHP2-mediated diseases. Herein, all substituents in the general formula (I) are as defined in the description.

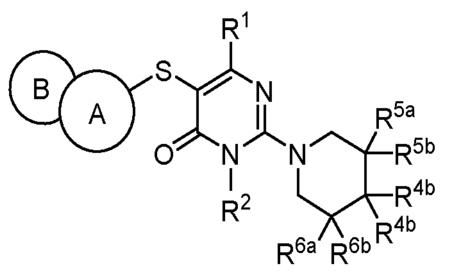

(I)

EP 4 092 024 A1

**Description**

TECHNICAL FIELD

The present invention relates to a novel pyrimidin-4(3*H*)-one heterocyclic compound for inhibiting or regulating SHP2 or pharmaceutically acceptable salt thereof, a pharmaceutical composition containing the compound or pharmaceutically acceptable salt thereof, a preparation method for the compound or pharmaceutically acceptable salt thereof, use of the compound or pharmaceutically acceptable salt thereof, or the pharmaceutical composition containing the compound or the pharmaceutically acceptable salt thereof in the preparation of a medicament for treating and/or preventing SHP2-mediated diseases, particularly cancers, and an administration method thereof.

BACKGROUND ART

Src homology 2 domain-containing phosphatase 2 (SHP2) is a nonreceptor protein tyrosine phosphatase encoded by protein tyrosine phosphatase nonreceptor type 11 (*PTPN11*) gene. SHP2 contains two Src homology (SH2) domains, one protein tyrosine phosphatase (PTP) domain, and one C-terminal tail. Under normal conditions, SHP2 adapts an autoinhibitory conformation, and its N-SH2 binds to PTP to block the substrate channel of the PTP catalytic site, thereby inhibiting PTP activity. When SH2 binds to diphosphotyrosine peptide (such as IRS-1), the autoinhibitory interaction of SH2-PTP is abandoned, and the PTP catalytic site is exposed to enable SHP2 to be in the active state to catalyze the dephosphorylation of tyrosine.

SHP2 is widely expressed, which, as an oncogene, mediates the activation of a variety of oncogenic cell signaling pathways, such as RAS-ERK, PI3K-AKT, and JAK-STAT pathways, and promotes the survival and proliferation of cancer cells. SHP2 can bind and dephosphorylate RAS, increase the association of RAS-RAF, and activate downstream cell proliferation signals. SHP2 also mediates compensatory activation pathways after kinases, such as MEK, are inhibited, thereby leading to drug resistance in tumor therapy (Ruess DA, et al., Nat. Med. 2018, 24, 954-960). Therefore, activation of SHP2 is closely related to the pathogenesis of a variety of diseases, such as leukemia, melanoma, breast cancer, lung cancer, colon cancer, neuroblastoma, and hepatocellular carcinoma.

SHP2 also plays an important role in immune checkpoint pathways of PD-1 and B- and T-lymphocyte attenuator (BTLA), which not only inhibits T cell activation, but also promotes T cell anergy (Li J, et al., Cancer Res. 2015, 75, 508-518).

Therefore, SHP2, as an anti-tumor target, attracts a lot of attention. However, high protein sequence homology of the PTP catalytic site and high hydrophilicity of the PTP catalytic pocket leads to poor selectivity, poor cell permeability, and low bioavaolability of the SHP2 catalytic site. The discovery of Novartis allosteric inhibitor SHP099 (Chen Y, et al., Nature 2016, 535, 148-52) provides a new channel for developing highly specific oral SHP2 inhibitors. At present, some patent applications of SHP2 inhibitors have been disclosed by a plurality of companies, including WO2015107495, WO2016203405, WO2018057884, WO2018013597, and WO2017211303. The present invention designs compounds having a structure represented by general formula (I), and it is found that the compounds with such a structure exhibited excellent SHP2 activity inhibition effect.

SUMMARY

The present invention provides a compound represented by general formula (I) as an SHP2 inhibitor, or prodrugs, stable isotope derivatives, pharmaceutically acceptable salts, and isomers thereof, and mixtures thereof:

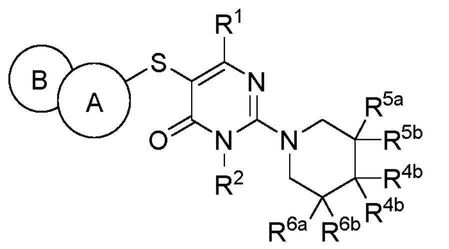

(I)

where:

ring A is selected from the group consisting of phenyl and 6-membered heteroaryl, ring B is selected from the group consisting of a 5-membered heteroaryl ring fused to ring A, where phenyl and the heteroaryl are optionally substituted

with one or more substituents selected from the group consisting of D, halogen, cyano, oxo, $C_{1-6}$ alkyl, $C_{3-6}$ cycloalkyl, 4- to 7-membered heterocyclyl, 5- to 6-membered heteroaryl, $-OR^a$, $-NR^aR^b$, $-C(O)R^a$, $-C(O)NR^aR^b$, $-S(O)_2R^a$, $-S(O)_2NR^aR^b$, $-NR^aS(O)_2R^b$, and $-P(O)(CH_3)_2$, where the alkyl, the cycloalkyl, the heterocyclyl, and the heteroaryl are optionally substituted with one or more substituents selected from the group consisting of D, halogen, cyano, oxo, $C_{1-6}$ alkyl, $C_{3-6}$ cycloalkyl, 4- to 7-membered heterocyclyl, $-OR^a$, $-NR^aR^b$, $-C(O)R^a$, and $-C(O)NR^aR^b$;

$R^1$ is selected from the group consisting of H, D, cyano, $C_{1-2}$ alkyl, cyclopropyl, $-OR^a$, $-NR^aR^b$, and $-C(O)NR^aR^b$, where one or more hydrogen atoms of the alkyl and the cyclopropyl are optionally substituted with one or more substituents selected from the group consisting of D and fluoro;

$R^2$ is selected from the group consisting of H, $C_{1-2}$ alkyl, and cyclopropyl, where one or more hydrogen atoms of the alkyl and cyclopropyl are optionally substituted with one or more substituents selected from the group consisting of D, fluoro, and hydroxyl;

$R^{4a}$ and $R^{4b}$ are each independently selected from the group consisting of H, D, halogen, cyano, $-OR^a$, $-NR^aR^b$, $-C(O)R^a$, $-C(O)NR^aR^b$, $C_{1-6}$ alkyl, $C_{3-6}$ cycloalkyl, 4- to 7-membered heterocyclyl, and 5- to 6-membered heteroaryl, with the proviso that the $R^{4a}$ and the $R^{4b}$ cannot simultaneously be selected from the group consisting of cyano, $-OR^a$, and $-NR^aR^b$, where the alkyl, the cycloalkyl, the heterocyclyl, and the heteroaryl are optionally substituted with one or more substituents selected from the group consisting of D, halogen, cyano, oxo, $C_{1-6}$ alkyl, $C_{3-6}$ cycloalkyl, 4- to 7-membered heterocyclyl, 5- to 6-membered heteroaryl, $-OR^a$, $-NR^aR^b$, $-C(O)R^a$, and $-C(O)NR^aR^b$; the $R^{4a}$ and the $R^{4b}$ optionally together with the carbon atom to which the $R^{4a}$ and the $R^{4b}$ are attached form a $C_{3-7}$ carbocyclic ring or 4- to 8-membered heterocyclic ring;

$R^{5a}$, $R^{5b}$, $R^{6a}$, and $R^{6b}$ are each independently selected from the group consisting of H, D, fluoro, and methyl;

$R^a$ and $R^b$ are each independently selected from the group consisting of H, $C_{1-6}$ alkyl, $C_{3-6}$ cycloalkyl, and 4- to 7-membered heterocyclyl, where the alkyl, the cycloalkyl, and the heterocyclyl are optionally substituted with one or more substituents selected from the group consisting of D, fluoro, cyano, oxo, hydroxyl, $-OCH_3$, and $-NH_2$.

An embodiment of the present invention relates to a compound represented by the foregoing general formula (I) or pharmaceutically acceptable salts, prodrugs, stable isotope derivatives, and isomers thereof, and mixtures thereof, representing a compound represented by general formula (II) or pharmaceutically acceptable salts, prodrugs, stable isotope derivatives, and isomers thereof, and mixtures thereof:

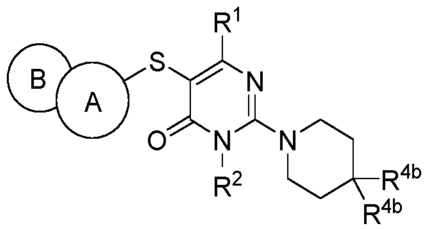

(II)

where:

ring A is selected from the group consisting of phenyl and 6-membered heteroaryl, ring B is selected from the group consisting of a 5-membered heteroaryl ring fused to ring A, where thephenyl and the heteroaryl are optionally substituted with one or more substituents selected from the group consisting of D, halogen, cyano, oxo, $C_{1-6}$ alkyl, $C_{3-6}$ cycloalkyl, 4- to 7-membered heterocyclyl, 5- to 6-membered heteroaryl, $-OR^a$, $-NR^aR^b$, $-C(O)R^a$, $-C(O)NR^aR^b$, $-S(O)_2R^a$, $-S(O)_2NR^aR^b$, and $-NR^aS(O)_2R^b$, where the alkyl, the cycloalkyl, the heterocyclyl, and the heteroaryl are optionally substituted with one or more substituents selected from the group consisting of D, halogen, cyano, oxo, $C_{1-6}$ alkyl, $C_{3-6}$ cycloalkyl, 4- to 7-membered heterocyclyl, $-OR^a$, $-NR^aR^b$, $-C(O)R^a$, and $-C(O)NR^aR^b$;

$R^1$ is selected from the group consisting of H, D, cyano, $C_{1-2}$ alkyl, $-OR^a$, and $-NR^aR^b$, where one or more hydrogen atoms of the alkyl are optionally substituted with one or more substituents selected from the group consisting of D and fluoro;

$R^2$ is selected from the group consisting of H and $C_{1-2}$ alkyl, where one or more hydrogen atoms of the alkyl are optionally substituted with one or more substituents selected from the group consisting of D and fluoro;

$R^{4a}$ and $R^{4b}$ are each independently selected from the group consisting of H, D, halogen, cyano, $-OR^a$, $-NR^aR^b$, $-C(O)NR^aR^b$, $C_{1-6}$ alkyl, $C_{3-6}$ cycloalkyl, 4- to 7-membered heterocyclyl, and 5- to 6-membered heteroaryl, with the proviso that the $R^{4a}$ and the $R^{4b}$ cannot simultaneously be selected from the group consisting of cyano, $-OR^a$, and $-NR^aR^b$, where the alkyl, the cycloalkyl, the heterocyclyl, and the heteroaryl are optionally substituted with one or more substituents selected from the group consisting of D, halogen, cyano, oxo, $C_{1-6}$ alkyl, $C_{3-6}$ cycloalkyl, 4- to 7-membered heterocyclyl, 5- to 6-membered heteroaryl, $-OR^a$, $-NR^aR^b$, $-C(O)R^a$, and $-C(O)NR^aR^b$; the $R^{4a}$ and the $R^{4b}$ optionally together with the carbon atom to which the $R^{4a}$ and the $R^{4b}$ are attached form a $C_{3-7}$ carbocyclic ring or 4- to 7-membered heterocyclic ring;

$R^a$ and $R^b$ are each independently selected from the group consisting of H, $C_{1-6}$ alkyl, $C_{3-6}$ cycloalkyl, and 4- to 7-membered heterocyclyl, where the alkyl, the cycloalkyl, and the heterocyclyl are optionally substituted with one or more substituents selected from the group consisting of D, fluoro, cyano, oxo, hydroxyl, $-OCH_3$, and $-NH_2$.

Another embodiment of the present invention relates to a compound represented by the foregoing general formula (I) or pharmaceutically acceptable salts, prodrugs, stable isotope derivatives, and isomers thereof, and mixtures thereof, representing a compound represented by general formula (III) or pharmaceutically acceptable salts, prodrugs, stable isotope derivatives, and isomers thereof, and mixtures thereof:

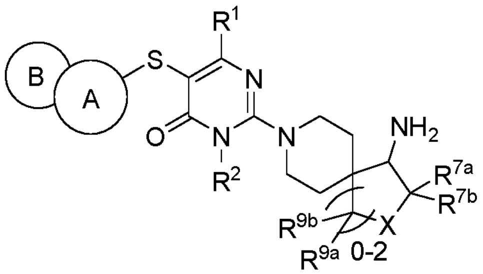

(III)

where:

ring A is selected from the group consisting of phenyl and 6-membered heteroaryl, ring B is selected from the group consisting of a 5-membered heteroaryl ring fused to ring A, where thephenyl and the heteroaryl are optionally substituted with one or more substituents selected from the group consisting of D, halogen, cyano, oxo, $C_{1-6}$ alkyl, $C_{3-6}$ cycloalkyl, 4- to 7-membered heterocyclyl, 5- to 6-membered heteroaryl, $-OR^a$, $-NR^aR^b$, $-C(O)R^a$, and $-C(O)NR^aR^b$, where the alkyl, the cycloalkyl, the heterocyclyl, and the heteroaryl are optionally substituted with one or more substituents selected from the group consisting of D, halogen, cyano, oxo, $C_{1-2}$ alkyl, $-OR^a$, and $-NR^aR^b$;

X is selected from the group consisting of -O- and $-CR^{8a}R^{8b}-$;

$R^1$ is selected from the group consisting of H, D, cyano, $C_{1-2}$ alkyl, and $-NR^aR^b$, where one or more hydrogen atoms of the alkyl are optionally substituted with one or more substituents selected from the group consisting of D and fluoro;

$R^2$ is selected from the group consisting of H and $C_{1-2}$ alkyl, where one or more hydrogen atoms of the alkyl are optionally substituted with one or more substituents selected from the group consisting of D and fluoro;

$R^{7a}$, $R^{7b}$, $R^{8a}$, $R^{8b}$, $R^{9a}$, and $R^{9b}$ are each independently selected from the group consisting of H, D, halogen, cyano, $C_{1-2}$ alkyl, and $-OR^c$, where one or more hydrogen atoms of the alkyl are optionally substituted with one or more substituents selected from the group consisting of D, fluoro, and hydroxyl, with the proviso that the $R^{7a}$ and $R^{7a}$, the $R^{8a}$ and $R^{8b}$, and the $R^{9a}$ and the $R^{9b}$ optionally cannot simultaneously be selected from the group consisting of $-OR^c$;

$R^a$ and $R^b$ are each independently selected from the group consisting of H, $C_{1-6}$ alkyl, $C_{3-6}$ cycloalkyl, and 4- to 7-membered heterocyclyl, where the alkyl, the cycloalkyl, and the heterocyclyl are optionally substituted with one or more substituents selected from the group consisting of D, fluoro, cyano, oxo, hydroxyl, $-OCH_3$, and $-NH_2$;

$R^c$ is selected from the group consisting of H and $C_{1-2}$ alkyl, where one or more hydrogen atoms of the alkyl are optionally substituted with one or more substituents selected from the group consisting of D and fluoro.

Another embodiment of the present invention relates to a compound represented by the foregoing general formula (I) or pharmaceutically acceptable salts, prodrugs, stable isotope derivatives, and isomers thereof, and mixtures thereof, representing a compound represented by general formula (IV) or pharmaceutically acceptable salts, prodrugs, stable isotope derivatives, and isomers thereof, and mixtures thereof:

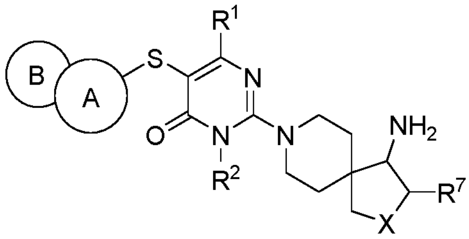

(IV)

where:

ring A is selected from the group consisting of phenyl and 6-membered heteroaryl, ring B is selected from the group consisting of a 5-membered heteroaryl ring fused to ring A, where the phenyl and the heteroaryl are optionally substituted with one or more substituents selected from the group consisting of D, halogen, cyano, oxo, $C_{1-2}$ alkyl, $-OR^a$, and $-NR^aR^b$, where one or more hydrogen atoms of the alkyl are optionally substituted with one or more substituents selected from the group consisting of D and fluoro;
X is selected from the group consisting of -O- and $-CH_2-$;
$R^1$ is independently selected from the group consisting of H, D, $C_{1-2}$ alkyl, and $-NR^aR^b$, where one or more hydrogen atoms of the alkyl are optionally substituted with one or more substituents selected from the group consisting of D and fluoro;
$R^2$ is selected from the group consisting of H and $C_{1-2}$ alkyl, where one or more hydrogen atoms of the alkyl are optionally substituted with one or more substituents selected from the group consisting of D and fluoro;
$R^7$ is selected from the group consisting of H, D, and $C_{1-2}$ alkyl, where one or more hydrogen atoms of the alkyl are optionally substituted with one or more substituents selected from the group consisting of D and fluoro;
$R^a$ and $R^b$ are each independently selected from the group consisting of H and $C_{1-2}$ alkyl, where one or more hydrogen atoms of the alkyl are optionally substituted with one or more substituents selected from the group consisting of D and fluoro.

The present invention further relates to a compound represented by the foregoing general formula (I), where the compound is selected from the group consisting of:

| Compound Number | Compound Structure and Nomenclature |
|---|---|
| 1. | 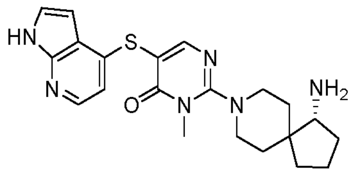 |
| | (*R*)-5-((1H-pyrrolo[2,3-*b*]pyridin-4-yl)thio)-2-(1-amino-8-azaspiro[4.5]decan-8-yl)-3-methylpyrimidin-4(3*H*)-one |
| 2. | 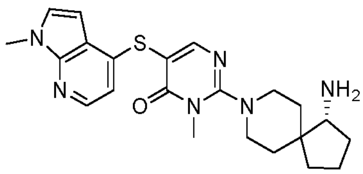 |
| | (*R*)-2-(1-amino-8-azaspiro[4.5]decan-8-yl)-3-methyl-5-((1-methyl-1H-pyrrolo [2,3-*b*]pyridin-4-yl)thio) pyrimidin-4(3*H*)-one |

(continued)

| Compound Number | Compound Structure and Nomenclature |
|---|---|
| 3. | 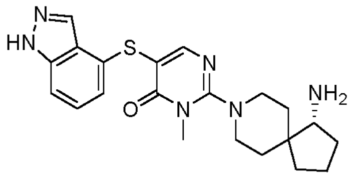 |
| | (*R*)-5-((1H-indazole-4-yl)thio)-2-(1-amino-8-azaspiro [4.5]decan-8-yl)-3-methylpyrimidm-4(3*H*)-one |
| 4. | 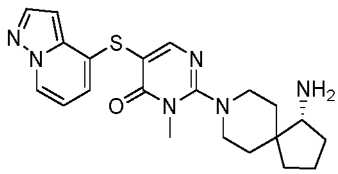 |
| | (*R*)-2-(1-amino-8-azaspiro[4.5]decan-8-yl)-3-methyl-5-(pyrazolo[1,5-*a*]pyridin-4-ylthio)pyrimidin-4 (3*H*)-one |
| 5. | 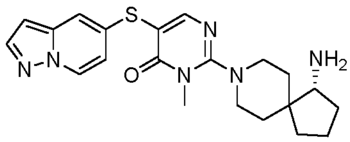 |
| | (*R*)-2-(1-amino-8-azaspiro[4.5]decan-8-yl)-3-methyl-5-(pyrazolo[1,5-*a*]pyridin-5-ylthio)pyrimidin-4 (3*H*)-one |
| 6. | 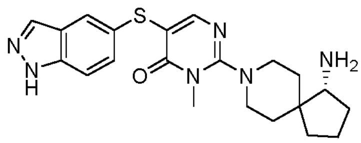 |
| | (*R*)-5-((1H-indazole-5-yl)thio)-2-(1-amino-8-azaspiro [4.5]decan-8-yl)-3-methylpyrimidn-4(3*H*)-one |
| 7. | 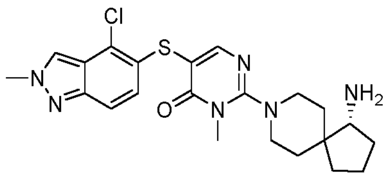 |
| | (*R*)-2-(1-amino-8-azaspiro[4.5]decan-8-yl)-5-((4-chloro-2-methyl-2H-indazole-5-yl)thio)-3-methylpyrimidin-4(3*H*)-one |
| 8. | 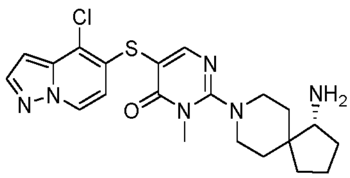 |
| | (*R*)-2-(1-amino-8-azaspiro[4.5]decan-8-yl)-5-((4-chloropyr azolo [1,5-*a*]pyridin-5-yl)thio)-3-methylpyrimidin-4(3*H*)-one |

(continued)

| Compound Number | Compound Structure and Nomenclature |
|---|---|
| 9. | 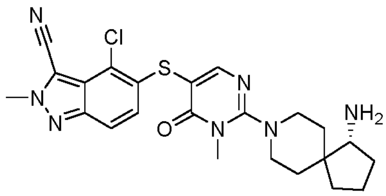 |
| | (*R*)-5-((2-(1-amino-8-azaspiro[4.5]decan-8-yl)-1-methyl-6-oxo-1,6-dihydropyrimidin-5-yl)thio)-4-chloro-2-methyl-2H-indazol e-3-carbonitrile |
| 10. | 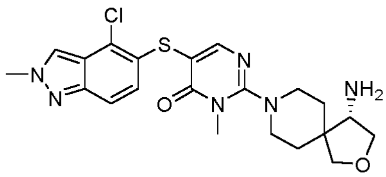 |
| | (*S*)-2-(4-amino-2-oxa-8-azaspiro[4.5]decan-8-yl)-5-((4-chl oro-2-methyl-2H-indazole-5-yl)thio)-3-methylpyrimidin-4(3*H*)-one |
| 11. | 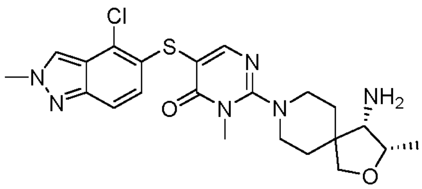 |
| | 2-((3*S*,4*S*)-4-amino-3-methyl-2-oxa-8-azaspiro[4.5]decan-8 -yl)-5-((4-chloro-2-methyl-2H-indazole-5-yl)thio)-3-methylpyrim idin-4(3*H*)-one |
| 12. | 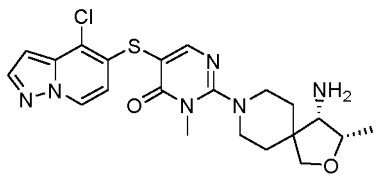 |
| | 2-((3*S*,4*S*)-4-amino-3-methyl-2-oxa-8-azaspiro[4.5]decan-8 -yl)-5-((4-chloropyrazolo[1,5-*a*]pyridin-5-yl)thio)-3-methylpyrim idin-4(3*H*)-one |
| 13. | 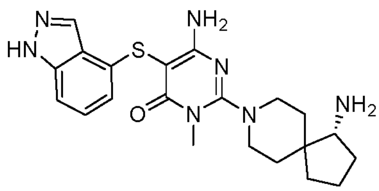 |
| | (*R*)-5-((1H-indazole-4-yl)thio)-6-amino-2-(1-amino-8-azas piro [4.5]decan-8-yl)-3-methylpyrimidin-4 (3*H*)-one |

(continued)

| Compound Number | Compound Structure and Nomenclature |
|---|---|
| 14. | 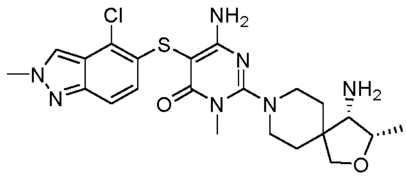 |
| | 6-amino-2-((3*S*,4*S*)-4-amino-3-methyl-2-oxa-8-azaspiro[4. 5]decan-8-yl)-5-((4-chloro-2-methyl-2H-indazole-5-yl)thio)-3-methyl pyrimidin-4(3*H*)-one |
| 15. | 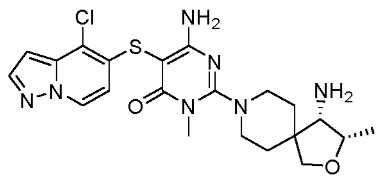 |
| | 6-amino-2-((3*S*,4*S*)-4-amino-3-methyl-2-oxa-8-azaspiro[4. 5]decan-8-yl)-5-((4-chloropyrazolo[1,5-*a*] pyridine-5-yl)thio)-3-methyl pyrimidin-4(3*H*)-one |
| 16. | 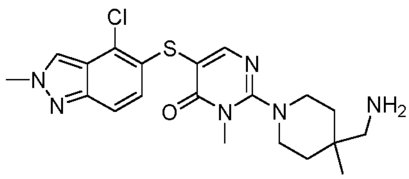 |
| | 2-(4-(ammomethyl)-4-methylpiperidin-1-yl)-5-((4-chloro-2 -methyl-2H-indazole-5-yl)thio)-3-methylpyrimidin-4(3*H*)-one |
| 17. | 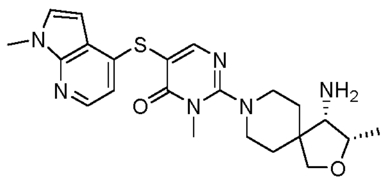 |
| | 2-((3*S*,4*S*)-4-amino-3-methyl-2-oxa-8-azaspiro[4.5]decan-8 -yl)-3-methyl-5-((1-methyl-1H-pyrrolo [2,3-*b*]pyridin-4-yl)thio)pyrimidin -4(3*H*)-one |
| 18. | 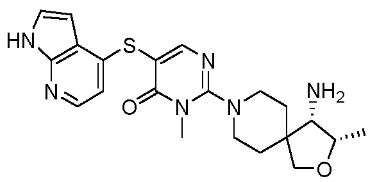 |
| | 5-((1H-pyrrolo[2,3-*b*]pyridin-4-yl)thio)-2-((3*S*,4*S*)-4-amino -3-methyl-2-oxa-8-azaspiro[4.5]decan-8-yl)-3-methylpynmidm-4(3*H*) -one |
| 19. | 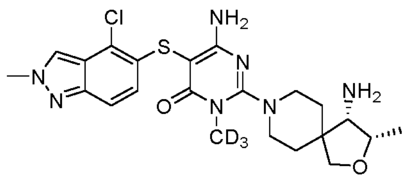 |
| | 2-((3*S*,4*S*)-4-amino-3-methyl-2-oxa-8-azaspiro[4.5]decan-8 -yl)-5-((4-chloro-2-methyl-2H-indazole-5-yl)thio)-3-(methyl-$d_3$ ) pyrimidin-4(3*H*)-one |

or pharmaceutically acceptable salts, prodrugs, stable isotope derivatives, and isomers thereof, and mixtures thereof.

The compound of the present invention can effectively inhibit SHP2 activity, with preferable $IC_{50}$ less than 50 nM. The compound of the present invention has a significant inhibitory effect on the proliferation of NCI-H358 cells, with preferable $IC_{50}$ less than 1,000 nM.

Another aspect of the present invention relates to a pharmaceutical composition, where the pharmaceutical composition comprises the compound represented by general formula (I) or pharmaceutically acceptable salts, prodrugs, stable isotope derivatives, and isomers thereof, and mixtures thereof, and pharmaceutically acceptable carriers and excipients.

The present invention further relates to a pharmaceutical composition, wherein the pharmaceutical composition comprises the compound represented by general formula (I) or pharmaceutically acceptable salts, prodrugs, stable isotope derivatives, and isomers thereof, and mixtures thereof, and at least one additional drug, where the at least one additional drug includes but is not limited to chemotherapeutic agents, targeted drugs, DNA synthesis inhibitors, antibody drugs, antibody-drug conjugates, antitumor drugs, and immunosuppressants.

The present invention further provides use of the compound represented by general formula (I) or pharmaceutically acceptable salts, prodrugs, stable isotope derivatives, and isomers thereof, and mixtures thereof, or the pharmaceutical composition in the preparation of a medicament, where the medicament is indicated for the treatment or prevention of SHP2-medicated diseases, where the diseases include but are not limited to leukemia, Noonan syndrome, leopard syndrome, neuroblastoma, lung cancer, breast cancer, colon cancer, esophageal cancer, gastric cancer, and head and neck cancer.

According to the present invention, the medicament can be in any dosage form, including but not limited to tablets, capsules, solutions, lyophilized preparations, and injectables.

The pharmaceutical formulation of the present invention can be administered in a form of dosage unit containing a predetermined dose of an active pharmaceutical ingredient. Depending on the disease being treated, the administration method, and patient's age, body weight and condition, such a unit may contain 0.5 mg to 1 g, preferably 1 mg to 700 mg, and more preferably 5 mg to 500 mg of a compound of the present invention. In addition, the pharmaceutical formulation can be prepared by methods known in the pharmaceutical field, for example, by mixing the active pharmaceutical ingredient with one or more excipients and/or adjuvants.

The pharmaceutical formulation of the present invention is suitable for administration via any appropriate approaches, including transoral (including oral or sublingual), transrectal, transnasal, topical (including oral, sublingual, or transdermal), vaginal, or parenteral (including subcutaneous, intramuscular, intravenous, or intradermal) approaches.

DETAILED DESCRIPTION OF THE INVENTION

Unless otherwise stated, the following terms in the description and in the claims have the following meanings.

"$C_{x-y}$" refers to a range of the number of carbon atoms, where both x and y are integers, for example, $C_{3-8}$ cycloalkyl stands for cycloalkyl having 3-8 carbon atoms, namely 3, 4, 5, 6, 7, or 8 carbon atoms. It should further be understood that "$C_{3-8}$" further includes any subrange, for example $C_{3-7}$, $C_{3-6}$, $C_{4-7}$, $C_{4-6}$, and $C_{5-6}$.

"Alkyl" refers to a saturated straight- or branched-chain hydrocarbyl group having 1 to 20 carbon atoms, for example, 1 to 8, 1 to 6, or 1 to 4 carbon atoms. Unrestricted examples of alkyl include but are not limited to methyl, ethyl, *n*-propyl, isopropyl, *n*-butyl, isobutyl, tert-butyl, sec-butyl, *n*-amyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1, 1,2-trimethylpropyl, 1,1 -dimethylbutyl, 1,2-dimethylbutyl, 2,2-dimethylbutyl, 1,3-dimethylbutyl, and 2-ethylbutyl.

"Cycloalkyl or carbocyclic ring" refers to a saturated cyclic hydrocarbyl substituent having 3 to 14 carbon ring atoms. Cycloalkyl can be a monocarbocyclic ring, usually containing 3 to 8, 3 to 7, or 3 to 6 carbon atoms. Unrestricted examples of monocyclic cycloalkyl include but are not limited to cyclopropyl, cyclobutyl, cyclopentyl, cyclohexyl, and cycloheptyl. Cycloalkyl can also be a fused bi- or tricarbocyclic ring, for example, decahydronaphthyl, bicyclo[2.2.2]octane, and spiro[3.3]heptane.

"Heterocyclyl or heterocyclic ring" refers to a saturated or partially unsaturated mono- or polycyclic group having 3 to 20 ring atoms, for example, 3 to 14, 3 to 12, 3 to 10, 3 to 8,, 3 to 6, or 5 to 6 ring atoms in which one or more are selected from nitrogen, oxygen, or $S(O)_m$ (where m is an integer from 0 to 2), excluding -O-O-, -O-S-, or -S-S- in the ring structure, and the remaining are carbon atoms. There are preferably 3 to 12, more preferably 3 to 10, further preferably 4 to 7, still further preferably 4 to 6, and most preferably 5 or 6 ring atoms, wherein 1 to 4, more preferably 1 to 3, and most preferably 1 to 2 are heteroatoms. Unrestricted examples of monoheterocyclic group include but are not limited to pyrrolidinyl, oxetanyl, piperidinyl, piperazinyl, tetrahydrofuranyl, tetrahydropyranyl, tetrahydrothiopyranyl, morpholinyl, thiomorpholinyl, homopiperazinyl, and azetidinyl. Polyheterocyclic group includes fused, bridged, or spiro polyheterocycle, for example, octahydrocyclopentadieno[c]pyrrole, octahydropyrrolo[1,2-a]pyrazine, 3,8-diazabicyclo[3.2.1]octane, 5-azaspiro[2.4]heptane, and 2-oxa-7-azaspiro[3.5]nonane.

"Aryl or aryl ring" refers to a monocyclic or fused polycyclic aromatic group having 6 to 14 carbon atoms,

preferably 6- to 10-membered, for example, phenyl and naphthyl, and most preferably phenyl. The aromatic ring can be fused to a heteroaryl, heterocyclic, or cycloalkyl ring, wherein the ring attached to a parent structure is an aryl ring. Unrestricted examples include but are not limited to:

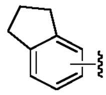 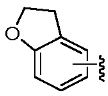 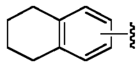 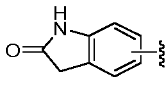 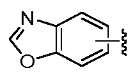 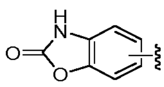 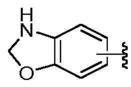

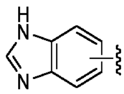 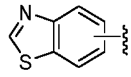 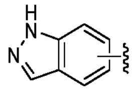 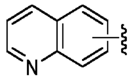 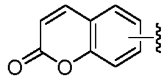 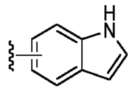 .

"Heteroaryl or heteroaryl ring" refers to a heteroaromatic system having 5 to 14 ring atoms of which 1 to 4 are selected from heteroatoms oxygen, sulphur, and nitrogen. Heteroaryl preferably is 5- to 10-membered, and more preferably, 5- or 6-membered, for example, furyl, thienyl, pyridyl, pyrrolyl, pyrimidinyl, pyrazinyl, pyrazolyl, imidazolyl, tetrazolyl, oxazolyl, isoxazolyl, thiazolyl, isothiazolyl, quinolinyl, isoquinolinyl, indolyl, and isoindolyl. The heteroaryl ring can be fused with an aryl, heterocyclic or cycloalkyl ring, where the ring attached to a parent structure is the heteroaryl ring. Unrestricted examples include but are not limited to:

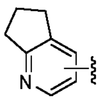 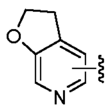 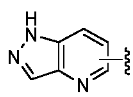 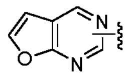 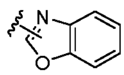 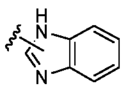

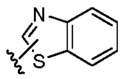 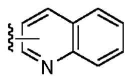 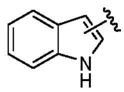 .

"Halogen" refers to F, Cl, Br, or I.

"Cyano" refers to -CN.

"Oxo" refers to =O.

"Optionally" means that an event or environment described hereinafter may but may not have to occur, and includes cases in which the event or environment occurs or does not. For example, "heterocyclic group optionally substituted with alkyl" means that the alkyl may be but is not necessarily present, and this description includes cases in which the heterocyclic group is substituted with the alkyl and cases in which the heterocyclic group is not substituted with the alkyl.

"Substituted" refers to that one or more hydrogen atoms in a group, preferably 5, and more preferably 1 to 3 are independently substituted with a corresponding number of substituents. It goes without saying that the substituents are located only in possible chemical positions thereof, and those skilled in the art can determine possible or impossible substitution without making much efforts (theoretically or experimentally). For example, amino or hydroxyl with free hydrogen may be unstable when bound to carbon atoms having unsaturated bonds (eg, olefinic). The substituents include but are not limited to halogen, cyano, nitro, oxo, $-SF_5$, $C_{1-4}$ alkyl, $C_{3-7}$ cycloalkyl, 4- to 7-membered heterocyclyl, phenyl, and 5-to 6-membered heteroaryl.

"Isomer" refers to a compound having the same molecular formula but different binding properties or orders thereof or different spatial arrangements of atoms thereof. An isomer having different spatial arrangement of atoms is called "stereoisomer". Stereoisomers include optical isomer, geometric isomer, and conformer.

The compounds of the present invention can exist in form of optical isomers. The optical isomers include enantiomers and diastereomers. An enantiomer is one of two stereoisomers that are mirror images of each other but non-superimposable. A racemic mixture or racemate is one that has equal amounts of left- and right-handed enantiomers of a chiral molecule. Diastereomers are stereoisomers that are not mirror images of one another and non-superimposable on one another. When a compound is a single isomer and has a determined absolute configuration, it is referred as a "R" or "S" isomer according to the configuration of substituents on the chiral carbon atom; when its absolute configuration is not determined, it is referred as a (+) or (-) isomer according to the measured optical rotation value. Methods for preparing and separating optical isomers are known in the art.

The compounds of the present invention can further exist in form of geometric isomers resulting from distribution

of substituents around carbon-carbon double bonds, carbon-nitrogen double bonds, cycloalkyls, or heterocyclic groups. The substituents around the carbon-carbon double bonds or carbon-nitrogen double bonds are designated to be in a Z- or E-configuraton, and those around the cycloalkyls or heterocyclic rings are designated to be in a *cis* or *trans* configuration.

The compounds of the present invention can further show tautomerism, for example, keto-enol tautomerism.

It should be noted that the present invention includes any tautomers or stereoisomers and a mixture thereof and is not merely limited to any tautomeric or stereoisomeric forms used in the compound nomenclature or chemical structural formula.

"Isotopes" include all isotopes of atoms present in the compounds of the present invention. The isotopes include those atoms having the same atomic numbers but in different mass. Examples of isotopes suitable to be incorporated into the compounds of the present invention may be hydrogen, carbon, nitrogen, oxygen, phosphorus, sulphur, fluorine, and chlorine, for example but not limited to $^{2}H$ (D), $^{3}H$, $^{13}C$, $^{14}C$, $^{15}N$, $^{17}O$, $^{18}O$, $^{31}P$, $^{32}P$, $^{35}S$, $^{18}F$, and $^{36}Cl$, respectively. Isotopically labeled compounds in the present invention may generally be prepared by conventional techniques known to those skilled in the art or by methods similar to those described in the embodiments using appropriate isotopically labeled reagents instead of non-isotopically labeled reagents. Such compounds have various potential use, for example, as standards and reagents for determining biological activities. In the case of stable isotopes, such compounds have a potential for beneficially altering biological, pharmacological, or pharmacokinetic properties. Deuterium (D) may be a preferrable isotope in the present invention. For example, hydrogen in methyl, methylene, or methine may be substituted with deuterium.

The compounds of the present invention may be administered in form of prodrug. "Prodrugs" refer to derivatives that can be converted to biologically active compounds under *in vivo* physiological conditions, such as oxidation, reduction, and hydrolysis (each of which can occur in the absence or presence of an enzyme). Examples of prodrugs may be compounds in the present invention in which an amino is acylated, alkylated, or phosphorylated, for example, eicosanoylamino, alanylamino, and pivaloyloxymethylamino; or a hydroxyl is acylated, alkylated, phosphorylated, or converted into boronate salt, for example, acetoxy, palmitoyloxy, pivaloyloxy, succinyloxy, fumaryloxy, and alanyloxy; a carboxyl is esterified or amidated; a thiol form a disulfide bridge with a carrier molecule, such as peptide, that selectively delivers the drug to a target and/or cytosol in a cell. Prodrugs may be prepared from the compounds of the present invention according to well-known methods.

"Pharmaceutical salts" or "pharmaceutically acceptable salts" refer to salts prepared from pharmaceutically acceptable bases or acids, including inorganic bases or acids and organic bases or acids. The present invention further includes the pharmaceutically acceptable salts of the compounds of the present invention that contain one or more acidic or basic groups. Therefore, the compounds of the present invention having acidic groups may exist in form of salt and are used according to the present invention, for example, serving as alkali metal salts, alkaline earth metal salts, or ammonium salts. More specific examples of these salts may include sodium salts, potassium salts, calcium salts, magnesium salts, or salts formed with ammonia or organic amines, for example, ethylamine, ethanolamine, triethanolamine or amino acids. The compounds of the present invention having basic groups may exist in form of salt and are used according to the present invention as salts made with inorganic or organic acids. Examples of suitable acids may include hydrochloric acid, hydrobromic acid, phosphoric acid, sulfuric acid, nitric acid, methanesulfonic acid, *p*-toluenesulfonic acid, naphthalene disulfonic acid, oxalic acid, acetic acid, tartaric acid, lactic acid, salicylic acid, benzoic acid, formic acid, propionic acid, trimethylacetic acid, propanedioic acid, succinic acid, pimelic acid, fumaric acid, maleic acid, malic acid, sulfamic acid, phenylpropionic acid, gluconic acid, ascorbic acid, isonicotinic acid, citric acid, adipic acid, and other acids known to those skilled in the art. On the condition that the compounds of the present invention contain both acidic and basic groups in the molecule, the present invention further includes internal salts or betains saltsin addition to the mentioned salt forms. Each salt may be obtained by conventional methods known to those skilled in the art, for example, by mixing a compound with an organic or inorganic acid or base in a solvent or dispersant, or by anion or cation exchange with another salt.

"Pharmaceutical composition" refers to a composition containing one or more compounds described herein or pharmaceutically acceptable salts, prodrugs, stable isotope derivatives, and isomers thereof, and mixtures thereof, and other components, such as pharmaceutically acceptable carriers and excipients. The pharmaceutical composition is intended for easy administration to bodies and better absorption of active pharmaceutical ingredients to exert biological activity.

Therefore, when "compound(s)", "compound(s) of the present invention", or "the compound(s) of the present invention" are mentioned in this application, all compound forms are included, such as pharmaceutically acceptable salts, prodrugs, stable isotope derivatives, and isomers thereof, and mixtures thereof.

Herein, the term "therapeutically effective dosage" refers to a dosage of the compound of the present invention capable of effectively inhibiting the function of SHP2 and/or treating or preventing the diseases.

Herein, the term "patients" refer to mammals, particularly human.

Synthesis methods

The present invention further provides a method for preparing the compounds. The compounds represented by general formula (I) of the present invention may be prepared by the following exemplary methods and examples, but these methods and examples should not be construed as limiting the scope of the present invention in any way. The compounds of the present invention may further be synthesized by synthesis techniques known to those skilled in the art or by using a combination of methods known in the art and the methods of the present invention. Products obtained from each step of reaction may be obtained by separation techniques known in the art, including but not limited to extraction, filtration, distillation, crystallization, and chromatographic separation. Starting materials and chemical reagents used for synthesis may be conventionally synthesized according to the literature (inquired from SciFinder) or purchased.

The heterocyclic compound represented by general formula (I) of the present invention may be synthesized according to a route shown in method A: 1) A1 and NH from the piperidine compound A2 undergo a substitution reaction by base catalysis or a condensation reaction in the presence of a condensing agent to give intermediate A3; 2) A3 and a sulfydryl from a 5,6-fused heteroaryl undergo Buchwald-Hartwig cross coupling reaction to produce product A4.

Method A:

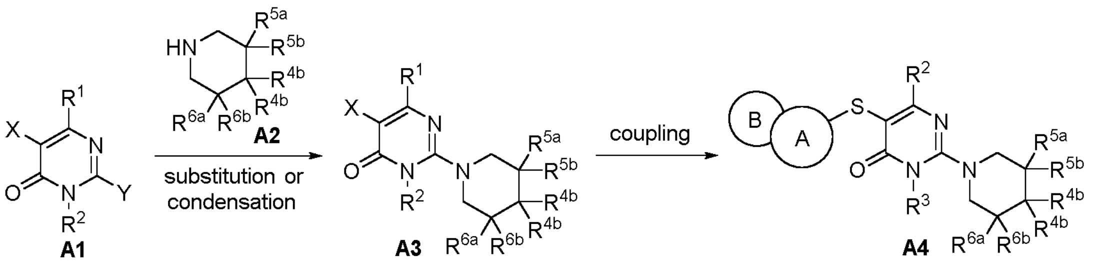


The heterocyclic compounds shown in general formula (I) of the present invention can also be synthesized according to the route shown in method B: 1) A1 and A2a undergo a substitution reaction by base catalysis or a condensation reaction in the presence of a condensing agent to give intermediate A3a; 2) keto of the A3a and (*R*)-2-methylpropane-2-sulfinamide undergo a reductive amination to give A5a; 3) the A5a and a sulfydryl from a 5,6-fused heteroaryl undergo Buchwald-Hartwig cross coupling reaction to produce A6a; 4) the A6a is deprotected in an acidic condition to obtain a product A4a.

Method B

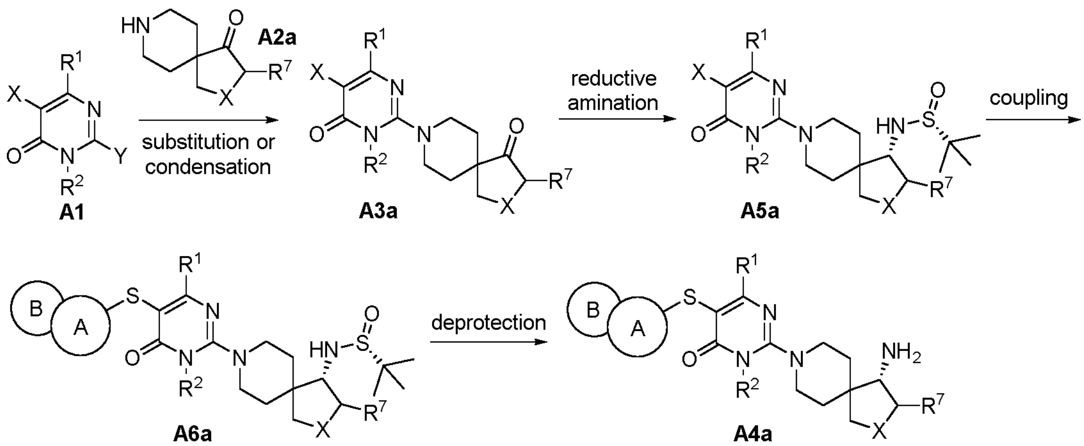

Example

The starting materials of the present invention were synthesized according to methods known in the art or purchased from chemical companies such as ABCR GmbH&Co. KG, Acros Organics, Aldrich Chemical Company, Accela ChemBio Inc., and Beijing Ouhe Technology Co., Ltd.

Structures of compounds were determined by nuclear magnetic resonance (NMR) or mass spectrometry (MS). NMR determination used a Bruker Ascend 400 MHz NMR spectrometer, the solvents were deuterated dimethyl sulfoxide (DMSO-$d_6$), deuterated chloroform ($CDC1_3$), or deuterated methanol ($CD_3OD$), etc, the internal standard was tetramethylsilane (TMS), and the chemical shifts were given in a unit of $10^{-6}$ (ppm). MS determination used an Agilent SQD (ESI) mass spectrometer (Agilent 6120).

High performance liquid chromatography (HPLC) determination used an Agilent 1260 DAD High Performance Liquid Chromatograph (Poroshell120 EC-C18, 50 × 3.0 mm, 2.7 μm column) or Waters Arc High Performance Liquid Chromatograph (Sunfire C18, 150 × 4.6 mm, 5 μm column).

Unless otherwise stated in the embodiments, reactions were carried out at room temperature (20 to 30°C).

Unless otherwise stated in the embodiments, reactions were carried out under an argon or nitrogen atmosphere. An argon or nitrogen atmosphere refers to that a reaction bottle is connected to an argon or nitrogen balloon with a volume of about 1 L.

A hydrogen atmosphere refers to that a reaction bottle is connected to a hydrogen balloon with a volume of about 1 L after being evacuated and filled with hydrogen (repeated three times).

Microwave reaction used a CEM Discover-SP Microwave Reactor.

Reaction processes in examples were monitored by using either Agilent LC/MS System (1260/6120) or thin-layer chromatography (TLC) in which silica gel plates were 0.15 to 0.2 mm in thickness (GF254, Qingdao Haiyang Chemical Co., Ltd.).

Compounds were purified by column chromatography or TLC, where 200- to 300-mesh silica gel (Qingdao Haiyang Chemical Co., Ltd.) was used in column chromatography, and silica gel GF254 plates (Qingdao Haiyang Chemical Co., Ltd.) with a thickness of 0.4 to 0.5 mm were used for TLC.

Eluent solvent systems for column chromatography or TLC usually included: a) dichloromethane-methanol system, b) petroleum ether-ethyl acetate system, or those systems shown in examples. The volume ratio of solvents was adjusted according to different polarities of compounds or further adjusted by addition of a small amount of triethylamine or other acidic or basic reagents.

Compounds were alternatively purified by Waters MS-guided Automated Preparation System (mass spectrometer detector: SQD2) with a reversed-phase column (XBridge-C18, 19 × 150 mm, 5 μm) eluting gradiently at a flow rate of 20 mL/min in an appropriate acetonitrile/water (containing 0.1% trifluoroacetic acid or formic acid, or 0.05% aqueous ammonia) according to the polarities of compounds. In some examples, 1 N diluted hydrochloric acid was added after purification by the automated preparation system, followed by solvent removal under reduced pressure to give hydrochloride salts.

The abbreviation DMF refers to *N*,*N*-dimethylformamide.

The abbreviation DIPEA refers to *N*,*N*-diisopropylethylamine.

The abbreviation DBU refers to 1,8-diazabicyclo[5.4.0]undec-7-ene.

The abbreviation NBS refers to *N*-bromosuccinimide.

The abbreviation NIS refers to *N*-iodosuccinimide.

The abbreviation XantPhos refers to 9,9-dimethyl-4,5-bis(diphenylphosphino)xanthene.

The abbreviation $Pd_2(dba)_3$ refers to tris(dibenzylideneacetone)dipalladium.

Castros reagent refers to benzotriazol-1-yloxytris(dimethylamino)phosphonium hexafluorophosphate.

Example 1

(*R*)-5-((1H-pyrrolo[2,3-*b*]pyridin-4-yl)thio)-2-(1-amino-8-azaspiro[4. 5]decan-8-yl)-3-methylpyrimidin-4(3*H*)-one hydrochloride

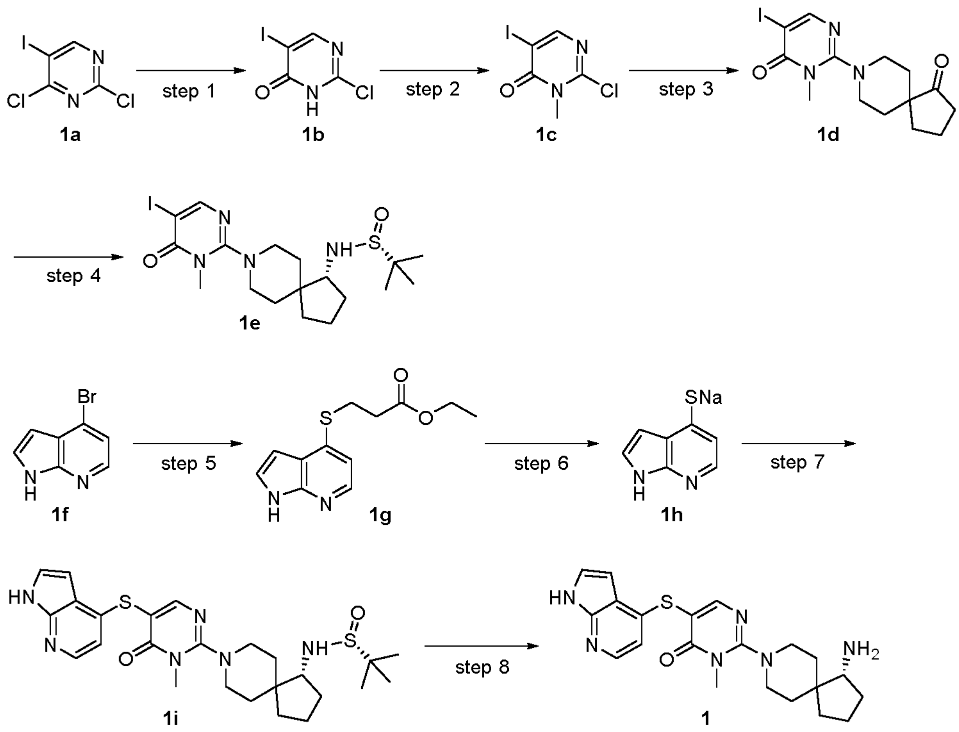

1a 1b 1c 1d 1e 1f 1g 1h 1i 1

Step 1

2-Chloro-5-iodopyrimidin-4(3*H*)-one (1b)

2,4-Dichloro-5-iodopyrimidine 1a (8.0 g, 29.1 mmol) was dissolved in tetrahydrofuran (THF) (100 mL), and sodium hydroxide solution (1 N, 45 mL) was added at 0°C; the reaction mixture was warmed up to room temperature and stirred for 16 h. The pH value was adjusted with citric acid to acidic, and the reaction mixture was extracted with ethyl acetate (60 mL × 3). The organic phases were combined, washed with saturated brine (30 mL × 3), dried over anhydrous sodium sulfate, and desolventized under reduced pressure to give target product 1b (6.7 g, solid), with a yield of 91%.

MS m/z (ESI): 257 [M+1]

Step 2

2-Chloro-5-iodo-3-methylpyrimidin-4(3*H*)-one (1c)

1b (5.6 g, 21.7 mmol) was dissolved in THF (100 mL), added with iodomethane (3.7 g, 26 mmol) and DIPEA (8.4 g, 65.1 mmol), heated to 60°C, and stirred for 16 h. The resulting mixture was poured into water (120 mL) and extracted with ethyl acetate (80 mL × 3). The organic phases were combined, washed with saturated brine (30 mL × 3), dried over anhydrous sodium sulfate, filtered, and concentrated under reduced pressure. The residue was purified by silica gel column chromatography (ethyl acetate/petroleum ether = 3/7) to give target product 1c (2.2 g, solid), with a yield of 37%.

MS m/z (ESI): 271 [M+1]

Step 3

8-(5-Iodo-1-methyl-6-oxo-1,6-dihydropyrimidin-2-yl)-8-azaspiro[4.5] decan-1-one (1d)

8-Azaspiro[4.5]decan-1-one (hydrochloride, 586 mg, 3.1 mmol) and potassium carbonate (1.51 g, 11 mmol) were added into a solution of 1c (1.0 g, 3.65 mmol) in acetonitrile (25 mL), and the reaction mixture was heated to 80°C and stirred for 16 h. After cooling to room temperature, the reaction mixture was poured into water (50 mL) and extracted with ethyl acetate (30 mL × 3). The organic phases were combined, washed with saturated brine (20 mL × 3), dried over anhydrous sodium sulfate, filtered, and concentrated under reduced pressure. The residue was purified by silica

gel column chromatography (ethyl acetate/petroleum ether = 7/3) to give target product 1d (1.2 g, solid), with a yield of 85%.
MS m/z (ESI): 388 [M+1]

Step 4

(*R*)-N-((R)-8-(5-iodo-1-methyl-6-oxo-1,6-dihydropyrimidin-2-yl)-8-a zaspiro[4.5]decan-1 -yl)-2-methylpropane-2-sulfinamide (1e)

(*R*)-2-methylpropane-2-sulfinamide (750 mg, 6.2 mmol) and tetraethyl titanate (2.82 g, 12.4 mmol) were added into a solution of 1d (1.2 g, 3.1 mmol) in THF (30 mL), and the reaction mixture was heated to 90°C and stirred for 16 h. After cooling to 0°C, the mixture was added with methanol (10 mL) and lithium borohydride in THF (2.0 M, 1.5 mL, 3 mmol) and stirred for 1 h. The reaction was quenched with an ammonium chloride solution, and the resulting mixture was filtered. The filtrate was extracted with ethyl acetate (25 mL × 3); the organic phases were combined, washed with saturated brine (20 mL × 3), dried over anhydrous sodium sulfate, filtered, and concentrated under reduced pressure. The residue was purified by silica gel column chromatography (ethyl acetate/petroleum ether = 2/1) to give target product 1e (1.4 g, solid), with a yield of 93%.
MS m/z (ESI): 493 [M+1]

Step 5

Ethyl 3-((1H-pyrrolo[2,3-*b*]pyridin-4-yl)thio)propionate (1g)

Ethyl 3-mercaptopropanoate (408 mg, 3.04 mmol), DIPEA (588 mg, 4.56 mmol), $Pd_2(dba)_3$ (140 mg, 0.152 mmol), and XantPhos (132 mg, 0.228 mmol) were added into a solution of 4-bromo-1H-pyrrolo[2,3-*b*]pyridine 1f (300 mg, 1.52 mmol) in dioxane (20 mL). The reaction mixture was heated to 100°C under a nitrogen atmosphere and reacted for 2 h. The reaction mixture was cooled to room temperature and desolventized under reduced pressure. The residue was purified by silica gel column chromatography (ethyl acetate/petroleum ether = 7/3) to give target product 1g (200 mg, oil), with a yield of 53%.
MS m/z (ESI): 251 [M+1]

Step 6

Sodium 1H-pyrrolo[2,3-*b*]pyridin-4-thiolate (1h)

Sodium methoxide in methanol (30%, 158 mg, 0.88 mmol) was added into a solution of 1g (200 mg, 0.8 mmol) in methanol (20 mL), and the reaction mixture was stirred at room temperature for 2 h. The reaction mixture was concentrated under reduced pressure to give target product 1h (100 mg, solid), with a yield of 73%. The product was used directly in the next step without purification.
MS m/z (ESI): 151 [M+1]

Step 7

(*R*)-N-((*R*)-8-(5-((1H-pyrrolo[2,3-*b*]pyridin-4-yl)thio)-1-methyl-6-oxo -1,6-dihydropyrimidin-2-yl)-8-azaspiro[4.5]decan-1-yl)-2-methylpropane-2-sulfinamide (1i)

1h (55 mg, 0.32 mmol) was dissolved in dioxane (6 mL), added with 1e (82 mg, 0.16 mmol), DIPEA (128 mg, 0.99 mmol), $Pd_2(dba)_3$ (30 mg, 0.033 mmol), and XantPhos (29 mg, 0.049 mmol). The reaction mixture was heated to 80°C under nitrogen blanket and reacted for 2 h. The reaction mixture was cooled to room temperature and desolventized under reduced pressure. The residue was purified by silica gel column chromatography (methanol/dichloromethane = 1/9) to give target product 1i (30 mg, solid), with a yield of 37%.
MS m/z (ESI): 515 [M+1]

Step 8

(*R*)-5-((1H-pyrrolo[2,3-*b*]pyridin-4-yl)thio)-2-(1-amino-8-azaspiro[4. 5]decan-8-yl)-3-methylpyrimidin-4(3*H*)-one hydrochloride (1)

1i (30 mg, 0.058 mmol) was dissolved in methanol (5 mL), added with HCl in dioxane (4.0 M, 5 mL), and the

reaction mixture was stirred at room temperature for 2 h. The reaction mixture was concentrated under reduced pressure, and residues were purified by preparative reversed-phase high-performance liquid chromatography (preparative RP-HPLC) to give target product 1 (7.1 mg, solid), with a yield of 31%.

MS m/z (ESI): 411 [M+1]

$^1$H NMR (400 MHz, DMSO-$d_6$) δ 12.68 (s, 1H), 8.24 - 8.14 (m, 5H), 7.68 (s, 1H), 6.91 - 6.87 (m, 1H), 6.74 (s, 1H), 3.75 - 3.64 (m, 2H), 3.43 (s, 3H), 3.19 - 3.08 (m, 3H), 2.08 - 2.05 (m, 1H), 1.92 - 1.64 (m, 7H), 1.54 - 1.51 (m, 1H), 1.44 - 1.41 (m, 1H).

Example 2

(*R*)-2-(1-amino-8-azaspiro[4.5]decan-8-yl)-3-methyl-5-((1-methyl-1H -pyrrolo[2,3-*b*]pyridin-4-yl)thio)pyrimidin-4(3*H*)-one formate

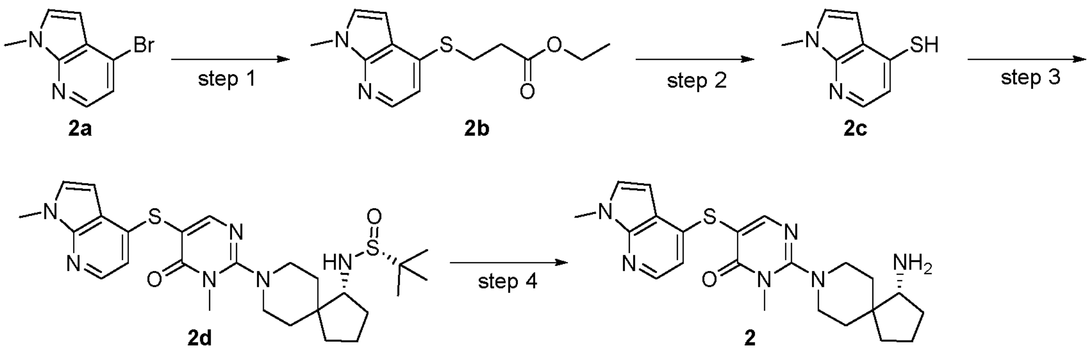

2a 2b 2c 2d 2

Step 1

Ethyl 3-((1-methyl-1H-pyrrolo[2,3-*b*]pyridin-4-yl)thio)propionate (2b)

$Pd_2(dba)_3$ (87 mg, 0.095 mmol) was added into a mixture of 4-bromo-1-methyl-1H-pyrrolo[2,3-*b*]pyridine 2a (200 mg, 0.95 mmol), ethyl 3-mercaptopropanoate (255 mg, 1.9 mmol), DIPEA (368 mg, 2.85 mmol), XantPhos (55 mg, 0.095 mmol), and dioxane (10 mL), and the reaction mixture was heated to 100°C under nitrogen blanket and reacted for 2 h. The reaction mixture was cooled to room temperature, added with water (10 mL), and extracted with ethyl acetate (20 mL × 3). The organic phase was dried over anhydrous sodium sulfate, filtered to remove the drying agent, the filtrate was desolventized under reduced pressure, and the residue was purified by silica gel column chromatography (ethyl acetate/petroleum ether = 3/7) to give target product 2b (240 mg, solid), with a yield of 96%.

MS m/z (ESI): 265 [M+1]

Step 2

1-Methyl-1H-pyrrolo[2,3-*b*]pyridine-4-thiol (2c)

Potassium tert-butoxide (204 mg, 1.82 mmol) was added into a solution of 2b (240 mg, 0.91 mmol) in THF (5 mL), and the reaction mixture was stirred at room temperature for 2 h. The reaction mixture was acidized with hydrochloric acid (6 N) to pH=4 and extracted with ethyl acetate (20 mL). The organic phase was dried over anhydrous sodium sulfate, the drying agent was removed by filtration, and the filtrate was desolventized under reduced pressure to give target product 2c (130 mg, solid), with a yield of 87%.

MS m/z (ESI): 165 [M+1]

Step 3

(*R*)-2-methyl-N-((*R*)-8-(1-methyl-5-((1-methyl-1H-pyrrolo[2,3-*b*]pyri din-4-yl)thio)-6-oxo-1,6-dihydropyrimidin-2-yl)-8-azaspiro[4.5]decan-1-yl )propane-2-sulfinamide (2d)

$Pd_2(dba)_3$ (20 mg, 0.022 mmol) was added into a mixture of 2c (50 mg, 0.24 mmol), 1e (110 mg, 0.22 mmol),

DIPEA (85 mg, 0.66 mmol), XantPhos (13 mg, 0.022 mmol), and dioxane (5 mL), and the reaction mixture was heated to 100°C under nitrogen blanket and reacted for 2 h. The reaction mixture was cooled to room temperature and desolventized under reduced pressure. The residue was purified by silica gel column chromatography (methanol/dichloromethane = 1/9) to give target product 2d (80 mg, solid), with a yield of 68%.

MS m/z (ESI): 529 [M+1]

Step 4

(*R*)-2-(1-amino-8-azaspiro[4.5]decan-8-yl)-3-methyl-5-((1-methyl-1H -pyrrolo[2,3-*b*]pyridin-4-yl)thio)pyrimidin-4(3*H*)-one formate (2)

2d (80 mg, 0.14 mmol) was dissolved in methanol (8 mL), added with a solution of HCl in dioxane (4.0 M, 2 mL), and stirred at room temperature for 2 h. The reaction mixture was concentrated under reduced pressure, and the residue was purified by preparative RP-HPLC to give target product 2 (21.7 mg, solid), with a yield of 34%.

MS m/z (ESI): 425 [M+1]

$^1H$ NMR (400 MHz, DMSO-$d_6$) δ 8.34 (s, 1H), 8.14 (s, 1H), 8.03 (d, *J* = 5.1 Hz, 1H), 7.51 (d, *J* = 3.5 Hz, 1H), 6.58 (d, *J* = 5.1 Hz, 1H), 6.44 (d, *J* = 3.5 Hz, 1H), 3.80 (s, 3H), 3.66 - 3.58 (m, 2H), 3.41 (s, 3H), 3.07 (t, *J* = 11.5 Hz, 2H), 2.99 - 2.96 (m, 1H), 2.0 - 1.92 (m, 1H), 1.80 - 1.68 (m, 4H), 1.60 - 1.53 (m, 3H), 1.40 (d, *J* = 12.9 Hz, 1H), 1.33 (d, *J* = 13.1 Hz, 1H).

Example 3

(*R*)-5-((1H-indazole-4-yl)thio)-2-(1-amino-8-azaspiro[4.5]decan-8-yl) -3-methylpyrimidin-4(3*H*)-one hydrochloride

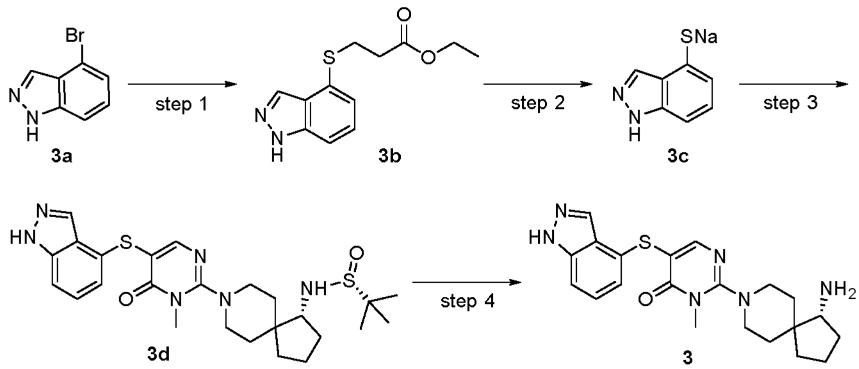


3a 3b 3c 3d 3

Step 1

Ethyl 3-((1H-indazole-4-yl)thio)propionate (3b)

Ethyl 3-mercaptopropanoate (680 mg, 5.06 mmol), DIPEA (979 mg, 7.59 mmol), $Pd_2(dba)_3$ (231 mg, 0.253 mmol), and XantPhos (219 mg, 0.397 mmol) were added into a solution of 4-bromo-1H-indazole 3a (500 mg, 2.53 mmol) in dioxane (20 mL). The reaction mixture was heated to 100°C under a nitrogen atmosphere and reacted for 2 h. The reaction mixture was cooled to room temperature and desolventized under reduced pressure. The residue was purified by silica gel column chromatography (ethyl acetate/petroleum ether = 2.6/1) to give target product 3b (560 mg, solid), with a yield of 89%.

MS m/z (ESI): 251 [M+1]

Step 2

Sodium 1H-indazole-4-thiolate (3c)

Sodium methoxide in methanol (30%, 443.5 mg, 2.46 mmol) was added into a solution of 3b (560 mg, 2.24 mmol) in methanol (20 mL), and the mixture was stirred at 30°C for 1 h. The reaction mixture was desolventized under reduced pressure to give target product 3c (200 mg, solid), with a yield of 51%. The product was used directly in the

next step without purification.

MS m/z (ESI): 151 [M+1]

Step 3

(*R*)-N-((*R*)-8-(5-((1H-indazole-4-yl)thio)-1-methyl-6-oxo-1,6-dihydro pyrimidin-2-yl)-8-azaspiro[4.5]decan-1-yl)-2-methylpropane-2-sulfinamid e (3d)

1e (50 mg, 0.10 mmol), DIPEA (37.2 mg, 0.30 mmol), $Pd_2(dba)_3$ (9.1 mg, 0.01 mmol), and XantPhos (8.7 mg, 0.015 mmol) were added into a solution of 3c (70 mg, 0.41 mmol) in dioxane (6 mL). The reaction mixture was heated to 80°C under nitrogen blanket and reacted for 4 h. The reaction mixture was cooled to room temperature and desolventized under reduced pressure. The residue was purified by silica gel column chromatography (methanol/dichloromethane = 1/9) to give target product 3d (50 mg, solid), with a yield of 96%.

MS m/z (ESI): 515 [M+1]

Step 4

(*R*)-5-((1H-indazole-4-yl)thio)-2-(1-amino-8-azaspiro[4.5]decan-8-yl) -3-methylpyrimidin-4(3*H*)-one hydrochloride (3)

3d (50 mg, 0.097 mmol) was dissolved in methanol (5 mL), added with a solution of HCl in dioxane (4.0 M, 5 mL), and stirred at room temperature for 2 h. The reaction mixture was concentrated under reduced pressure, and the residue was purified by preparative RP-HPLC to give target product 3 (16 mg, solid), with a yield of 41%.

MS m/z (ESI): 411 [M+1]

$^1$H NMR (400 MHz, DMSO-$d_6$) $\delta$ 8.14 (brs, 3H), 8.06 (s, 1H), 7.97 (s, 1H), 7.40 (d, *J* = 8.4 Hz, 1H), 7.24 (dd, *J* = 8.3, 7.2 Hz, 1H), 6.83 (d, *J* = 6.8 Hz, 1H), 3.60 (d, *J* = 13.5 Hz, 1H), 3.52 (d, *J* = 13.3 Hz, 1H), 3.40 (s, 3H), 3.17 - 3.12 (m, 1H), 3.05 - 2.98 (m, 2H), 2.07 - 2.04 (m, 1H), 1.77 - 1.62 (m, 7H), 1.49 (d, *J* = 12.8 Hz, 1H), 1.39 (d, *J* = 13.1 Hz, 1H).

Example 4

(*R*)-2-(1-amino-8-azaspiro[4.5]decan-8-yl)-3-methyl-5-(pyrazolo[1,5-*a*]pyridin-4-ylthio)pyrimidin-4(3*H*)-one formate

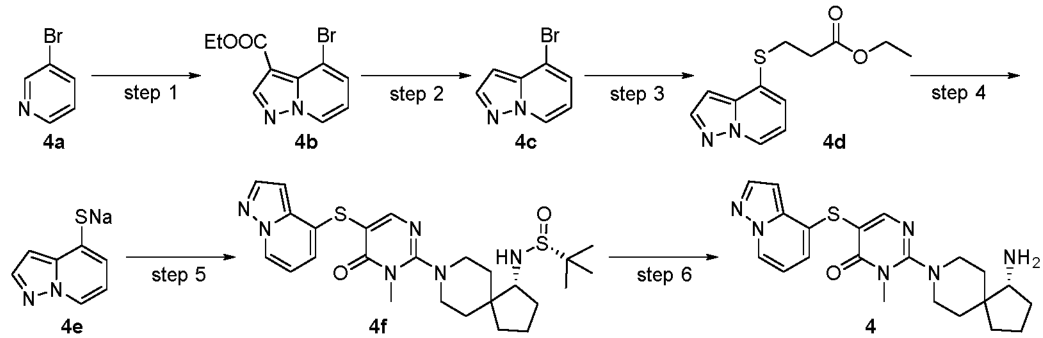


4a 4b 4c 4d 4e 4f 4

Step 1

Ethyl 4-bromopyrazolo[1,5-*a*]pyridine-3-carboxylate (4b)

O-(2,4-dinitrophenyl)hydroxylamine (6.37 g, 32.0 mmol) was added into a solution of 3-bromopyridine 4a (5.0 g, 32.0 mmol) in acetonitrile (25 mL), and the mixture was heated to 40°C and stirred for 16 h. The reaction mixture was desolventized under reduced pressure, and the residue was slurried in ether and dried to give a yellow solid (3.8 g).

The above product (2.0 g, 5.6 mmol) was dissolved in DMF (20 mL), added with potassium carbonate (12.1 g, 88.1 mmol) and ethyl propiolate (7.1 g, 52.8 mmol), and the reaction mixture was stirred at room temperature for 20 h. The resulting mixture was poured into water (50 mL) and extracted with ethyl acetate (30 mL $\times$ 3). The organic phases were combined, washed with saturated brine (20 mL $\times$ 3), dried over anhydrous sodium sulfate, and desolventized under reduced pressure. The residue was purified by silica gel column chromatography (ethyl acetate/petroleum ether = 3/7) to give target product 4b (350 mg, solid), with a yield of 8%.

MS m/z (ESI): 269 [M+1]

Step 2

4-Bromopyrazolo[1,5-*a*]pyridine (4c)

4b (350 mg, 1.30 mmol) was dissolved in a hydrobromic acid solution (12 mL), heated to 100°C, and stirred for 6 h. The reaction mixture was cooled to room temperature, neutralized with sodium hydroxide solution (2 N), and extracted with ethyl acetate (20 mL × 3). The organic phases were combined, washed with saturated brine (15 mL × 3), dried over anhydrous sodium sulfate, and desolventized under reduced pressure. The residue was purified by silica gel column chromatography (ethyl acetate/petroleum ether = 1/4) to give target product 4c (200 mg, solid), with a yield of 79%.

MS m/z (ESI): 197 [M+1]

Step 3

Ethyl 3-(pyrazolo[1,5-*a*]pyridin-4-ylthio)propionate (4d)

Ethyl 3-mercaptopropanoate (680 mg, 5.06 mmol), DIPEA (394 mg, 3.06 mmol), $Pd_2(dba)_3$ (93.3 mg, 0.102 mmol), and XantPhos (88.4 mg, 0.153 mmol) were added into a solution of 4c (200 mg, 1.02 mmol) in dioxane (20 mL). The reaction mixture was heated to 100°C under a nitrogen atmosphere and reacted for 2 h. The reaction mixture was cooled to room temperature and desolventized under reduced pressure. The residue was purified by silica gel column chromatography (ethyl acetate/petroleum ether = 3/7) to give target product 4d (240 mg, solid), with a yield of 94%.

MS m/z (ESI): 251 [M+1]

Step 4

Sodium pyrazolo[1,5-*a*]pyridine-4-thiolate (4e)

Sodium methoxide in methanol (30%, 182 mg, 1.01 mmol) was added into a solution of 4d (230 mg, 0.92 mmol) in methanol (20 mL), and the reaction mixture was stirred at 30°C for 4 h. The reaction mixture was desolventized under reduced pressure to give target product 4e (150 mg, solid), with a yield of 95%. The product was used directly in the next step without purification.

MS m/z (ESI): 151 [M+1]

Step 5

(*R*)-2-methyl-N-((*R*)-8-(1-methyl-6-oxo-5-(pyrazolo[1,5-*a*]pyridin-4-ylthio)-1,6-dihydropyrimidin-2-yl)-8-azaspiro[4.5]decan-1-yl)propane-2-s ulfinamide (4f)

1e (70 mg, 0.142 mmol), DIPEA (54.9 mg, 0.426 mmol), $Pd_2(dba)_3$ (12.9 mg, 0.0142 mmol), and XantPhos (12.3 mg, 0.0213 mmol) were added into 4e (85.3 mg, 0.495 mmol) in dioxane (6 mL). The reaction mixture was heated to 80°C under nitrogen blanket and reacted for 4 h. The reaction mixture was cooled to room temperature and desolventized under reduced pressure. The residue was purified by silica gel column chromatography (methanol/dichloromethane = 1/13) to give target product 4f (40 mg, solid), with a yield of 55%.

MS m/z (ESI): 515 [M+1]

Step 6

(*R*)-2-(1-amino-8-azaspiro[4.5]decan-8-yl)-3-methyl-5-(pyrazolo[1,5-*a*]pyridin-4-ylthio)pyrimidin-4(3*H*)-one formate (4)

4f (40 mg, 0.078 mmol) was dissolved in methanol (5 mL), added with HCl in dioxane (4.0 M, 5 mL), and stirred at room temperature for 2 h. The reaction mixture was concentrated under reduced pressure, and the residue was purified by preparative RP-HPLC to give target product 4 (16 mg, solid), with a yield of 51%.

MS m/z (ESI): 411 [M+1]

$^1H$ NMR (400 MHz, DMSO-$d_6$) δ 8.56 (d, *J* = 6.9 Hz, 1H), 8.39 (s, 1H), 8.09 (s, 1H), 8.03 (d, *J* = 2.2 Hz, 1H), 6.89 (d, *J* = 7.0 Hz, 1H), 6.80 (t, *J* = 7.0 Hz, 1H), 6.63 (d, *J* = 1.7 Hz, 1H), 3.64 - 3.53 (m, 2H), 3.39 (s, 3H), 3.05 - 2.97 (m, 3H), 1.98 - 1.95 (m, 1H), 1.80 - 1.67 (m, 4H), 1.64 - 1.53 (m, 3H), 1.40 (d, *J* = 13.0 Hz, 1H), 1.32 (d, *J* = 13.3 Hz, 1H).

Example 5

(*R*)-2-(1-amino-8-azaspiro[4.5]decan-8-yl)-3-methyl-5-(pyrazolo[1,5-*a*]pyridin-5-ylthio)pyrimidin-4(3*H*)-one hydrochloride

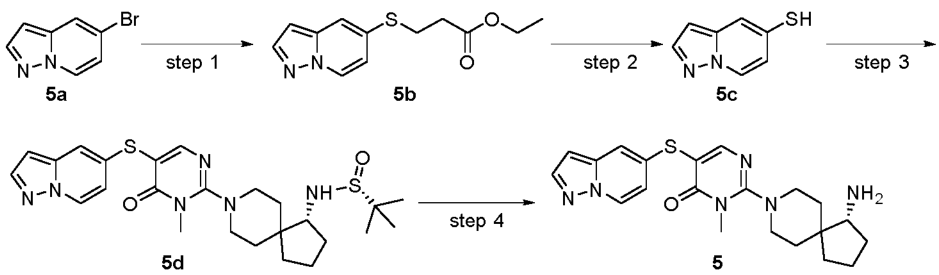


Step 1

Ethyl 3-(pyrazolo[1,5-*a*]pyridin-5-ylthio)propionate (5b)

Ethyl 3-mercaptopropanoate (326 mg, 2.43 mmol), DIPEA (627 mg, 4.86 mmol), $Pd_2(dba)_3$ (148 mg, 0.162 mmol), and XantPhos (140 mg, 0.243 mmol) were added into a solution of 5-bromopyrazolo[1,5-*a*]pyridine 5a (320 mg, 1.62 mmol) in dioxane (20 mL). The reaction mixture was heated to 100°C under a nitrogen atmosphere and reacted for 6 h. The reaction mixture was cooled to room temperature and desolventized under reduced pressure. The residue was purified by silica gel column chromatography (ethyl acetate/petroleum ether = 3/1) to give target product 5b (400 mg, oil), with a yield of 97%.

MS m/z (ESI): 251 [M+1]

Step 2

Pyrazolo[1,5-a]pyridine-5-thiol (5c)

Sodium methoxide in methanol (30%, 317 mg, 1.76 mmol) was added into a solution of 5b (400 mg, 1.6 mmol) in methanol (20 mL), and the reaction mixture was stirred at room temperature for 2 h. The reaction mixture was poured into water (100 mL), adjusted to weakly acidic pH with citric acid, and extracted with ethyl acetate (30 mL × 3). The organic phases were combined, washed with saturated brine (20 mL × 3), dried over anhydrous sodium sulfate, filtered to remove the drying agent, and desolventized under reduced pressure. The residue was purified by silica gel column chromatography (ethyl acetate/petroleum ether = 2/3) to give target product 5c (100 mg, oil), with a yield of 42%.

MS m/z (ESI): 151 [M+1]

Step 3

(*R*)-2-methyl-N-((*R*)-8-(1-methyl-6-oxo-5(pyrazolo[1,5-*a*]pyridin-5-y lthio)-1,6-dihydropyrimidin-2-yl)-8-azaspiro[4.5]decan-1-yl)propane-2-sul finamide (5d)

5c (30 mg, 0.20 mmol) was dissolved in dioxane (6 mL), and supplemented with 1e (50 mg, 0.10 mmol), DIPEA (38.7 mg, 0.30 mmol), $Pd_2(dba)_3$ (9.0 mg, 0.010 mmol), and XantPhos (8.5 mg, 0.015 mmol). The reaction mixture was heated to 80°C under nitrogen blanket and reacted for 2 h. The reaction mixture was cooled to room temperature and desolventized under reduced pressure. The residue was purified by silica gel column chromatography (methanol/dichloromethane = 1/9) to give target product 5d (60 mg, solid, crude product).

MS m/z (ESI): 515 [M+1]

Step 4

($R$)-2-(1-amino-8-azaspiro[4.5]decan-8-yl)-3-methyl-5-(pyrazolo[1,5-$a$]pyridin-5-ylthio)pyrimidin-4(3$H$)-one hydrochloride (5)

5d (60 mg, crude product) was dissolved in methanol (5 mL), added with HCl in dioxane (4.0 M, 5 mL), and stirred at room temperature for 2 h. The reaction mixture was concentrated under reduced pressure, and residues were purified by preparative RP-HPLC to give target product 5 (14.1 mg, solid), with a yield of 34% in two steps.

MS m/z (ESI): 411 [M+1]

$^1$H NMR (400 MHz, DMSO-$d_6$) δ 8.56 (d, $J$ = 7.3 Hz, 1H), 8.13 (s, 1H), 8.06 (brs, 3H), 7.95 (s, 1H), 7.94 (d, $J$ = 2.2 Hz, 2H), 7.33 (d, $J$ = 1.4 Hz, 1H), 6.68 (dd, $J$ = 7.3, 2.1 Hz, 1H), 6.44 (dd, $J$ = 2.2, 0.7 Hz, 1H), 3.66 (d, $J$ = 13.4 Hz, 1H), 3.58 (d, $J$ = 13.3 Hz, 1H), 3.41 (s, 3H), 3.21 - 3.14 (m, 1H), 3.10 - 3.02 (m, 2H), 2.07 - 2.03 (m, 1H), 1.87 - 1.58 (m, 7H), 1.50 (d, $J$ = 12.8 Hz, 1H), 1.41 (d, $J$ = 12.9 Hz, 1H).

Example 6

($R$)-5-((1H-indazole-5-yl)thio)-2-(1-amino-8-azaspiro[4.5]decan-8-yl) -3-methylpyrimidin-4(3$H$)-one hydrochloride

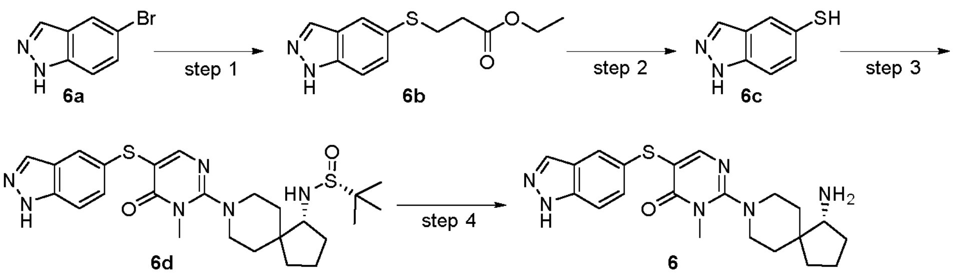


6a 6b 6c 6d 6

Step 1

Ethyl 3-((1H-indazole-5-yl)thio)propionate (6b)

$Pd_2(dba)_3$ (243 mg, 0.265 mmol) was added into a mixture of 5-bromo-1H-indazole 6a (520 mg, 2.65 mmol), ethyl 3-mercaptopropanoate (533 mg, 3.98 mmol), DIPEA (1.02 g, 7.95 mmol), XantPhos (153 mg, 0.265 mmol), and dioxane (10 mL), and then the reaction mixture was heated to 100°C under nitrogen blanket and reacted for 2 h. The reaction mixture was cooled to room temperature and desolventized under reduced pressure. The residue was purified by silica gel column chromatography (ethyl acetate/petroleum ether = 1/1) to give target product 6b (630 mg, solid), with a yield of 95%.

MS m/z (ESI): 251 [M+1]

Step 2

1H-Indazole-5-thiol (6c)

Potassium tert-butoxide (672 mg, 6.0 mmol) was added into a solution of 6b (500 mg, 2.0 mmol) in THF (10 mL), and the mixture was stirred at room temperature for 2 h. The reaction mixture was acidized with hydrochloric acid (6 N) to pH=4 and extracted with dichloromethane (20 mL). The organic phase was dried over anhydrous sodium sulfate, and filtered to remove the drying agent, and the filtrate was desolventized under reduced pressure to give target product 6c (300 mg, solid), with a yield of 100%.

MS m/z (ESI): 151 [M+1]

Step 3

(*R*)-N-((*R*)-8-(5-((1H-indazole-5-yl)thio)-1-methyl-6-oxo-1,6-dihydro pyrimidin-2-yl)-8-azaspiro[4.5]decan-1-yl)-2-methylpropane-2-sulfinamid e (6d)

$Pd_2(dba)_3$ (18 mg, 0.02 mmol) was added into a mixture of 6c (60 mg, 0.4 mmol), 1e (100 mg, 0.2 mmol), DIPEA (77 mg, 0.6 mmol), XantPhos (12 mg, 0.02 mmol), and dioxane (5 mL), and then the reaction mixture was heated to 100°C under nitrogen blanket and reacted for 2 h. The reaction mixture was cooled to room temperature and desolventize under reduced pressure. The residue was purified by silica gel column chromatography (ethyl acetate/petroleum ether = 1/1) to give target product 6d (70 mg, solid), with a yield of 67%.
MS m/z (ESI): 515 [M+1]

Step 4

(*R*)-5-((1H-indazole-5-yl)thio)-2-(1-amino-8-azaspiro[4.5]decan-8-yl) -3-methylpyrimidin-4(3*H*)-one hydrochloride (6)

6d (70 mg, 0.14 mmol) was dissolved in methanol (5 mL), added with HCl in dioxane (4.0 M, 5 mL), and stirred at room temperature for 2 h. The reaction mixture was concentrated under reduced pressure, and the residue was purified by preparative RP-HPLC to give target product 6 (30 mg, solid), with a yield of 54%.
MS m/z (ESI): 411 [M+1]
$^1H$ NMR (400 MHz, DMSO-$d_6$) δ 8.28 (s, 3H), 8.05 (s, 1H), 7.79 (s, 1H), 7.69 (s, 1H), 7.55 (d, *J* = 8.7 Hz, 1H), 7.34 (dd, *J* = 8.7, 1.6 Hz, 1H), 3.56 (d, *J* = 13.2 Hz, 1H), 3.49 (d, *J* = 13.5 Hz, 1H), 3.39 (s, 3H), 3.17 - 3.13 (m, 1H), 3.06 - 2.96 (m, 2H), 2.07 - 2.01 (m, 1H), 1.90 - 1.61 (m, 7H), 1.52 (d, *J* = 13.1 Hz, 1H), 1.39 (d, *J* = 13.0 Hz, 1H).

Example 7

(*R*)-2-(1-amino-8-azaspiro[4.5]decan-8-yl)-5-((4-chloro-2-methyl-2H-indazole-5-yl)thio)-3-methylpyrimidin-4(3*H*)-one hydrochloride

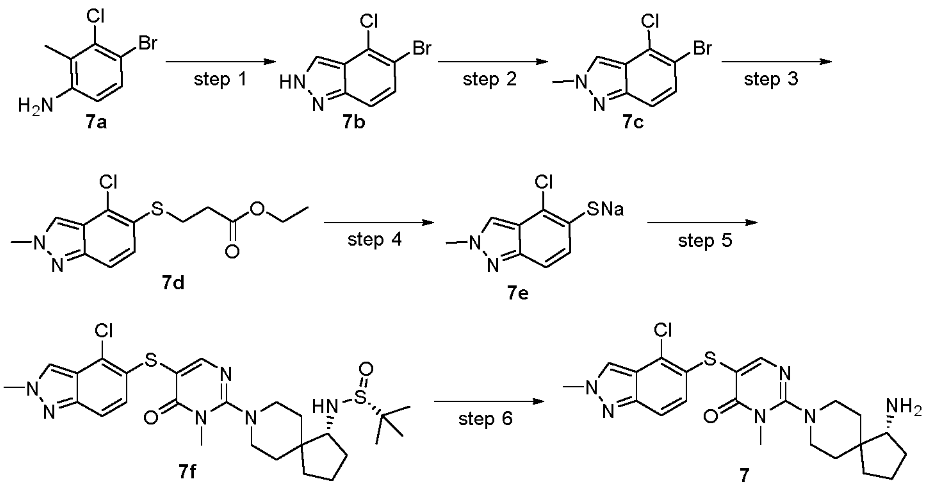


Step 1

5-Bromo-4-chloro-2H-indazole (7b)

An aqueous solution (4 mL) of sodium nitrite (396 mg, 5.73 mmol) was added into 4-bromo-3-chloro-2-methylaniline 7a (1.0 g, 4.58 mmol) in acetic acid (20 mL) cooled on an ice bath and the mixture was stirred at room temperature for 1 h. Most of the solvent was removed by rotary evaporation, and the residue was suspended in water (30 mL) and filtered. The filter cake was washed with water (25 mL×3) and air-dried to give target product 7b (780 mg, solid), with a yield of 74%. The product was used directly in the next step without purification.
MS m/z (ESI): 231 [M+1]

Step 2

5-Bromo-4-chloro-2-methyl-2H-indazole (7c)

Trimethyloxonium tetrafluoroborate (733 mg, 4.95 mmol) was added into 7b (760 mg, 3.3 mmol) in ethyl acetate (15 mL) cooled on an ice bath, and the reaction mixture was stirred at room temperature for 4 h. The reaction mixture was diluted with petroleum ether (30 mL), stirred for 10 min, and filtered. The filtrate was added into ethyl acetate (20 mL), and washed with sodium bicarbonate solution (30 mL $\times$ 3) and saturated brine (20 mL $\times$ 3). The organic phase was dried over anhydrous sodium sulfate and concentrated under reduced pressure. The residue was purified by silica gel column chromatography (ethyl acetate/petroleum ether = 1.4/1) to give target product 7c (460 mg, solid), with a yield of 57%.

MS m/z (ESI): 245 [M+1]

Step 3

Ethyl 3-((4-chloro-2-methyl-2H-indazole-5-yl)thio)propionate (7d)

Ethyl 3-mercaptopropanoate (483 mg, 3.6 mmol), DIPEA (697 mg, 5.4 mmol), $Pd_2(dba)_3$ (165 mg, 0.18 mmol), and XantPhos (156 mg, 0.27 mmol) were added into a solution of 7c (440 mg, 1.8 mmol) in dioxane (20 mL). The reaction mixture was heated to 100°C under a nitrogen atmosphere and reacted for 16 h. The reaction mixture was cooled to room temperature and desolventized under reduced pressure. The residue was purified by silica gel column chromatography (ethyl acetate/petroleum ether = 1.7/1) to give target product 7d (300 mg, solid), with a yield of 59%.

MS m/z (ESI): 299 [M+1]

Step 4

Sodium 4-chloro-2-methyl-2H-indazole-5-thiolate (7e)

Sodium methoxide in methanol (30%, 93 mg, 0.52 mmol) was added into a solution of 7d (140 mg, 0.47 mmol) in methanol (20 mL), and stirred at 30°C for 32 h. The reaction mixture was desolventized under reduced pressure to give target product 7e (100 mg, solid), with a yield of 96%. The product was used directly in the next step without purification.

MS m/z (ESI): 199 [M+1]

Step 5

(*R*)-N-((*R*)-8-(5-((4-chloro-2-methyl-2H-indazole-5-yl)thio)-1-methyl -6-oxo-1,6-dihydropyrimidin-2-yl)-8-azaspiro[4.5]decan-1-yl)-2-methylpr opane-2-sulfinamide (7f)

7e (80 mg, 0.36 mmol) was dissolved in dioxane (8 mL), and supplemented with 1e (100 mg, 0.20 mmol), DIPEA (77 mg, 0.60 mmol), $Pd_2(dba)_3$ (18 mg, 0.020 mmol), and XantPhos (18 mg, 0.030 mmol). The reaction mixture was heated to 90°C under nitrogen blanket and reacted for 2 h. After cooling down to room temperature, the reaction mixture was poured into a mixed solvent of ethyl acetate (15 mL) and petroleum ether (15 mL) and filtered. The filter cake was dried to give target product 7f (61 mg, solid), with a yield of 54%.

MS m/z (ESI): 563 [M+1]

Step 6

(*R*)-2-(1-amino-8-azaspiro[4.5]decan-8-yl)-5-((4-chloro-2-methyl-2H-indazole-5-yl)thio)-3-methylpyrimidin-4(3*H*)-one hydrochloride (7)

7f (61 mg, 0.11 mmol) was dissolved in methanol (5 mL), added with HCl in dioxane (4.0 M, 5 mL), and stirred at room temperature for 2 h. The reaction mixture was concentrated under reduced pressure, and the residue was purified by preparative RP-HPLC to give target product 7 (16 mg, solid), with a yield of 31%.

MS m/z (ESI): 459 [M+1]

$^1H$ NMR (400 MHz, DMSO-$d_6$) $\delta$ 8.43 (s, 1H), 8.12 (brs, 3H), 7.90 (s, 1H), 7.51 (dd, *J* = 9.0, 0.7 Hz, 1H), 6.99 (d, *J* = 9.0 Hz, 1H), 4.17 (s, 3H), 3.60 (d, *J* = 13.3 Hz, 1H), 3.53 (d, *J* = 13.3 Hz, 1H), 3.40 (s, 3H), 3.16 - 3.13 (m, 1H), 3.06 - 2.98 (m, 2H), 2.07 - 2.04 (m, 1H), 1.87 - 1.60 (m, 7H), 1.49 (d, *J* = 13.0 Hz, 1H), 1.39 (d, *J* = 13.1 Hz, 1H).

Example 8

(*R*)-2-(1-amino-8-azaspiro[4.5]decan-8-yl)-5-((4-chloropyrazolo[1,5-*a*]pyridin-5-yl)thio)-3-methylpyrimidin-4(3*H*)-one formate

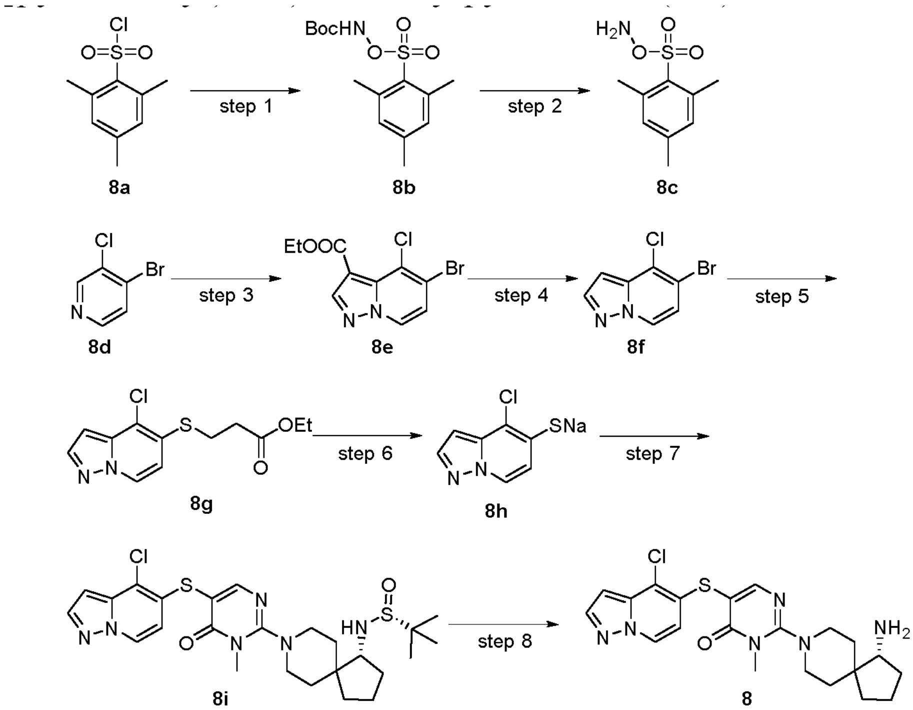


Step 1

*tert*-Butyl [[(2,4,6-trimethylphenyl)sulfonyl]oxy]carbamate (8b)

2,4,6-Trimethylbenzenesulfonyl chloride 8a (2.0 g, 9.15 mmol) and *tert*-butyl hydroxycarbamate (1.22 g, 9.15 mmol) were dissolved in tert-butyl methyl ether (30 mL) and cooled to 0°C, triethylamine (1.4 mL, 10.1 mmol) was added dropwise, and the reaction mixture was heated to room temperature and stirred for 4 h. The reaction mixture was washed with water, and the organic phase was dried over anhydrous sodium sulfate and desolventized under reduced pressure. The residue was purified by silica gel column chromatography (ethyl acetate/petroleum ether = 1/5) to give target product 8b (2.5 g, solid), with a yield of 87%.

MS m/z (ESI): 338 [M+23], 216 [M+1-100]

Step 2

O-(mesitylsulfonyl)hydroxylamine (8c)

8b (2.5 g, 7.93 mmol) was added into trifluoroacetic acid (20 mL) in portions at 0°C, and the reaction mixture was stirred at 0°C for 2 h. Ice water was added slowly and the mixture was stirred for 15 min. After filtration, the filter cake was washed with water until the filtrate was neutral. The filter cake was dried to give target product 8c (1.5 g, solid), with a yield of 88%.

MS m/z (ESI): 216 [M+1]

Step 3

Ethyl 5-bromo-4-chloropyrazolo[1,5-*a*]pyridine-3-carboxylate (8e)

4-Bromo-3-chloropyridine 8d (1.4 g, 7.28 mmol) was dissolved in acetonitrile (25 mL) and added with 8c (1.6

g, 7.28 mmol), and the reaction mixture was reacted overnight under stirring at 40°C. After filtration, the filter cake was dried to give a yellow solid product (1.7 g).

The above product (1.6 g, 3.92 mmol) and ethyl propiolate (0.42 g, 4.32 mmol) were dissolved in DMF (15 mL), added with potassium carbonate (1.1 g, 7.84 mmol), and stirred at room temperature for 3 h. The reaction was quenched with water and the reaction mixture was extracted with ethyl acetate (100 mL × 3). The organic phases were combined, washed with saturated brine, dried over anhydrous sodium sulfate, and desolventized under reduced pressure. The esidue was purified by silica gel column chromatography (ethyl acetate/petroleum ether = 1/2) to give target product 8e (120 mg, solid), with a yield of 5.8%.

MS m/z (ESI): 303, 305 [M+1]

Step 4

5-Bromo-4-chloropyrazolo[1,5-*a*]pyridine (8f)

8e (110 mg, 0.36 mmol) was dissolved in hydrobromic acid solution (3 mL), heated to 100°C, and stirred for 3 h. The reaction mixture was cooled to room temperature, neutralized with sodium hydroxide solution (2 M), and extracted with dichloromethane (30 mL × 3). The organic phases were combined, washed with saturated brine, dried over anhydrous sodium sulfate, and desolventized under reduced pressure. The residue was purified by silica gel column chromatography (ethyl acetate/petroleum ether = 3/7) to give target product 8f (65 mg, solid), with a yield of 77%.

MS m/z (ESI): 231, 233 [M+1]

Step 5

Ethyl 3-((4-chloropyrazolo[1,5-*a*]pyridin-5-yl)thio)propionate (8g)

Ethyl 3-mercaptopropanoate (38 mg, 0.29 mmol), DIPEA (67 mg, 0.52 mmol), $Pd_2(dba)_3$ (24 mg, 0.026 mmol), and XantPhos (30 mg, 0.052 mmol) were added into a solution of 8f (60 mg, 0.26 mmol) in dioxane (5 mL). The reaction mixture was heated to 100°C under a nitrogen atmosphere and reacted for 3 h. The reaction mixture was cooled to room temperature and desolventized under reduced pressure. The residue was purified by silica gel column chromatography (methanol/dichloromethane = 1/28) to give target product 8g (60 mg, solid), with a yield of 81%.

MS m/z (ESI): 285 [M+1]

Step 6

Sodium 4-chloropyrazolo[1,5-*a*]pyridine-5-thiolate (8h)

Sodium methoxide in methanol (30%, 45.5 mg, 0.25 mmol) was added into the solution of 8g (60 mg, 0.21 mmol) in methanol (3 mL), and the mixture was stirred at 30°C for 4 h. The reaction mixture was desolventized under reduced pressure to give target product 8h (40 mg, solid), with a yield of 92%. The product was used directly in the next step without purification.

MS m/z (ESI): 185 [M+1]

Step 7

(*R*)-N-((*R*)-8-(5-((4-chloropyrazolo[1,5-*a*]pyridin-5-yl)thio)-1-methyl -6-oxo-1,6-dihydropyrimidin-2-yl)-8-azaspiro[4.5]decan-1-yl)-2-methylpr opane-2-sulfinamide (8i)

8h (40 mg, 0.19 mmol) was dissolved in dioxane (5 mL), and supplemented with 1e (95 mg, 0.19 mmol), DIPEA (49 mg, 0.38 mmol), $Pd_2(dba)_3$ (17 mg, 0.02 mmol), and XantPhos (22 mg, 0.04 mmol). The reaction mixture was heated to 100°C under nitrogen blanket and reacted for 4 h. The reaction mixture was cooled to room temperature and desolventized under reduced pressure. The residue was purified by silica gel column chromatography (methanol/dichloromethane = 1/28) to give target product 8i (32 mg, oil), with a yield of 30%.

MS m/z (ESI): 549 [M+1]

Step 8

(*R*)-2-(1-amino-8-azaspiro[4.5]decan-8-yl)-5-((4-chloropyrazolo[1,5-*a*]pyridin-5-yl)thio)-3-methylpyrimidin-4(3*H*)-one formate (8)

8i (32 mg, 0.06 mmol) was dissolved in methanol (2 mL), added with HCl in dioxane (4.0 M, 1 mL), and stirred at room temperature for 1 h. The reaction mixture was concentrated under reduced pressure, and the residue was purified by preparative RP-HPLC to give target product 8 (13 mg, solid), with a yield of 47%.
MS m/z (ESI): 445 [M+1]
$^1$H NMR (400 MHz, DMSO-$d_6$) δ 8.54 (d, *J* = 7.3 Hz, 1H), 8.35 (s, 1H), 8.19 (s, 1H), 8.06 (d, *J* = 2.2 Hz, 1H), 6.60 (*d, J* = 1.8 Hz, 1H), 6.48 (d, *J* = 7.3 Hz, 1H), 3.69 - 3.60 (m, 2H), 3.41 (s, 3H), 3.09 (t, *J* = 11.7 Hz, 2H), 3.02 - 2.99 (m, 1H), 2.00 - 1.91 (m, 1H), 1.76 - 1.50 (m, 7H), 1.42 (d, *J* = 13.2 Hz, 1H), 1.35 (d, *J* = 12.9 Hz, 1H).

Example 9

(*R*)-5-((2-(1-amino-8-azaspiro[4.5]decan-8-yl)-1-methyl-6-oxo-1,6-di hydropyrimidin-5-yl)thio)-4-chloro-2-methyl-2H-indazole-3-carbonitrile

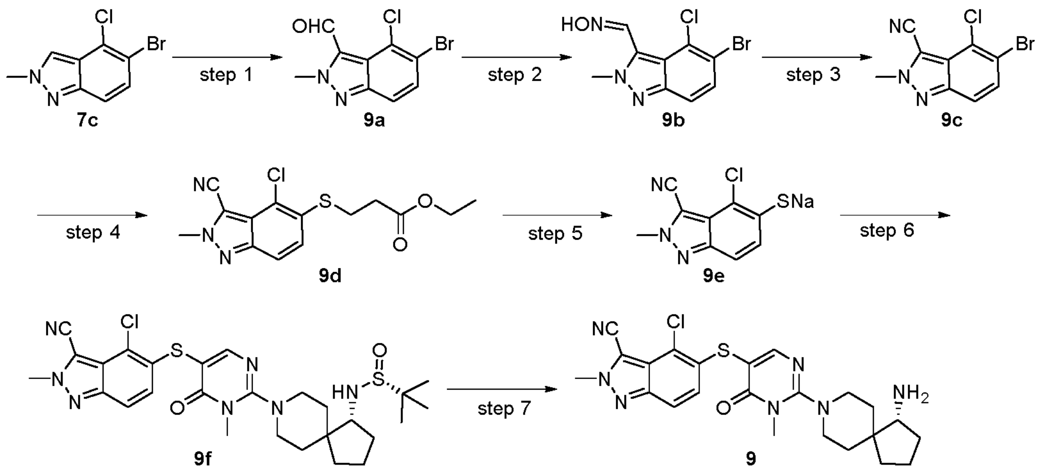

7c 9a 9b 9c 9d 9e 9f 9

Step 1

5-Bromo-4-chloro-2-methyl-2H-indazole-3-carbaldehyde (9a)

7c (800 mg, 3.28 mmol) was dissolved in THF (20 mL), cooled to -78°C, added with lithium diisopropylamide in THF (2 M, 2.95 mL, 5.9 mmol), stirred for 90 min, warmed up to 0°C, and kept stirring for 30 min. The reaction mixture was then cooled to -78°C, added with DMF (0.76 mL) dropwise, stirred for 0.5 h, warmed up to room temperature, and kept stirring for 2 h. The reaction was quenched with saturated ammonium chloride solution, and the mixture was extracted with ethyl acetate. The organic phase was dried over anhydrous sodium sulfate, filtered to remove the drying agent, and desolventized under reduced pressure. The residue was purified by silica gel column chromatography (ethyl acetate/petroleum ether = 3/7) to give target product 9a (520 mg, solid), with a yield of 60%.
MS m/z (ESI): 273 [M+1]

Step 2

5-Bromo-4-chloro-2-methyl-2H-indazole-3-carboxaldoxime (9a)

9a (490 mg, 1.8 mmol) was dissolved in isopropanol (8 mL), methanol (8 mL), and water (8 mL), added with hydroxylamine hydrochloride (497 mg, 7.2 mmol) and sodium carbonate (763 mg, 7.2 mmol), heated to 50°C, and stirred for 16 h. The resulting mixture was poured into water (60 mL) and extracted with ethyl acetate (50 mL × 3). The organic phases were combined, washed with saturated brine (20 mL × 3), dried over anhydrous sodium sulfate, and desolventized under reduced pressure. The residue was purified by silica gel column chromatography (ethyl acetate/petroleum ether

= 3/7) to give target product 9b (470 mg, solid), with a yield of 92%.
MS m/z (ESI): 288 [M+1]

Step 3

5-Bromo-4-chloro-2-methyl-2H-indazole-3-carbonitrile (9c)

9b (470 mg, 1.63 mmol) was dissolved in acetonitrile (30 mL), added with copper acetate (147 mg, 0.81 mmol), heated to 85°C, and stirred overnight. The reaction mixture was desolventized under reduced pressure, and the residue was poured into water (50 mL) and extracted with ethyl acetate (30 mL × 3). The organic phases were combined, washed with saturated brine (25 mL × 3), dried over anhydrous sodium sulfate, and desolventized under reduced pressure to give target product 9c (390 mg, solid), with a yield of 89%.
MS m/z (ESI): 270 [M+1]

Step 4

Ethyl 3-((4-chloro-3-cyano-2-methyl-2H-indazole-5-yl)thio)propionate (9d)

Ethyl 3-mercaptopropanoate (385 mg, 2.8 mmol), DIPEA (542 mg, 4.2 mmol), $Pd_2(dba)_3$ (128 mg, 0.14 mmol), and XantPhos (121 mg, 0.21 mmol) were added into a solution of 9c (380 mg, 1.4 mmol) in dioxane (5 mL). The reaction mixture was heated to 100°C under nitrogen blanket and reacted for 16 h. The reaction mixture was cooled to room temperature and desolventized under reduced pressure. The residue was purified by silica gel column chromatography (ethyl acetate/petroleum ether = 1/1) to give target product 9d (350 mg, solid), with a yield of 77%.
MS m/z (ESI): 324 [M+1]

Step 5

Sodium 4-chloro-3-cyano-2-methyl-2H-indazole-5-thiolate (9e)

Sodium methoxide in methanol (30%, 130 mg, 0.72 mmol) was added into a solution of 9d (195 mg, 0.60 mmol) in methanol (20 mL), and stirred at 30°C for 16 h. The reaction mixture was desolventized under reduced pressure to give target product 9e (220 mg, solid, crude product). The product was used directly in the next step without purification.
MS m/z (ESI): 224 [M+1]

Step 6

(*R*)-N-((*R*)-8-(5-((4-chloro-3-cyano-2-methyl-2H-indazole-5-yl)thio)-1-methyl-6-oxo-1,6-dihydropyrimidin-2-yl)-8-azaspiro[4.5]decan-1-yl)-2-methylpropane-2-sulfinamide (9f)

9e (89.2 mg, 0.36 mmol) was dissolved in dioxane (6 mL), and supplemented with 1e (120 mg, 0.24 mmol), DIPEA (92.8 mg, 0.72 mmol), $Pd_2(dba)_3$ (21.9 mg, 0.024 mmol), and XantPhos (20.8 mg, 0.036 mmol). The reaction mixture was heated to 80°C under nitrogen blanket and reacted for 16 h. The reaction mixture was cooled to room temperature and desolventized under reduced pressure. The residue was purified by silica gel column chromatography (methanol/dichloromethane = 1/12) to give target product 9f (90 mg, solid), with a yield of 63% in two steps.
MS m/z (ESI): 588 [M+1]

Step 7

(*R*)-5-((2-(1-amino-8-azaspiro[4.5]decan-8-yl)-1-methyl-6-oxo-1,6-di hydropyrimidin-5-yl)thio)-4-chloro-2-methyl-2H-indazole-3-carbonitrile formate (9)

9f (90 mg, 0.15 mmol) was dissolved in methanol (10 mL), added with HCl in dioxane (4.0 M, 10 mL), and stirred at room temperature for 2 h. The reaction mixture was concentrated under reduced pressure, and the residue was purified by preparative RP-HPLC to give target product 9 (14 mg, solid), with a yield of 19%.
MS m/z (ESI): 484 [M+1]
$^1H$ NMR (400 MHz, DMSO-$d_6$) $\delta$ 8.14 (s, 1H), 7.79 (brs, 3H), 7.75 (d, *J* = 9.0 Hz, 1H), 7.05 (d, *J* = 9.1 Hz, 1H), 4.34 (s, 3H), 3.69 (d, *J* = 14.1 Hz, 1H), 3.61 (d, *J* = 14 Hz, 1H), 3.41 (s, 3H), 3.24 - 3.16 (m, 1H), 3.13 - 3.06 (m, 2H), 2.08 - 2.04 (m, 1H), 1.80 - 1.64 (m, 7H), 1.49 - 1.41 (m, 2H).

Example 10

(*S*)-2-(4-amino-2-oxa-8-azaspiro[4.5]decan-8-yl)-5-((4-chloro-2-meth yl-2H-indazole-5-yl)thio)-3-methylpyrimidin-4(3*H*)-one

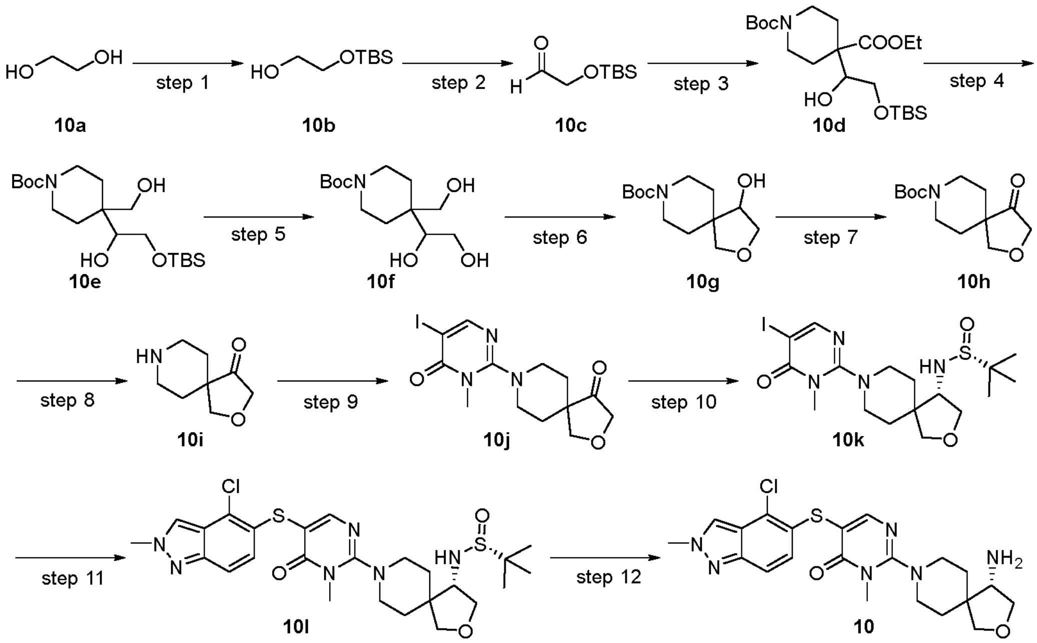


10a 10b 10c 10d 10e 10f 10g 10h 10i 10j 10k 10l 10

Step 1

2-((*tert*-Butyldimethylsilyl)oxy)ethan-1-ol (10b)

*tert*-Butyldimethylsilyl chloride (50 g, 334 mmol) was added dropwise into a mixture of ethylene glycol 10a (124 g, 2 mol), imidazole (34 g, 500 mmol), and dichloromethane (500 mL) at 0°C, and the reaction mixture was stirred at room temperature for 16 h. The reaction mixture was diluted with water (300 mL) and dichloromethane (600 mL), and separated. The aqueous phase was extracted with dichloromethane (300 mL × 3). The organic phases were combined, dried over anhydrous sodium sulfate, filtered, and concentrated under reduced pressure. The residue was purified by silica gel column chromatography (ethyl acetate/petroleum ether = 1/5) to give target product 10b (35 g, oil), with a yield of 10%.

MS m/z (ESI): 177 [M+1]

Step 2

2-((*tert*-Butyldimethylsilyl)oxy)acetaldehyde (10c)

Oxalyl chloride (27.6 g, 218.9 mmol) was added dropwise into dichloromethane (500 mL) at -30°C, and the mixture was cooled to -78°C to which was added dropwise dimethyl sulfoxide (21.7 g, 278.6 mmol). The reaction mixture was stirred at -78°C for 30 min, added with a solution of 10b (35 g, 199 mmol) in dichloromethane (100 mL) slowly and stirred for 1 h. Triethylamine (100.5 g, 995 mmol) was added dropwise, and the mixture was stirred at -78°C for 30 min and then at room temperature overnight. The reaction mixture was washed with water (300 mL), dilute hydrochloric acid (1 N, 400 mL × 2), saturated sodium bicarbonate solution (400 mL), and saturated brine (400 mL) successively. The organic phase was dried over anhydrous sodium sulfate, filtered, and concentrated under reduced pressure to give target product 10c (34 g, oil), with a yield of 98%.

$^1H$ NMR (400 MHz, $CDCl_3$) δ 9.60 (s, 1H), 4.11 (s, 2H), 0.82 (s, 9H), 0.00 (s, 6H).

Step 3

1-*tert*-Butyl 4-ethyl 4-(2-((tert-butyldimethylsilyl)oxy)-1-hydroxyethyl)piperidine-1,4-dicarbox ylate (10d)

1-*tert*-Butyl 4-ethylpiperidine-1,4-dicarboxylate (50 g, 197 mmol) in THF (100 mL) was added dropwise into a solution of lithium diisopropylamide in THF (2 M, 148 mL, 296 mmol) and THF (400 mL) at -10°C, and the reaction mixture was stirred at 0°C for 30 min. 10c (34 g, 197 mmol) was added, and the reaction mixture was stirred at 0°C for 1 h and at room temperature for 1 h. A mixture of saturated sodium bicarbonate solution and water (400 mL, 1/4 v/v) was added, followed by ethyl acetate (200 mL) , and the resulting mixture was separated. The aqueous phase was extracted with ethyl acetate (200 mL × 3). The organic phases were combined, dried over anhydrous sodium sulfate, filtered, and concentrated under reduced pressure. The residue was purified by silica gel column chromatography (ethyl acetate/petroleum ether = 2/3) to give target product 10d (75 g, oil), with a yield of 88%.
MS m/z (ESI): 332 [M+1-100]

Step 4

*tert-Butyl* 4-(2-((*tert*-butyldimethylsilyl)oxy)-1-hydroxyethyl)-4-(hydroxymethyl)pip eridine-1-carboxylate (10e)

Lithium borohydride in THF (2 M, 130 mL, 261 mmol) was added into 10d (75 g, 174 mmol) in THF (600 mL), and the mixture was stirred at room temperature for 2 h. After cooling to 0°C, the reaction was quenched with saturated sodium bicarbonate solution and water (150 mL, 1/2 v/v), and the reaction mixture was diluted with ethyl acetate (200 mL) and filtered. The filtrate was separated and the aqueous phase was extracted with ethyl acetate (200 mL × 3). The organic phases were combined, washed with saturated brine, dried over anhydrous sodium sulfate, filtered, and concentrated under reduced pressure to give target product 10e (55 g, oil), with a yield of 81%.
MS m/z (ESI): 290 [M+1-100]

Step 5

*tert-Butyl* 4-(1,2-dihydroxyethyl)-4-(hydroxymethyl)piperidine-1-carboxylate (10f)

Tetrabutylammonium fluoride in THF (1 M, 193 mL, 193 mmol) was added into 10e (55 g, 128.5 mmol) in THF (400 mL), and the mixture was stirred at room temperature for 2 h. The reaction mixture was diluted with saturated sodium bicarbonate solution and water (100 mL, 1/2 v/v), added with ethyl acetate (200 mL), and two phases were separated. The aqueous phase was extracted with ethyl acetate (200 mL × 3). The organic phases were combined, dried over anhydrous sodium sulfate, filtered, and concentrated under reduced pressure. The residue was purified by silica gel column chromatography (ethyl acetate/petroleum ether = 1/4 to 1/0) to give target product 10f (23 g, oil), with a yield of 65%.
MS m/z (ESI): 176 [M+1-100]

Step 6

*tert*-Butyl 4-hydroxy-2-oxa-8-azaspiro[4.5]decane-8-carboxylate (10g)

Sodium hydride (60%, 11.7 g, 292.6 mmol) was suspended in THF (100 mL) and cooled to 0°C, and supplemented with dropwise 10f (23 g, 83.6 mmol) in THF (100 mL) and *p*-toluenesulfonyl chloride (16 g, 83.6 mmol) in THF (200 mL). The reaction mixture was stirred at 0°C for 2 h. Saturated ammonium chloride solution (50 mL) was added slowly, and the reaction mixture was stirred vigorously until no gas was produced. Saturated ammonium chloride solution (100 mL) and saturated brine (100 mL) were added, and the resulting mixture was extracted with ethyl acetate (200 mL × 3). The organic phases were combined, dried over anhydrous sodium sulfate, filtered, and concentrated under reduced pressure. The residue was purified by silica gel column chromatography (ethyl acetate/petroleum ether = 0/1 to 1/0) to give target product 10h (8.5 g, oil), with a yield of 39%.
MS m/z (ESI): 158 [M+1-100]

Step 7

*tert*-Butyl 4-oxo-2-oxa-8-azaspiro[4.5]decane-8-carboxylate (10h)

A reaction mixture of 10g (8.5 g, 32.9 mmol), Dess-Martin periodinane (27.4 g, 64.56 mmol) and dichloromethane

(200 mL) was stirred at 0°C for 2 h. Saturated sodium bicarbonate solution and saturated sodium thiosulfate solution (100 mL, 1/1 v/v) were added, and the resulting mixture was stirred vigorously. Two phases were separated and the aqueous phase was extracted with dichloromethane (200 mL × 3). The organic phases were combined, dried over anhydrous sodium sulfate, filtered, and concentrated under reduced pressure. The residue was purified by silica gel column chromatography (ethyl acetate/petroleum ether = 2/3) to give target product 10h (6.2 g, oil), with a yield of 74%.

MS m/z (ESI): 156 [M+1-100]

Step 8

2-Oxa-8-azaspiro[4.5]decan-4-one (10i)

HCl in dioxane (4 M, 2 mL) was added into 10h (500 mg, 1.96 mmol) in dichloromethane (8 mL), and the mixture was stirred at room temperature for 1 h. The reaction mixture was desolventized under reduced pressure to give target product 10i (hydrochloride, 370 mg, solid), with a yield of 99%. The product was used directly in the next step without purification.

MS m/z (ESI): 156 [M+1]

Step 9

8-(5-Iodo-1-methyl-6-oxo-1,6-dihydropyrimidin-2-yl)-2-oxa-8-azaspi ro[4.5]decan-4-one (10j)

1c (100 mg, 0.37 mmol) was added into a mixture of 10i (hydrochloride, 106 mg, 0.555 mmol), potassium carbonate (153 mg, 1.11 mmol), and acetonitrile (4 mL), and the mixture was heated to 80°C and stirred for 16 h. The reaction mixture was cooled to room temperature, mixed with water (5 mL) and ethyl acetate (20 mL), and phase-separated. The aqueous phase was extracted with ethyl acetate (10 mL × 3). The organic phases were combined, dried over anhydrous sodium sulfate, filtered, and concentrated under reduced pressure. The residue was purified by silica gel column chromatography (ethyl acetate/petroleum ether = 1/4) to give target product 10j (70 mg, solid), with a yield of 49%.

MS m/z (ESI): 390 [M+1]

Step 10

(*R*)-N-((*S*)-8-(5-iodo-1-methyl-6-oxo-1,6-dihydropyrimidin-2-yl)-2-o xa-8-azaspiro[4.5]decan-4-yl)-2-methylpropane-2-sulfinamide (10k)

A reaction mixture of 10j (60 mg, 0.15 mmol), (*R*)-2-methylpropane-2-sulfinamide (36.3 mg, 0.3 mmol), tetraethyl titanate (137 mg, 0.6 mmol), and THF (5 mL) was heated to 90°C and stirred for 4 h. The mixture was cooled to 0°C, added with methanol (2 mL) and lithium borohydride in THF (2.0 M, 0.15 mL, 0.3 mmol) and stirred for 1 h. The reaction mixture was mixed with water (5 mL) and dichloromethane (20 mL), and two layers were separated. The aqueous phase was extracted with dichloromethane (10 mL × 3). The organic phases were combined, dried over anhydrous sodium sulfate, filtered, and concentrated under reduced pressure. The residue was purified by silica gel column chromatography (methanol/dichloromethane = 1/9) to give target product 10k (70 mg, solid), with a yield of 92%.

MS m/z (ESI): 495 [M+1]

Step 11

(*R*)-N-((*S*)-8-(5-((4-chloro-2-methyl-2H-indazole-5-yl)thio)-1-methyl -6-oxo-1,6-dihydropyrimidin-2-yl)-2-oxa-8-azaspiro[4.5]decan-4-yl)-2-me thylpropane-2-sulfinamide (101)

$Pd_2(dba)_3$ (12.8 mg, 0.014 mmol) was added into a mixture of 10k (70 mg, 0.14 mmol), 7e (37.4 mg, 0.17 mmol), DIPEA (54 mg, 0.42 mmol), XantPhos (8.1 mg, 0.014 mmol), and dioxane (4 mL), and the reaction mixture was heated to 100°C under nitrogen blanket and reacted for 2 h. The reaction mixture was cooled to room temperature, mixed with water (5 mL) and dichloromethane (20 mL), and two layers were separated. The aqueous phase was extracted with dichloromethane (10 mL × 3). The organic phases were combined, dried over anhydrous sodium sulfate, filtered, and desolventized under reduced pressure. The residue was purified by silica gel column chromatography (methanol/dichloromethane = 1/9) to give target product 101 (50 mg, solid), with a yield of 63%.

MS m/z (ESI): 565 [M+1]

Step 12

(*S*)-2-(4-amino-2-oxa-8-azaspiro[4.5]decan-8-yl)-5-((4-chloro-2-meth yl-2H-indazole-5-yl)thio)-3-methylpyrimidin-4(3*H*)-one (10)

101 (50 mg, 0.09 mmol) was dissolved in methanol (8 mL), added with HCl in dioxane (4.0 M, 2 mL), and stirred at room temperature for 2 h. The reaction mixture was concentrated under reduced pressure, and the residue was purified by preparative RP-HPLC to give target product 10 (5.8 mg, solid), with a yield of 14%.
MS m/z (ESI): 461 [M+1]
$^1$H NMR (400 MHz, DMSO-$d_6$) δ 8.43 (s, 1H), 7.91 (s, 1H), 7.51 (d, *J* = 9.0 Hz, 1H), 6.99 (d, *J* = 9.0 Hz, 1H), 4.18 (s, 3H), 4.02 - 3.95 (m, 1H), 3.69 (d, *J* = 8.5 Hz, 1H), 3.63 (d, *J* = 8.5 Hz, 1H), 3.39 (s, 3H), 3.15 - 3.13 (m, 1H), 3.08 - 3.00 (m, 2H), 1.84 - 1.76 (m, 1H), 1.72 - 1.65 (m, 1H), 1.54 - 1.43 (m, 2H).

Example 11

2-((3*S*,4*S*)-4-amino-3-methyl-2-oxa-8-azaspiro[4.5]decan-8-yl)-5-((4-chloro-2-methyl-2H-indazole-5-yl)thio)-3-methylpyrimidin-4(3*H*)-one formate

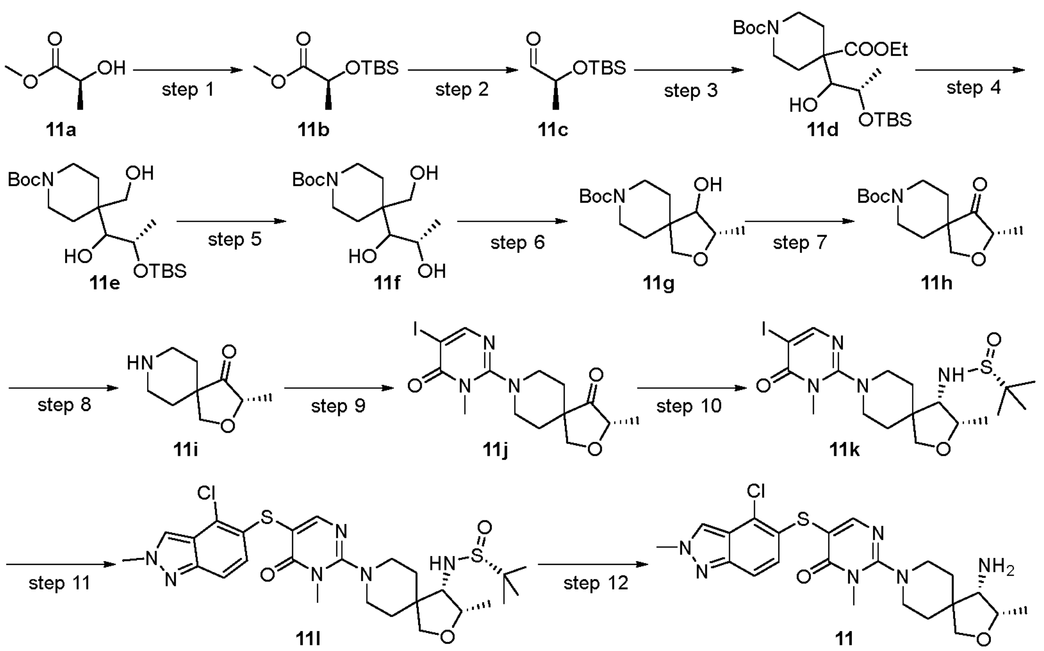


Step 1

Methyl (*S*)-2-((tert-butyldimethylsilyl)oxy)propanoate (11b)

Methyl (*S*)-2-hydroxypropanoate 11a (70 g, 672 mmol) was added dropwise into imidazole (68.5 g, 1008 mmol) in dichloromethane (800 mL) at 0°C, *tert*-butyldimethylsilyl chloride (120 g, 807 mmol) was added slowly, and the reaction mixture was stirred at room temperature for 16 h. The reaction mixture was diluted with water (600 mL) and extracted with dichloromethane (500 mL × 2). The organic phases were combined, washed with dilute hydrochloric acid (1 N, 300 mL × 3), dried over anhydrous sodium sulfate, filtered, and concentrated under reduced pressure to give target product 11b (150 g, oil), with a yield of 99%.
$^1$H NMR (400 MHz, $CDCl_3$) δ 4.32 (q, *J* = 6.7 Hz, 1H), 3.70 (s, 3H), 1.38 (d, *J* = 6.8 Hz, 3H), 0.88 (s, 9H), 0.08 (s, 3H), 0.05 (s, 3H).

Step 2

(*S*)-2-((*tert*-butyldimethylsilyl)oxy)propionaldehyde (11c)

11b (151.2 g, 693 mmol) in dichloromethane (1,200 mL) was cooled to -78°C, diisobutylaluminium hydride in *n*-hexane (1 M, 832 mL, 832 mmol) was added dropwise, and the reaction mixture was warmed up to -40°C and stirred for 1 h. The reaction mixture was poured into saturated potassium sodium tartrate solution (140 mL), mixed with ether (100 mL), and stirred at room temperature for 2 h. The two layers were separated, and the aqueous phase was extracted with ether. The organic phases were combined, washed with saturated brine, dried over anhydrous sodium sulfate, filtered, and concentrated under reduced pressure. The residue was purified by silica gel column chromatography (ethyl acetate/petroleum ether = 4/1) to give target product 11c (62.3 g, oil), with a yield of 48%.
$^1$H NMR (400 MHz, $CDCl_3$) $\delta$ 9.60 (s, 1H), 4.09 (q, *J* = 6.8 Hz, 1H), 1.27 (d, *J* = 6.9 Hz, 3H), 0.91 (s, 9H), 0.10 (s, 3H), 0.08 (s, 3H).

Step 3

1-*tert*-Butyl 4-ethyl 4-(2*S*)-2-((*tert*-butyldimethylsilyl)oxy)-1-hydroxypropyl)piperidine-1,4-di carboxylate (11d)

1-*tert*-Butyl 4-ethylpiperidine-1,4-dicarboxylate (72.8 g, 283.5 mmol) in THF (50 mL) was added dropwise into a solution of lithium diisopropylamide in THF (1 M, 425 mL, 425 mmol) and THF (800 mL) at -50°C, and the reaction mixture was stirred at -10°C for 1 h. 11c (53.3 g, 283.5 mmol) was added, and stirred at -10°C for 1 h. The reaction mixture was warmed to 0°C and stirred for 1 h, and then stirred at room temperature for 1 h. A mixture of saturated sodium bicarbonate solution and water (400 mL, 1/4 v/v) was added, ethyl acetate (500 mL) was added, and the resulting mixture was separated. The aqueous phase was extracted with ethyl acetate (500 mL $\times$ 3). The organic phases were combined, dried over anhydrous sodium sulfate, filtered, and concentrated under reduced pressure to give target product 11d (120 g, oil, crude product). The product was used directly in the next step without purification.
MS m/z (ESI): 346 [M+1-100]

Step 4

*tert-Butyl* 4-(2*S*)-2-((*tert*-butyldimethylsilyl)oxy)-1-hydroxypropyl)-4-(hydroxymeth yl)piperidine-1-carboxylate (11e)

Lithium borohydride in THF (2 M, 270 mL, 539.2 mmol) was added into 11d (120 g, 269.6 mmol) in THF (1,200 mL), and the reaction mixture was stirred at room temperature for 16 h. After cooling to 0°C, the reaction was quenched with saturated sodium bicarbonate solution and water (600 mL, 1/2 v/v), and the mixture was diluted with ethyl acetate (800 mL) and filtered. Two layers of the filtrate were separated and the aqueous phase was extracted with ethyl acetate (500 mL $\times$ 3). The organic phases were combined, washed with saturated brine (300 mL $\times$ 3), dried over anhydrous sodium sulfate, filtered, and concentrated under reduced pressure to give target product 11e (90 g, oil, crude product). The product was used directly in the next step without purification.
MS m/z (ESI): 404 [M+1]

Step 5

*tert-Butyl* 4-((2*S*)-1,2-dihydroxypropyl)-4-(hydroxymethyl)piperidine-1-carboxylate (11f)

Tetrabutylammonium fluoride in THF (1 M, 303 mL, 303 mmol) was added into 11e (90 g, 223 mmol) in THF (1300 mL), and the reaction mixture was stirred at room temperature overnight. The reaction mixture was diluted with saturated sodium bicarbonate solution and water (800 mL, 1/2 v/v), ethyl acetate (800 mL) was added, two layers were seperated, and the aqueous phase was extracted with ethyl acetate (500 mL $\times$ 3). The organic phases were combined, washed with saturated brine (300 mL $\times$ 3), dried over anhydrous sodium sulfate, filtered, and concentrated under reduced pressure. The residue was purified by silica gel column chromatography (ethyl acetate/petroleum ether = 49/1) to give target product 11f (24.5 g, oil), with a yield of 26% in three steps.
MS m/z (ESI): 290 [M+1]

Step 6

*tert-Butyl* (3*S*)-4-hydroxy-3-methyl-2-oxa-8-azaspiro[4.5]decane-8-carboxylate (11g)

Sodium hydride (60%, 10.8 g, 270.0 mmol) was suspended in THF (600 mL) and cooled to 0°C. 11f (22.3 g, 77.1 mmol) in THF (40 mL) was added dropwise, and *p*-toluenesulfonyl chloride (14.6 g, 77.1 mmol) in THF (50 mL) was added. The reaction mixture was stirred at 0°C for 6 h. After cooling to -20°C, the reaction mixture was added with saturated ammonium chloride solution (10 mL) slowly, and stirred vigorously until no gas was produced. The mixture was extracted with ethyl acetate (300 mL × 3). The organic phases were combined, washed with saturated brine (150 mL × 3), dried over anhydrous sodium sulfate, filtered, and concentrated under reduced pressure. The residue was purified by silica gel column chromatography (ethyl acetate/petroleum ether = 1/1) to give target product 11g (12.1 g, oil), with a yield of 58%.

MS m/z (ESI): 172 [M+1-100]

Step 7

*tert-Butyl* (*S*)-3-methyl-4-oxo-2-oxa-8-azaspiro[4.5]decane-8-carboxylate (11 h)

Dess-Martin periodinane (37.8 g, 89.2 mmol) was added slowly into 11g (12.1 g, 44.6 mmol) in dichloromethane (140 mL) at 0°C, and the reaction mixture was stirred at room temperature for 16 h. Saturated sodium bicarbonate solution was added until the solution was neutral, and the solution was extracted with dichloromethane (150 mL × 3). The organic phases were combined, washed with saturated brine (80 mL × 3), dried over anhydrous sodium sulfate, filtered, and concentrated under reduced pressure. The residue was purified by silica gel column chromatography (ethyl acetate/petroleum ether = 3/7) to give target product 11h (8.36 g, oil), with a yield of 70%.

MS m/z (ESI): 170 [M+1-100]

Step 8

(*S*)-3-methyl-2-oxa-8-azaspiro[4.5]decan-4-one (11i)

11h (1.2 g, 4.46 mmol) was dissolved in HCl in dioxane (4 M, 20 mL), and stirred at room temperature for 2 h. The reaction mixture was desolventized under reduced pressure to give target product 11i (hydrochloride, 800 mg, solid), with a yield of 87%.

MS m/z (ESI): 170 [M+1]

Step 9

(*S*)-8-(5-iodo-1-methyl-6-oxo-1,6-dihydropyrimidin-2-yl)-3-methyl-2 -oxa-8-azaspiro[4.5]decan-4-one (11j)

11i (hydrochloride, 108 mg, 0.529 mmol) and potassium carbonate (199 mg, 1.44 mmol) were added into 2-chloro-5-iodo-3-methylpyrimidin-4(3H)-one 1c (130 mg, 0.481 mmol) in acetonitrile (20 mL), and the reaction mixture was heated to 80°C and stirred for 16 h. The reaction mixture was cooled to room temperature, mixed with water (50 mL), and extracted with ethyl acetate (30 mL × 3). The organic phases were combined, washed with saturated brine (20 mL × 3), dried over anhydrous sodium sulfate, filtered, and concentrated under reduced pressure. The residue was purified by silica gel column chromatography (ethyl acetate/petroleum ether = 1.3/1) to give target product 11j (180 mg, solid), with a yield of 92%.

MS m/z (ESI): 404 [M+1]

Step 10

(*R*)-N-((3*S*,4*S*)-8-(5-iodo-1-methyl-6-oxo-1,6-dihydropyrimidin-2-yl) -3-methyl-2-oxa-8-azaspiro[4.5]decan-4-yl)-2-methylpropane-2-sulfinamid e (11k)

(*R*)-2-methylpropane-2-sulfinamide (108 mg, 0.892 mmol) and tetraethyl titanate (813 mg, 3.59 mmol) were added into a solution of 11j (180 mg, 0.446 mmol) in THF (2 mL), heated to 90°C, and stirred for 16 h. After cooling to 0°C, the mixture was added with methanol (5 mL) and lithium borohydride in THF (2.0 M, 0.23 mL, 0.46 mmol) and stirred for 1 h. The reaction was quenched with saturated ammonium chloride solution, and the resulting mixture was filtered. The filtrate was extracted with ethyl acetate (25 mL × 3). The organic phases were combined, washed with

saturated brine (20 mL × 3), dried over anhydrous sodium sulfate, filtered, and concentrated under reduced pressure. The residue was purified by silica gel column chromatography (methanol/dichloromethane = 1/16) to give target product 11k (90 mg, solid), with a yield of 39%.

MS m/z (ESI): 509 [M+1]

Step 11

(*R*)-N-((3*S*,4*S*)-8-(5-((4-chloro-2-methyl-2H-indazole-5-yl)thio)-1-me thyl-6-oxo-1,6-dihydropyrimidin-2-yl)-3-methyl-2-oxa-8-azaspiro[4.5]dec an-4-yl)-2-methylpropane-2-sulfinamide (111)

7e (60 mg, 0.27 mmol), DIPEA (60.7 mg, 0.471 mmol), XantPhos (14.2 mg, 0.023 mmol), and $Pd_2(dba)_3$ (14.4 mg, 0.0157 mmol) were added into 11k (80 mg, 0.157 mmol) in dioxane (5 mL). The reaction mixture was heated to 80°C under nitrogen protection and reacted for 4 h. The reaction mixture was cooled to room temperature and concentrated under reduced pressure. The residue was purified by silica gel column chromatography (methanol/dichloromethane = 1/13) to give target product 111 (55 mg, solid), with a yield of 60%.

MS m/z (ESI): 579 [M+1]

Step 12

2-((3*S*,4*S*)-4-amino-3-methyl-2-oxa-8-azaspiro[4.5]decan-8-yl)-5-((4-chloro-2-methyl-2H-indazole-5-yl)thio)-3-methylpyrimidin-4(3*H*)-one formate (11)

111 (55 mg, 0.095 mmol) was dissolved in methanol (5 mL), mixed with HCl in dioxane (4.0 M, 5 mL), and stirred at room temperature for 2 h. The reaction mixture was concentrated under reduced pressure, and the residue was purified by preparative RP-HPLC to give target product 11 (16.8 mg, solid), with a yield of 37%.

MS m/z (ESI): 475 [M+1]

$^1$H NMR (400 MHz, DMSO-$d_6$) δ 8.43 (s, 1H), 8.21 (s, 1H), 7.91 (s, 1H), 7.51 (d, *J* = 9.1 Hz, 1H), 6.99 (d, *J* = 9.0 Hz, 1H), 4.18 (s, 3H), 4.11 - 4.05 (m, 1H), 3.68 (d, *J* = 8.6 Hz, 1H), 3.51 (d, *J* = 8.6 Hz, 1H), 3.47 - 3.43 (m, 2H), 3.41 (s, 3H), 3.20 - 3.03 (m, 2H), 3.00 (d, *J* = 4.9 Hz, 1H), 1.89 - 1.82 (m, 1H), 1.77 - 1.72 (m, 1H), 1.62 - 1.53 (m, 2H), 1.11 (d, *J* = 6.4 Hz, 3H).

Example 12

2-((3*S*,4*S*)-4-amino-3-methyl-2-oxa-8-azaspiro[4.5]decan-8-yl)-5-((4-chloropyrazolo[1,5-*a*]pyridin-5-yl)thio)-3-methylpyrimidin-4(3*H*)-one formate

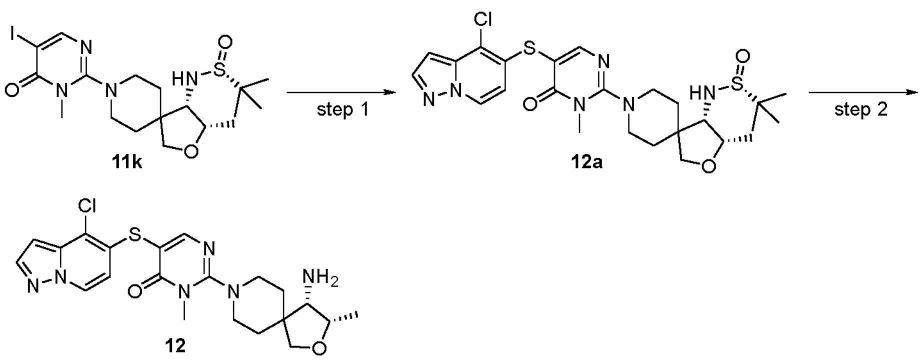


11k 12a 12

Step 1

(*R*)-N-((3*S*,4*S*)-8-(5-((4-chloropyrazolo[1,5-*a*]pyridin-5-yl)thio)-1-me thyl-6-oxo-1,6-dihydropyrimidin-2-yl)-3-methyl-2-oxa-8-azaspiro[4.5]dec an-4-yl)-2-methylpropane-2-sulfinamide (12a)

11k (140 mg, 0.28 mmol) was dissolved in dioxane (16 mL), and supplemented with 8h (60 mg, 0.29 mmol), DIPEA (71.5 mg, 0.56 mmol), $Pd_2(dba)_3$ (25.3 mg, 0.03 mmol), and XantPhos (32 mg, 0.06 mmol). The reaction mixture

was heated to 100°C under nitrogen blanket and reacted for 16 h. The reaction mixture was cooled to room temperature and desolventized under reduced pressure. The residue was purified by silica gel column chromatography (methanol/dichloromethane = 1/25) to give target product 12a (100 mg, solid), with a yield of 64%.

MS m/z (ESI): 565 [M+1]

Step 2

2-((3*S*,4*S*)-4-amino-3-methyl-2-oxa-8-azaspiro[4.5]decan-8-yl)-5-((4-chloropyrazolo[1,5-*a*]pyridin-5-yl)thio)-3-methylpyrimidin-4(3*H*)-one formate (12)

12a (86 mg, 0.166 mmol) was dissolved in methanol (5 mL), mixed with HCl in dioxane (4.0 M, 1 mL), and stirred at room temperature for 1 h. The reaction mixture was concentrated under reduced pressure, and the residue was purified by preparative RP-HPLC to give target product 12 (16.8 mg, solid), with a yield of 19%.

MS m/z (ESI): 461 [M+1]

$^1$H NMR (400 MHz, DMSO-$d_6$) δ 8.51 (d, *J* = 7.3 Hz, 1H), 8.26 (s, 1H), 8.17 (s, 1H), 8.04 (d, *J* = 2.1 Hz, 1H), 6.59 (d, *J* = 1.5 Hz, 1H), 6.48 (d, *J* = 7.3 Hz, 1H), 4.14 - 4.07 (m, 1H), 3.72 (d, *J* = 8.7 Hz, 1H), 3.57 - 3.51 (m, 3H), 3.39 (s, 3H), 3.21 - 3.07 (m, 2H), 3.06 (d, *J* = 4.9 Hz, 1H), 1.88 - 1.75 (m, 2H), 1.66 - 1.55 (m, 2H), 1.12 (d, *J* = 6.4 Hz, 3H).

Example 13

(*R*)-5-((1H-indazole-4-yl)thio)-6-amino-2-(1-amino-8-azaspiro[4.5]de can-8-yl)-3-methylpyrimidin-4(3*H*)-one formate

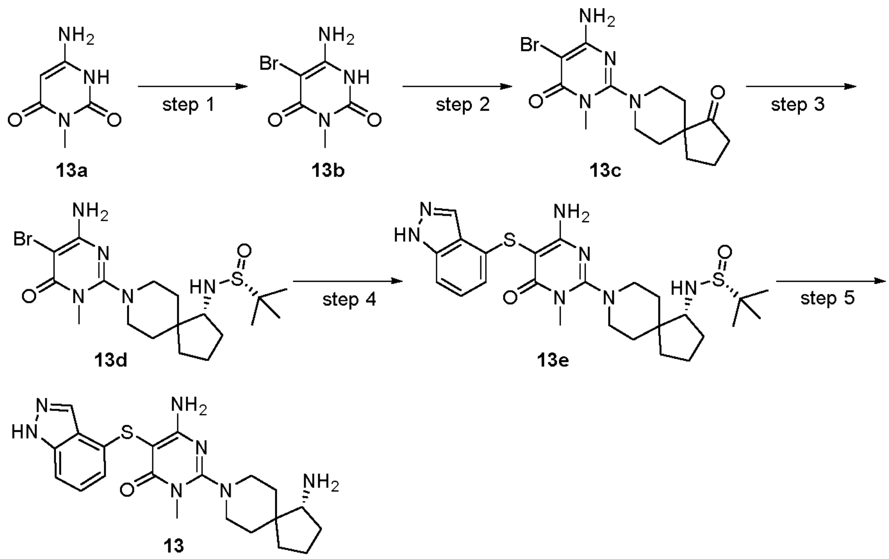


13a 13b 13c 13d 13e 13

Step 1

6-Amino-5-bromo-3-methylpyrimidine-2,4(1*H*,3*H*)-dione (13b)

6-Amino-3-methylpyrimidine-2,4(1*H*,3*H*)-dione (1.0 g, 7.08 mmol) was dissolved in DMF (6 mL), added with NBS (1.38 g, 7.8 mmol), and stirred at room temperature for 16 h. The reaction mixture was diluted with water (20 mL) and filtered. The filter cake was washed with water (5 mL × 2) and vacuum-dried to give target product 13b (1.0 g, solid), with a yield of 64%.

MS m/z (ESI): 220 [M+1]

Step 2

8-(4-Amino-5-bromo-1-methyl-6-oxo-1,6-dihydropyrimidin-2-yl)-8-a zaspiro[4.5]decan-1-one (13c)

13b (200 mg, 0.91 mmol) was dissolved in DMF (5 mL), added with 8-azaspiro[4.5]decan-1-one (167 mg, 1.1

mmol), Castros reagent (1.2 g, 2.73 mmol), and DBU (692 mg, 4.55 mmol), and stirred at room temperature overnight. The reaction mixture was diluted with ethyl acetate (50 mL), and washed with water (10 mL × 3) and saturated brine (10 mL). The organic phase was desolventized under reduced pressure and the residue was purified by silica gel column chromatography (ethyl acetate/petroleum ether = 7/3) to give target product 13c (170 mg, oil), with a yield of 53%.

MS m/z (ESI): 355 [M+1]

Step 3

(*R*)-N-((*R*)-8-(4-amino-5-bromo-1-methyl-6-oxo-1,6-dihydropyrimidi n-2-yl)-8-azaspiro[4.5]decan-1-yl)-2-methylpropane-2-sulfinamide (13d)

(*R*)-2-methylpropane-2-sulfinamide (110 mg, 0.9 mmol) and tetraethyl titanate (411 mg, 1.8 mmol) were added into 13c (160 mg, 0.45 mmol) in THF (15 mL), heated to 90°C, and stirred overnight. After cooling to room temperature, the mixture was added with methanol (5 mL) and lithium borohydride in THF (2.0 M, 0.5 mL) and stirred for 1 h. The reaction mixture was concentrated under reduced pressure, and the residue was purified by silica gel column chromatography (methanol/dichloromethane = 1/20) to give target product 13d (200 mg, oil), with a yield of 96%.

MS m/z (ESI): 460 [M+1]

Step 4

(*R*)-N-((*R*)-8-(5-((1H-indazole-4-yl)thio)-4-amino-1-methyl-6-oxo-1, 6-dihydropyrimidin-2-yl)-8-azaspiro[4.5]decan-1-yl)-2-methylpropane-2-s ulfinamide (13e)

13d (100 mg, 0.22 mmol) was dissolved in dioxane (10 mL), and supplemented with sodium 1H-indazole-4-thiolate 3c (75 mg, 0.44 mmol), DIPEA (84 mg, 0.66 mmol), $Pd_2(dba)_3$ (40 mg, 0.044 mmol), and XantPhos (51 mg, 0.088 mmol). The reaction mixture was heated to 100°C under nitrogen blanket and stirred overnight. The reaction mixture was cooled to room temperature and desolventized under reduced pressure. The residue was purified by preparative thin-layer chromatography to give target product 13e (30 mg, solid), with a yield of 26%.

MS m/z (ESI): 530 [M+1]

Step 5

(*R*)-5-((1H-indazole-4-yl)thio)-6-amino-2-(1-amino-8-azaspiro[4.5]de can-8-yl)-3-methylpyrimidin-4(3*H*)-one formate (13)

13e (30 mg, 0.056 mmol) was dissolved in methanol (2 mL), added with HCl in dioxane (4.0 M, 2 mL), and stirred at room temperature for 1 h. The reaction mixture was concentrated under reduced pressure, and the residue was purified by preparative RP-HPLC to give target product 13 (5 mg, solid), with a yield of 21%.

MS m/z (ESI): 426 [M+1]

$^1H$ NMR (400 MHz, $CD_3OD$) δ 8.54 (s, 1H), 8.12 (s, 1H), 7.30 (d, *J* = 8.3 Hz, 1H), 7.20 (t, *J* = 7.7 Hz, 1H), 6.78 (d, *J* = 7.0 Hz, 1H), 3.65 - 3.57 (m, 2H), 3.42 (s, 3H), 3.27 - 3.25 (m, 1H), 3.09 (t, *J* = 12.3 Hz, 2H), 2.22 - 2.17 (m, 1H), 1.85 - 1.72 (m, 7H), 1.54 (t, *J* = 13.5 Hz, 2H).

Example 14

6-Amino-2-((3*S*,4*S*)-4-amino-3-methyl-2-oxa-8-azaspiro[4.5]decan-8-yl)-5-((4-chloro-2-methyl-2H-indazole-5-yl)thio)-3-methylpyrimidin-4(3*H* )-one formate

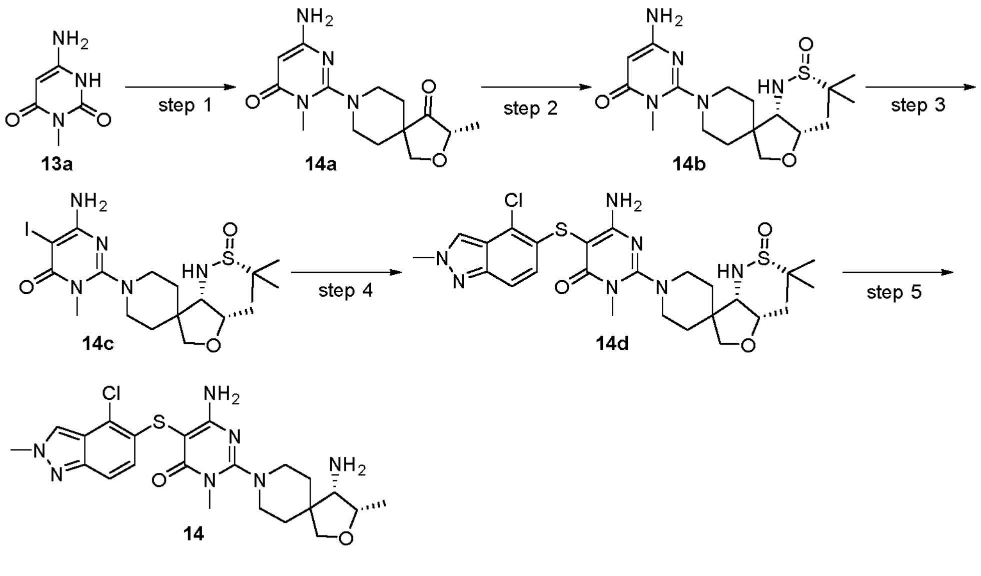

13a 14a 14b 14c 14d 14

Step 1

(*S*)-8-(4-amino-1-methyl-6-oxo-1,6-dihydropyrimidin-2-yl)-3-methyl-2-oxa-8-azaspiro[4.5]decan-4-one (14a)

6-Amino-3-methylpyrimidine-2,4(1*H*,3*H*)-dione 13a (400 mg, 2.8 mmol) was dissolved in DMF (25 mL), added with 11i (576 mg, 2.8 mmol), Castros reagent (6.18 g, 14 mmol), and DBU (1.29 g, 8.5 mmol), and stirred at room temperature overnight. The reaction was quenched with water (60 mL), and the reaction mixture was extracted with a mixed solvent of dichloromethane and methanol (10/1 v/v, 50 mL × 3). The organic phases were combined, washed with saturated brine (20 mL × 3), dried over anhydrous sodium sulfate, and desolventized under reduced pressure. The residue was purified by silica gel column chromatography (methanol/dichloromethane = 1/9) to give target product 14a (700 mg, oil), with a yield of 84%.

MS m/z (ESI): 293 [M+1]

Step 2

(*R*)-N-((3*S*,4*S*)-8-(4-amino-1-methyl-6-oxo-1,6-dihydropyrimidin-2-y l)-3-methyl-2-oxa-8-azaspiro[4.5]decan-4-yl)-2-methylpropane-2-sulfinam ide (14b)

(*R*)-2-Methylpropane-2-sulfinamide (557 mg, 4.6 mmol) and tetraethyl titanate (2.06 g, 9.2 mmol) were added into 14a (700 mg, 2.3 mmol) in THF (30 mL), and the reaction mixture was heated to 90°C and stirred for 16 h. After cooling to 0°C, the mixture was added with methanol (10 mL) and lithium borohydride in THF (2.0 M, 1.35 mL) and stirred for 1 h. The reaction was quenched with ammonium chloride solution, the mixture was filtered, and the filtrate was extracted with ethyl acetate (25 mL × 3). The organic phases were combined, dried over anhydrous sodium sulfate, filtered, and concentrated under reduced pressure. The residue was purified by silica gel column chromatography (methanol/dichloromethane = 1/10) to give target product 14b (80 mg, solid), with a yield of 8.7%.

MS m/z (ESI): 398 [M+1]

Step 3

(*R*)-N-((3*S*,4*S*)-8-(4-amino-5-iodo-1-methyl-6-oxo-1,6-dihydropyrimidin-2-yl)-3-methyl-2-oxa-8-azas piro[4.5]decan-4-yl)-2-methylpropane-2-sulfinamide (14c)

14b (80 mg, 0.2 mmol) was dissolved in acetonitrile (10 mL), added with NIS (54.2 mg, 0.24 mmol), and stirred at room temperature for 1.5 h. The reaction mixture was desolventized under reduced pressure and the residue was purified by silica gel column chromatography (methanol/dichloromethane = 1/12) to give target product 14c (100 mg, solid), with a yield of 95%.

MS m/z (ESI): 524 [M+1]

Step 4

(*R*)-N-((3*S*,4*S*)-8-(4-amino-5-((4-chloro-2-methyl-2H-indazole-5-yl)t hio)-1-methyl-6-oxo-1,6-dihydropyrimidin-2-yl)-3-methyl-2-oxa-8-azaspir o[4.5]decan-4-yl)-2-methylpropane-2-sulfinamide (14d)

14c (50 mg, 0.095 mmol) was dissolved in dioxane (6 mL), and supplemented with 7e (22.8 mg, 0.114 mmol), DIPEA (36.7 mg, 0.285 mmol), $Pd_2(dba)_3$ (8.6 mg, 0.0095 mmol), and XantPhos (8.2 mg, 0.014 mmol). The reaction mixture was heated to 80°C under nitrogen blanket and stirred for 16 h. After cooling to room temperature, the reaction mixture was diluted with ethyl acetate (100 mL) and washed with saturated brine. The organic phase was dried over anhydrous sodium sulfate and desolventized under reduced pressure. The residue was purified by silica gel column chromatography (methanol/dichloromethane = 1/14) to give target product 14d (46 mg, solid), with a yield of 82%.
MS m/z (ESI): 594 [M+1]

Step 5

6-Amino-2-((3*S*,4*S*)-4-amino-3-methyl-2-oxa-8-azaspiro[4.5]decan-8-yl)-5-((4-chloro-2-methyl-2H-indazole-5-yl)thio)-3-methylpyrimidin-4(3*H* )-one formate (14)

14d (46 mg, 0.077 mmol) was dissolved in methanol (5 mL), added with HCl in dioxane (4.0 M, 5 mL), and stirred at room temperature for 2 h. The reaction mixture was concentrated under reduced pressure and the residue was purified by preparative RP-HPLC to give target product 14 (6.5 mg, solid), with a yield of 17%.
MS m/z (ESI): 490 [M+1]
$^1$H NMR (400 MHz, DMSO-$d_6$) δ 8.33 (s, 1H), 8.20 (s, 1H), 7.46 (d, *J* = 9.0 Hz, 1H), 6.76 (d, *J* = 9.0 Hz, 1H), 4.15 (s, 3H), 4.10 - 4.06 (m, 1H), 3.69 (d, *J* = 8.5 Hz, 1H), 3.52 (d, *J* = 8.5 Hz, 1H), 3.39 - 3.37 (m, 2H), 3.29 (s, 3H), 3.11 - 2.97 (m, 3H), 1.85 - 1.71 (m, 2H), 1.63 - 1.54 (m, 2H), 1.11 (d, *J* = 6.4 Hz, 3H).

Example 15

6-Amino-2-((3*S*,4*S*)-4-amino-3-methyl-2-oxa-8-azaspiro[4.5]decan-8-yl)-5-((4-chloropyrazolo[1,5-*a*]pyridine-5-yl)thio)-3-methylpyrimidin-4(3 *H*)-one formate

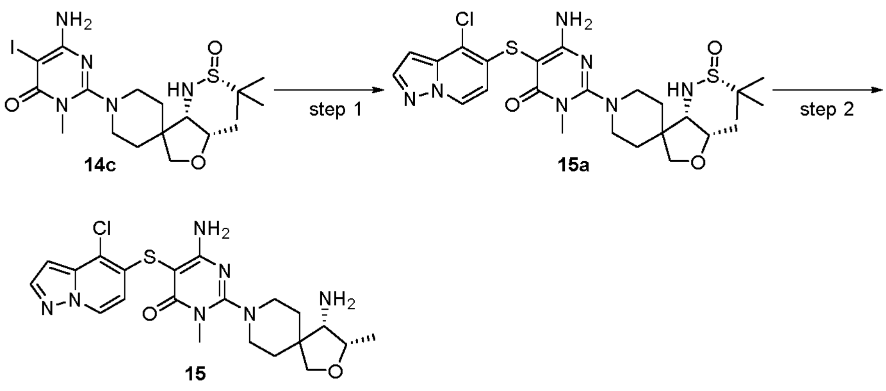


14c 15a 15

Step 1

(*R*)-N-((3*S*,4*S*)-8-(4-amino-5-((4-chloropyrazolo[1,5-*a*]pyridin-5-yl)th io)-1-methyl-6-oxo-1,6-dihydropyrimidin-2-yl)-3-methyl-2-oxa-8-azaspiro [4.5]decan-4-yl)-2-methylpropane-2-sulfinamide (15a)

14c (40 mg, 0.076 mmol) was dissolved in dioxane (4 mL), and supplemented with 8h (31.3 mg, 0.152 mmol), DIPEA (29.4 mg, 0.228 mmol), $Pd_2(dba)_3$ (6.9 mg, 0.0076 mmol), and XantPhos (6.5 mg, 0.0114 mmol). The reaction mixture was heated to 80°C under nitrogen blanket and stirred for 16 h. After cooling to room temperature, the reaction mixture was diluted with ethyl acetate (100 mL) and washed with saturated brine. The organic phase was dried over anhydrous sodium sulfate and desolventized under reduced pressure. The residue was purified by silica gel column chromatography (methanol/dichloromethane = 1/17) to give target product 15a (35 mg, solid), with a yield of 79%.

MS m/z (ESI): 580 [M+1]

Step 2

6-Amino-2-((3*S*,4*S*)-4-amino-3-methyl-2-oxa-8-azaspiro[4.5]decan-8-yl)-5-((4-chloropyrazolo[1,5-*a*]pyridine-5-yl)thio)-3-methylpyrimidin-4(3 *H*)-one formate (15)

15a (35 mg, 0.06 mmol) was dissolved in methanol (5 mL), added with HCl in dioxane (4.0 M, 5 mL), and stirred at room temperature for 2 h. The reaction mixture was concentrated under reduced pressure and the residue was purified by preparative RP-HPLC to give target product 15 (4.2 mg, solid), with a yield of 15%.
MS m/z (ESI): 476 [M+1]
$^1$H NMR (400 MHz, $CD_3OD$) δ 8.52 (s, 1H), 8.28 (d, *J* = 8.0 Hz, 1H), 7.93 (d, *J* = 2.3 Hz, 1H), 6.55 (d, *J* = 1.7 Hz, 1H), 6.45 (d, *J* = 7.3 Hz, 1H), 4.28 - 4.23 (m, 1H), 3.89 (d, *J* = 8.9 Hz, 1H), 3.77 (d, *J* = 8.9 Hz, 1H), 3.63 - 3.56 (m, 2H), 3.43 (s, 3H), 3.24 - 3.02 (m, 3H), 1.97 - 1.90 (m, 2H), 1.79 - 1.69 (m, 2H), 1.26 (d, *J* = 6.5 Hz, 3H).

Example 16

2-(4-(Aminomethyl)-4-methylpiperidin-1-yl)-5-((4-chloro-2-methyl-2 H-indazole-5-yl)thio)-3-methylpyrimidin-4(3*H*)-one formate

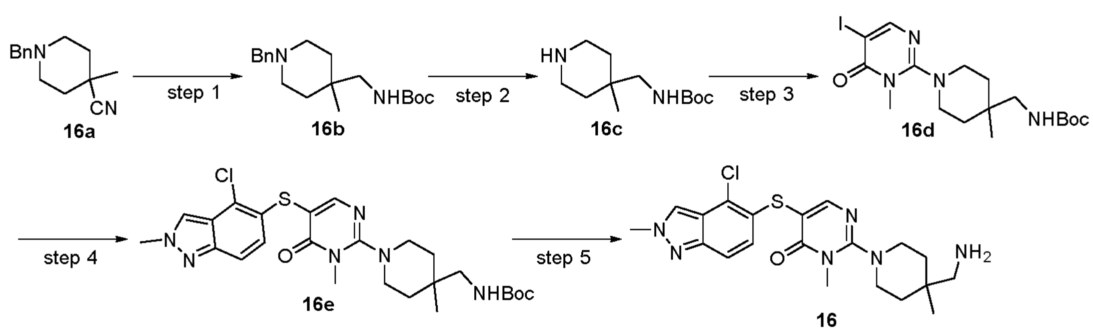


Step 1

*tert*-Butyl ((1-benzyl-4-methylpiperidin-4-yl)methyl)carbamate (16b)

A reaction mixture of 1-benzyl-4-methylpiperidine-4-carbonitrile 16a (2 g, 9.3 mmol), di-*tert*-butyl dicarbonate (6.1 g, 27.9 mmol), nickel chloride hexahydrate (2.2 g, 9.3 mmol), and methanol (50 mL) was stirred at room temperature for 15 min. After cooling to 0°C, the reaction mixture was mixed with sodium borohydride (1.77 g, 46.5 mmol), warmed up to room temperature and stirred for 8 h. The reaction mixture was desolventized under reduced pressure, and the residue was suspended in dichloromethane and filtered. The filtrate was desolventized under reduced pressure and the residue was purified by silica gel column chromatography (ethyl acetate/petroleum ether = 1/1) to give target product 16b (1.2 g, oil), with a yield of 40%.
MS m/z (ESI): 319 [M+1]

Step 2

*tert*-Butyl ((4-methylpiperidin-4-yl)methyl)carbamate (16c)

16b (1.2 g, 3.77 mmol) was dissolved in methanol (20 mL), added with palladium on carbon (10%, water content 55%, 1.2 g), and stirred at room temperature for 3 h under a hydrogen atmosphere. The reaction mixture was filtered, the filter cake was washed with methanol, and the filtrate was concentrated to give target product 16c (810 mg, oil), with a yield of 94%.
MS m/z (ESI): 229 [M+1]

Step 3

*tert-Butyl* ((1-(5-iodo-1-methyl-6-oxo-1,6-dihydropyrimidin-2-yl)-4-methylpiperidin -4-yl)methyl)carbamate (16d)

1c (150 mg, 0.55 mmol) was added into a mixture of 16c (150 mg, 0.66 mmol), potassium carbonate (228 mg, 1.65 mmol), and acetonitrile (10 mL), heated to 90°C, and stirred for 16 h. The reaction mixture was concentrated under reduced pressure, and the residue was purified by silica gel column chromatography (methanol/dichloromethane = 1/9) to give target product 16d (150 mg, solid), with a yield of 58%.

MS m/z (ESI): 463 [M+1]

Step 4

*tert-Butyl* ((1-(5-((4-chloro-2-methyl-2H-indazole-5-yl)thio)-1-methyl-6-oxo-1,6-dih ydropyrimidin-2-yl)-4-methylpiperidin-4-yl)methyl)carbamate (16e)

A reaction mixture of 16d (150 mg, 0.32 mmol), 7e (86 mg, 0.39 mmol), DIPEA (124 mg, 0.96 mmol), $Pd_2(dba)_3$ (29 mg, 0.032 mmol), XantPhos (18.5 mg, 0.032 mmol), and dioxane (4 mL) was heated to 90°C under nitrogen blanket and stirred for 3 h. The reaction mixture was desolventized under reduced pressure and the residue was purified by silica gel column chromatography (methanol/dichloromethane = 1/9) to give target product 16e (130 mg, solid), with a yield of 75%.

MS m/z (ESI): 533 [M+1]

Step 5

2-(4-(Aminomethyl)-4-methylpiperidin-1-yl)-5-((4-chloro-2-methyl-2 H-indazole-5-yl)thio)-3-methylpyrimidin-4(3*H*)-one formate (16)

16e (130 mg, 0.24 mmol) was dissolved in dichloromethane (5 mL), added with trifluoroacetic acid (1 mL), and stirred at room temperature for 1 h. The reaction mixture was concentrated under reduced pressure and the residue was purified by preparative RP-HPLC to give target product 16 (32.4 mg, solid), with a yield of 31%.

MS m/z (ESI): 433 [M+1]

$^1H$ NMR (400 MHz, DMSO-$d_6$) $\delta$ 8.43 (s, 1H), 8.37 (s, 1H), 7.91 (s, 1H), 7.51 (d, *J* = 9.0 Hz, 1H), 6.99 (d, *J* = 9.0 Hz, 1H), 4.18 (s, 3H), 3.40 - 3.36 (m, 5H), 3.17 (t, *J* = 10.2 Hz, 2H), 2.65 (s, 2H), 1.63 - 1.57 (m, 2H), 1.42 (d, *J* = 11.1 Hz, 2H), 1.01 (s, 3H).

Example 17

2-((3*S*,4*S*)-4-amino-3-methyl-2-oxa-8-azaspiro[4.5]decan-8-yl)-3-met hyl-5-((1-methyl-1H-pyrrolo[2,3-*b*]pyridin-4-yl)thio)pyrimidin-4(3*H*)-one formate

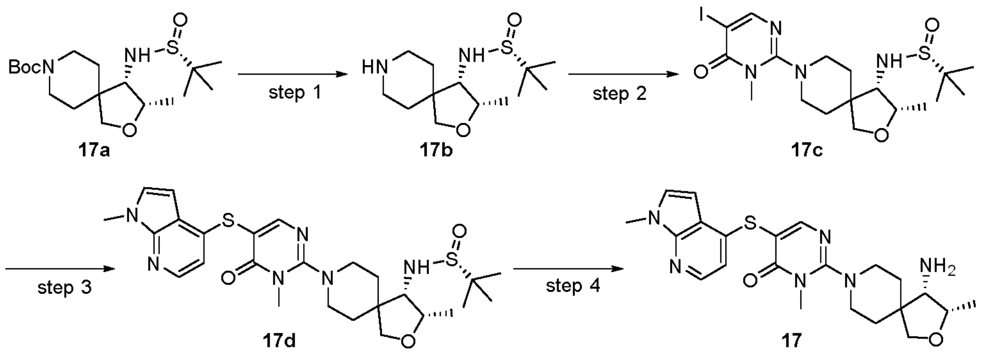

Step 1

(*R*)-2-methyl-N-((3*S*,4*S*)-3-methyl-2-oxa-8-azaspiro[4.5]decan-4-yl)p ropane-2-sulfinamide (17b)

*tert-Butyl* (3*S*,4*S*)-4-(((*R*)-tert-butylsulfinyl)amino)-3-methyl-2-oxa-8-azaspiro[4.5]d ecane-8-carboxylate 17a (300 mg, 0.8 mmol) was dissolved in dichloromethane (5 mL), added with trifluoroacetic acid (1 mL), and stirred at room temperature for 1 h. The reaction mixture was concentrated under reduced pressure to give target product 17b (350 mg, crude product). The product was used directly in the next step without purification.

MS m/z (ESI): 275 [M+1]

Step 2

(*R*)-N-((3*S*,4*S*)-8-(5-iodo-1-methyl-6-oxo-1,6-dihydropyrimidin-2-yl) -3-methyl-2-oxa-8-azaspiro[4.5]decan-4-yl)-2-methylpropane-2-sulfinamid e (17c)

A reaction mixture of 1c (216 mg, 0.8 mmol), 17b (crude product, 350 mg, 0.8 mmol), potassium carbonate (562 mg, 4.05 mmol), and acetonitrile (10 mL) was heated to 80°C, and stirred for 16 h. The reaction mixture was concentrated under reduced pressure and the residue was purified by silica gel column chromatography (methanol/dichloromethane = 1/9) to give target product 17c (360 mg, solid), with a yield of 89%.

MS m/z (ESI): 509 [M+1]

Step 3

(*R*)-2-methyl-N-((3*S*,4*S*)-3-methyl-8-(1-methyl-5-((1-methyl-1H-pyrr olo[2,3-*b*]pyridin-4-yl)thio)-6-oxo-1,6-dihydropyrimidin-2-yl)-2-oxa-8-aza spiro[4.5]decan-4-yl)propane-2-sulfinamide (17d)

A reaction mixture of 17c (180 mg, 0.35 mmol), 2c (70 mg, 0.42 mmol), DIPEA (135 mg, 1.05 mmol), $Pd_2(dba)_3$ (32 mg, 0.035 mmol), XantPhos (20 mg, 0.035 mmol), and dioxane (5 mL) was heated to 90°C under nitrogen blanket and stirred for 2 h. The reaction mixture was cooled to room temperature and desolventized under reduced pressure. The residue was purified by silica gel column chromatography (methanol/dichloromethane = 1/9) to give target product 17d (160 mg, solid), with a yield of 83%.

MS m/z (ESI): 545 [M+1]

Step 4

2-((3*S*,4*S*)-4-amino-3-methyl-2-oxa-8-azaspiro[4.5]decan-8-yl)-3-met hyl-5-((1-methyl-1H-pyrrolo[2,3-*b*]pyridin-4-yl)thio)pyrimidin-4(3*H*)-one formate (17)

17d (160 mg, 0.29 mmol) was dissolved in methanol (4 mL), added with HCl in dioxane (4.0 M, 2 mL), and stirred at room temperature for 2 h. The reaction mixture was concentrated under reduced pressure and the residue was purified by preparative RP-HPLC to give target product 17 (36 mg, solid), with a yield of 28%.

MS m/z (ESI): 441 [M+1]

$^1$H NMR (400 MHz, DMSO-$d_6$) $\delta$ 8.22 (s, 1H), 8.14 (s, 1H), 8.03 (d, *J* = 5.1 Hz, 1H), 7.51 (d, *J* = 3.5 Hz, 1H), 6.58 (d, *J* = 5.1 Hz, 1H), 6.44 (d, *J* = 3.5 Hz, 1H), 4.12 - 4.04 (m, 1H), 3.80 (s, 3H), 3.70 (d, *J* = 8.6 Hz, 1H), 3.53 - 3.48 (m, 3H), 3.40 (s, 3H), 3.19 - 3.08 (m, 2H), 3.02 (d, *J* = 4.9 Hz, 1H), 1.88 - 1.84 (m, 2H), 1.64 - 1.54 (m, 2H), 1.11 (d, *J* = 6.4 Hz, 3H).

Example 18

5-((1H-pyrrolo[2,3-*b*]pyridin-4-yl)thio)-2-((3*S*,4*S*)-4-amino-3-methyl-2-oxa-8-azaspiro[4.5]decan-8-yl)-3-methylpyrimidin-4(3*H*)-one formate

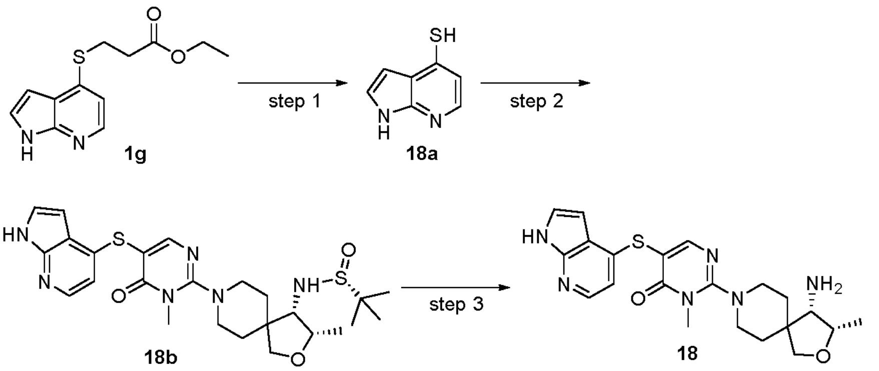


1g 18a 18b 18

Step 1

1H-pyrrolo[2,3-*b*]pyridine-4-thiol (18a)

Potassium *tert*-butoxide (224 mg, 2 mmol) was added into 1g (250 mg, 1 mmol) in THF (5 mL), and the reaction mixture was stirred at room temperature for 2 h. The reaction was quenched with water and the resulting mixture was washed with ethyl acetate. The aqueous phase was acidized with hydrochloric acid (6 N) to pH=4 and extracted with ethyl acetate. The organic phase was dried over anhydrous sodium sulfate, the drying agent was removed by filtration, and the filtrate was desolventized under reduced pressure to give target product 18a (100 mg, solid), with a yield of 67%.
MS m/z (ESI): 151 [M+1]

Step 2

(*R*)-N-((3*S*,4*S*)-8-(5-((1H-pyrrolo[2,3-*b*]pyridin-4-yl)thio)-1-methyl-6 -oxo-1,6-dihydropyrimidin-2-yl)-3-methyl-2-oxa-8-azaspiro[4.5]decan-4-y l)-2-methylpropane-2-sulfinamide (18b)

A reaction mixture of 17c (180 mg, 0.35 mmol), 18a (63 mg, 0.42 mmol), DIPEA (135 mg, 1.05 mmol), $Pd_2(dba)_3$ (32 mg, 0.035 mmol), XantPhos (20 mg, 0.035 mmol), and dioxane (5 mL) was heated to 90°C under nitrogen blanket and stirred for 2 h. The reaction mixture was cooled to room temperature and desolventized under reduced pressure. The residue was purified by silica gel column chromatography (methanol/dichloromethane = 1/9) to give target product 18b (150 mg, solid), with a yield of 80%.
MS m/z (ESI): 531 [M+1]

Step 3

5-((1H-pyrrolo[2,3-*b*]pyridin-4-yl)thio)-2-((3*S*,4*S*)-4-amino-3-methyl-2-oxa-8-azaspiro[4.5]decan-8-yl)-3-methylpyrimidin-4(3*H*)-one formate (18)

18b (150 mg, 0.28 mmol) was dissolved in methanol (4 mL), added with HCl in dioxane (4.0 M, 2 mL), and stirred at room temperature for 2 h. The reaction mixture was concentrated under reduced pressure and the residue was purified by preparative RP-HPLC to give target product 18 (37.9 mg, solid), with a yield of 31%.
MS m/z (ESI): 427 [M+1]
$^1$H NMR (400 MHz, DMSO-$d_6$) $\delta$ 11.73 (s, 1H), 8.25 (s, 1H), 8.14 (s, 1H), 7.99 (d, *J* = 5.1 Hz, 1H), 7.47 - 7.45 (m, 1H), 6.54 (d, *J* = 5.1 Hz, 1H), 6.42 (d, *J* = 2.3 Hz, 1H), 4.15 - 4.05 (m, 1H), 3.71 (d, *J* = 8.5 Hz, 1H), 3.52 - 3.48 (m, 3H), 3.41 (s, 3H), 3.24 - 3.05 (m, 3H), 1.89 - 1.75 (m, 2H), 1.65 - 1.55 (m, 2H), 1.12 (d, *J* = 6.2 Hz, 3H).

Example 19

2-((3*S*,4*S*)-4-amino-3-methyl-2-oxa-8-azaspiro[4.5]decan-8-yl)-5-((4-chloro-2-methyl-2H-indazole-5-yl)thio-3-methyl-$d_3$)pyrimidin-4(3*H*)-one

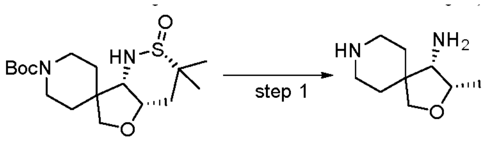


19a 19b

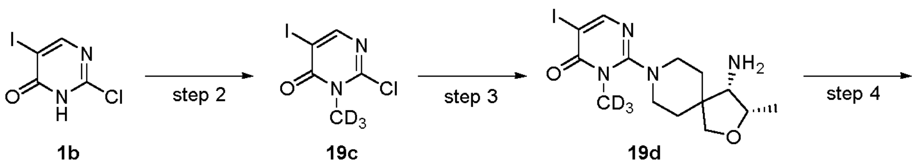


1b 19c 19d

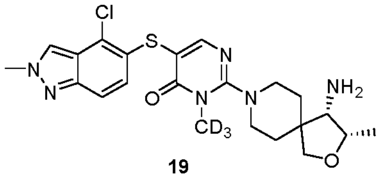

19

Step 1

(3*S*,4*S*)-3-methyl-2-oxa-8-azaspiro[4.5]decan-4-amine (19b) *tert-Butyl* (3*S*,4*S*)-4-(((*R*)-tert-butylsulfinyl)amino)-3-methyl-2-oxa-8-azaspiro[4.5]d ecane-8-carboxylate 19a (95.0 g, 254 mmol) and HCl in methanol (4 M, 1 L) were stirred at room temperature for 4 h. The reaction mixture was concentrated under reduced pressure. The residue was dissolved in methanol (500 mL), and to which was added slowly a mixed solvent of petroleum ether and ethyl acetate (1/1, 4 L). The resulting mixture was stirred at room temperature for 2 h. After filtration, the filter cake was dried to give target product 19b (64.1 g, solid, dihydrochloride), with a yield of >100%.

MS m/z (ESI): 171 [M+1]

$^1$H NMR (400 MHz, DMSO-$d_6$) δ 9.12 (brs, 2H), 8.33 (s, 3H), 4.25 - 4.19 (m, 1H), 3.82 (d, *J* = 9.1 Hz, 1H), 3.64 (d, *J* = 9.1 Hz, 1H), 3.47 (brs, 1H), 3.27 - 3.24 (m, 1H), 3.19 - 3.16 (m, 1H), 2.94 - 2.85 (m, 2H), 2.03 - 1.85 (m, 2H), 1.79 - 1.67 (m, 2H), 1.23 (d, *J* = 6.5 Hz, 3H).

Step 2

2-Chloro-5-iodo-3-(methyl-$d_3$)pyrimidin-4(3*H*)-one (19c)

DBU (3.85 g, 25.3 mmol) was added into 2-chloro-5-iodopyrimidin-4(3*H*)-one 1b (5.0 g, 19.5 mmol) in THF (100 mL), the resulting mixture was cooled to 0°C, and supplemented with iodomethane-$d_3$ (3.39 g, 23.4 mmol). The reaction mixture was heated to 50°C and stirred for 6 h. The reaction mixture was desolventized under reduced pressure. The residue was mixed with ethyl acetate (3.5 L), and washed with dilute hydrochloric acid (0.5 N, 1 L × ) and brine (800 mL × 2). The organic phase was dried over anhydrous sodium sulfate, filtered, and concentrated under reduced pressure. The residue was purified by silica gel column chromatography (ethyl acetate/petroleum ether = 1/19 to 1/4) to give target product 19c (3.3 g, solid), with a yield of 62%.

MS m/z (ESI): 274[M+1]

Step 3

2-((3*S*,4*S*)-4-amino-3-methyl-2-oxa-8-azaspiro[4.5]decan-8-yl)-5-iod o-3-(methyl-$d_3$)pyrimidin-4(3*H*)-one (19d)

A reaction mixture of 19c (1.8 g, 6.5 mmol), 19b (1.67 g, 6.9 mmol, dihydrochloride), cesium carbonate (9.5 g, 29.3 mmol), and acetonitrile (60 mL) was stirred at room temperature for 3 h under nitrogen blanket. The residue was purified by silica gel column chromatography (ethyl acetate/petroleum ether = 9/1) to give target product 19d (2.4 g, solid), with a yield of 91%.

MS m/z (ESI): 408[M+1]

Step 4

2-((3*S*,4*S*)-4-amino-3-methyl-2-oxa-8-azaspiro[4.5]decan-8-yl)-5-((4-chloro-2-methyl-2H-indazole-5-yl)thio)-3-(methyl-*$d_3$*)pyrimidin-4(3*H*)-one (19)

A reaction mixture of 7e (1.9 g, 9.0 mmol), 19d (2.3 g, 5.65 mmol), cuprous iodide (430 mg, 2.26 mmol), 1,10-phenanthroline (814 mg, 4.52 mmol), potassium phosphate (2.3 g, 11.3 mmol), and dioxane (50 mL) was heated to 100°C under nitrogen blanket and stirred overnight. The reaction mixture was cooled to room temperature and desolventized under reduced pressure. The residue was purified by silica gel column chromatography (methanol/ dichloromethane = 1/9). The resulting crude product (2.5 g) was mixed with acetonitrile (25 mL), and stirred at 90°C for 3 h and at room temperature for 16 h. After filtration, the filter cake was vacuum-dried at 40°C for 4 h to give target product 19 (1.47 g, solid), with a yield of 54%.

MS m/z (ESI): 478[M+1]

$^1$H NMR (400 MHz, DMSO-$d_6$) δ 8.42 (s, 1H), 7.91 (s, 1H), 7.50 (dd, *J* = 9.0, 0.9 Hz, 1H), 6.98 (d, *J* = 9.0 Hz, 1H), 4.17 (s, 3H), 4.05 - 4.02 (m, 1H), 3.64 (d, *J* = 8.5 Hz, 1H), 3.47 (d, *J* = 8.5 Hz, 1H), 3.48 - 3.37 (m, 2H), 3.15 - 3.05 (m, 2H), 2.90 (d, *J* = 5.1 Hz, 1H), 1.86 - 1.80 (m, 1H), 1.74 - 1.68 (m, 1H), 1.59 - 1.50 (m, 2H), 1.38 (brs, 2H), 1.07 (d, *J* = 6.4 Hz, 3H).

Biological experiments

SHP2 activity inhibition measurement

The effect of the compound of the present invention on SHP2 activity is evaluated by rapid fluorescence detection method using the surrogate substrate DiFMUP.

The experimental method is outlined as follows:
Recombinant human SHP2 full-length protein was expressed and purified by the protein purification and identification platform of Tsinghua University; diphosphopeptide (H2N-LN(pY)IDLDLV-(dPEG8)LST(pY)ASINFQK-amide) was synthesized by Nanjing GenScript Biotechnology Co., Ltd.; surrogate substrate DiFMUP was purchased from Thermo Fisher Scientific (Cat. No. D6567); reaction buffer contained the following components: 60 mM HEPES (pH 7.2), 75 mM NaCl, 75 mM KCl, 1 mM EDTA, 0.05% TWEEN 20, and 5 mM DTT.

The compound was dissolved in DMSO (Sigma, Cat. No. D5879) and diluted to 100 μM. The resulting solution was serially diluted 4-fold with DMSO to a minimum concentration of 6.1 nM, and each concentration point was diluted 25-fold with the reaction buffer.

10 μL of compound solution and 10 μL of 0.25 nM SHP2 protein solution were added to a 384-well microplate (Corning, Cat. No. 3575), mixed well, and incubated at room temperature for 30 min. 10 μL of 0.5 μM diphosphopeptide (dissolved in the reaction buffer) was added, mixed well, and incubated at room temperature for 30 to 60 min. Subsequently, 10 μL of 60 μM DiFMUP solution (dissolved in the reaction buffer) was added, mixed well, and let stand at room temperature. After 30 min, fluorescence signals were measured at 340 nm (excitation wavelength)/450 nm (emission wavelength) using a microplate reader (EnSpire, Perkin Elmer). The fluorescence intensity value was positively correlated with the degree of dephosphorylation of the substrate, thereby reflecting the catalytic activity of SHP2. In the experiment, protein-free group was used as a 100% inhibition group, and protein-added-but-compound-free group was used as a 0% inhibition group. An inhibition curve of the compound was plotted by XLfit software and the inhibitory $IC_{50}$ was calculated. Experimental results are shown in the table below.

| Compound Number | $IC_{50}$ (nM) |
|---|---|
| 1. | 23 |
| 2. | 20 |
| 3. | 20 |
| 4. | 126 |
| 5. | 801 |
| 6. | 877 |
| 7. | 12 |
| 8. | 8.6 |

(continued)

| Compound Number | $IC_{50}$ (nM) |
|---|---|
| 9. | 8.8 |
| 10. | 36 |
| 11. | 4.0 |
| 12. | 3.1 |
| 13. | 8.7 |
| 14. | 2.5 |
| 15. | 2.7 |
| 16. | 56 |
| 17. | 14 |
| 18. | 5.5 |
| 19. | 11 |

The compounds in the embodiments of the present invention have an inhibitory effect on SHP2 activity, with preferred $IC_{50}$ of less than 50 nM.

Determination of NCI-H358 cell proliferation inhibition

The effect of the compound of the present invention on proliferation of human non-small cell lung cancer cell line NCI-H358 is evaluated by luminescent cell viability assay.

The experimental method is outlined as follows:

The compound was dissolved in DMSO (Sigma, Cat. No. D5879) and diluted to 5 mM. The resulting solution was serially diluted 4-fold with DMSO to a minimum concentration of 0.31 μM, and each concentration point was diluted 50-fold with RPMI 1640 Medium (Thermo Fisher Scientific, Cat. No. 11995073). When the $IC_{50}$ value of the compound was low, the initial concentration of the compound was further reduced.

NCI-H358 cells (ATCC, Cat. No. CRL-5807) were cultured in RPMI 1640 Complete Medium [RPMI 1640 Medium was supplemented with 10% FBS (GBICO, Cat. No. 10099-141) and 100 units/mL Penicillin-Streptomycin (Thermo Fisher Scientific, Cat. No. 15140122)]. The cells (15,000 cells/mL) were seeded in 90 μL of Complete Medium on a 96-well plate. After culturing overnight, 10 μL of compound solution was added to each well and cultured at 37°C in a 5% $CO_2$ incubator for 5 days. According to the instruction from the CellTilter-Glo (CTG) kit (Promega, Cat. No. G7572), the cell culture plate was removed from incubator and equilibrated to room temperature. The cells were lysed fully with 50 μL of CTG reagent and placed at room temperature for 10 min. A luminescence signal was read by the microplate reader (EnVision, Perkin Elmer). In the experiment, the group containing 10 μM positive control RMC-4550 was used as a negative control (100% inhibition), and the group containing 0.2% DMSO was used as a positive control (0% inhibition). An inhibition curve of the compound was plotted by XLfit software and the inhibitory $IC_{50}$ was calculated. Experimental results are shown in the table below.

| Compound Number | $IC_{50}$ (nM) |
|---|---|
| 1 | 310 |
| 2 | 383 |
| 3 | 434 |
| 4 | 2,359 |
| 7 | 575 |
| 8 | 309 |
| 9 | 1,345 |
| 10 | 1,763 |
| 11 | 165 |
| 12 | 264 |

(continued)

| Compound Number | $IC_{50}$ (nM) |
|---|---|
| 14 | 108 |
| 15 | 342 |
| 17 | 540 |
| 18 | 335 |
| 19 | 222 |

The compounds in the embodiments of the present invention have an inhibitory effect on cell proliferation, with preferred $IC_{50}$ of less than 1,000 nM.

Determination of the blockade on hERG potassium channel

The effect of the compound of the present invention on possible arrhythmia is evaluated by determining the blockade of hERG potassium channel.

The experimental method is outlined as follows:

Extracellular solution: 140 mM NaCl, 3.5 mM KCl, 1 mM $MgCl_2$, 2 mM $CaCl_2$, 10 mM D-glucose, 10 mM HEPES, and 1.25 mM $NaH_2PO_4$, pH = 7.4.

Electrode solution: 20 mM KCl, 115 mM K-aspartate, 1 mM $MgCl_2$, 5 mM EGTA, 10 mM HEPES, and 2 mM $Na_2$-ATP, pH = 7.2.

Compound solution: a test compound was dissolved in DMSO as a 10 mM stock solution, which was diluted with DMSO to 3 mM and then with the extracellular solution to a 3 μM solution for subsequent use.

Cell culture: HEK293 cell line stably expressing hERG potassium channel (Creacell, Cat. No. A-0320) was cultured in Dulbecco's Modified Eagle Medium (DMEM, Gibco, Cat. No. 11995-065) supplemented with 10% fetal bovine serum (Gibco, Cat. No. 1428478) and 0.8 mg/mL G418 (Amresco, Cat. No. E859-5G), where the culture temperature was 37°C, and the carbon dioxide concentration was 5%. The used medium was removed and the cell line was washed once with PBS (Gibco, Cat. No. 1009-141). 1 mL of TrypLE™ Express (Gibco, Cat. No. 12604021) was added, and the cell line was incubated at 37°C for 30 s. When the cells were detached from the bottom of the cell culture dish, 5 mL of Complete Medium preheated at 37°C was added, and the cell suspension was transferred into a sterile centrifuge tube and centrifuged at 1,000 rpm for 5 min to collect cells. The cells were seeded in 6 cm cell culture dishes where each was seeded with $2.5*10^5$ cells (the final volume: 5 mL). Before patch-clamp experiment, $3*10^3$ cells were spread on a coverslip, cultured on a 24-well plate (final volume: 500 μL), and detected after 18 h.

A voltage stimulation way for recording whole-cell hERG potassium current by the whole-cell patch clamp was as following: the cell membrane was clamped at a voltage of -80 mV after a whole-cell seal was formed. The clamp voltage was depolarized from -80 mV to -50 mV and held for 0.5 s (as leakage current detection), stepped to 30 mV and held for 2.5 s, and rapidly restored to -50 mV and held for 4 s to excite the peak tail current of the hERG channel, in which the hERG potassium current was recorded every 10 s. The experimental data was acquired by EPC-10 USB Patch Clamp Amplifier (HEKA) and stored in PatchMaster (HEKA v2x73) software.

Measurement: A glass capillary tube (Sutter Instruments) was made into a recording electrode using a microelectrode puller (Sutter Instruments). A coverslip with cells taken from the 24-well plate placed in the incubator was placed under an inverted microscope. The electrode solution was poured into the recording electrode, and the microelectrode puller (Sutter Instruments) was manipulated to let the recording electrode contact with the surface of cells and provide negative pressure suction to form a GΩ seal. A rapid capacitance compensation was provided to give negative pressure suction continuously until the cell membrane was broken, forming a whole-cell recording mode. In the whole-cell recording mode, slow capacitance compensation was conducted, while membrane capacitance and series resistance were recorded, during which compensation of leakage current was not provided. When the hERG peak tail current in the whole-cell recording was stable for 3-5 min, 8 mL of compound-free extracellular solution (blank control) and 8 mL of 3 μM test compound solution were perfused under gravity and flowed through the recording bath successively to act on the cells for 5 min (or until the current was stable). The current of each cell measured in the compound-free extracellular solution was used as its own control group. Independently, 2 to 3 cells were measured repeatedly. All electrophysiological experiments were conducted at room temperature.

Data analysis: First of all, the current after action of the test compound and the current of the blank control were

normalized $(\frac{peak\ tail\ current\ compound}{peak\ tail\ current\ vehicle})$, and the inhibition percentage corresponding to the concentration of the compound ($1 - \frac{peak\ tail\ current\ compound}{peak\ tail\ current\ vehicle}$) was calculated.

| Compound Number | hERG current inhibition percentage at 3 μM solubility (*n* = 2) |
|---|---|
| 7 | 10% |
| 8 | 45% |
| 9 | 65% |
| 11 | -0.5% |
| 12 | 21% |
| 14 | -4% |
| 15 | 1% |

## Claims

**1.** A compound represented by general formula (I):

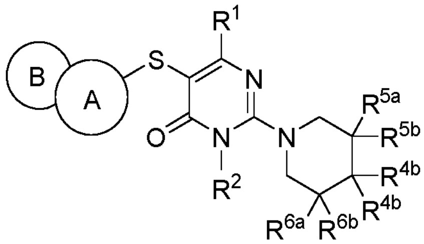

(I)

or pharmaceutically acceptable salts, prodrugs, stable isotope derivatives, isomers thereof, or mixtures thereof, wherein:

ring A is selected from the group consisting of phenyl ringor 6-membered heteroaryl ring, ring B is a 5-membered heteroaryl ring fused to ring A, wherein the phenyl ringand the heteroaryl ringare optionally substituted with one or more substituents selected from the group consisting of D, halogen, cyano, oxo, $C_{1-6}$ alkyl, $C_{3-6}$ cycloalkyl, 4- to 7-membered heterocyclyl, 5- to 6-membered heteroaryl, $-OR^a$, $-NR^aR^b$, $-C(O)R^a$, $-C(O)NR^aR^b$, $-S(O)_2R^a$, $-S(O)_2NR^aR^b$, $-NR^aS(O)_2R^b$, and $-P(O)(CH_3)_2$, wherein the alkyl, the cycloalkyl, the heterocyclyl, and the heteroaryl are optionally substituted with one or more substituents selected from the group consisting of D, halogen, cyano, oxo, $C_{1-6}$ alkyl, $C_{3-6}$ cycloalkyl, 4- to 7-membered heterocyclyl, $-OR^a$, $-NR^aR^b$, $-C(O)R^a$, and $-C(O)NR^aR^b$;

$R^1$ is selected from the group consisting of H, D, cyano, $C_{1-2}$ alkyl, cyclopropyl, $-OR^a$, $-NR^aR^b$, and $-C(O)NR^aR^b$, wherein one or more hydrogen atoms of the alkyl and the cyclopropyl are optionally substituted with one or more substituents selected from the group consisting of D and fluoro;

$R^2$ is selected from the group consisting of H, $C_{1-2}$ alkyl, and cyclopropyl, wherein one or more hydrogen atoms of the alkyl and the cyclopropyl are optionally substituted with one or more substituents selected from the group consisting of D, fluoro, and hydroxyl;

$R^{4a}$ and $R^{4b}$ are each independently selected from the group consisting of H, D, halogen, cyano, $-OR^a$, $-NR^aR^b$, $-C(O)R^a$, $-C(O)NR^aR^b$, $C_{1-6}$ alkyl, $C_{3-6}$ cycloalkyl, 4- to 7-membered heterocyclyl, and 5- to 6-membered heteroaryl, with the proviso that the $R^{4a}$ and the $R^{4b}$ cannot be simultaneously selected from the group consisting of cyano, $-OR^a$, and $-NR^aR^b$, wherein the alkyl, the cycloalkyl, the heterocyclyl, and the heteroaryl are optionally substituted with one or more substituents selected from the group consisting of D, halogen, cyano, oxo, $C_{1-6}$ alkyl, $C_{3-6}$ cycloalkyl, 4- to 7-membered heterocyclyl, 5- to 6-membered heteroaryl, $-OR^a$, $-NR^aR^b$, $-C(O)R^a$, and $-C(O)NR^aR^b$; the $R^{4a}$ and the $R^{4b}$ optionally together with the carbon atom to which the $R^{4a}$ or the $R^{4b}$ are

attached form a $C_{3-7}$ carbocyclic ring or 4- to 8-membered heterocyclic ring;
$R^{5a}$, $R^{5b}$, $R^{6a}$, and $R^{6b}$ are each independently selected from the group consisting of H, D, fluoro, and methyl;
$R^a$ and $R^b$ are each independently selected from the group consisting of H, $C_{1-6}$ alkyl, $C_{3-6}$ cycloalkyl, and 4- to 7-membered heterocyclyl, wherein the alkyl, the cycloalkyl, and the heterocyclyl are optionally substituted with one or more substituents selected from the group consisting of D, fluoro, cyano, oxo, hydroxyl, $-OCH_3$, and $-NH_2$.

**2.** A compound according to claim 1 or pharmaceutically acceptable salts, prodrugs, stable isotope derivatives, isomers thereof, mixtures thereof, represented by general formula (II) shown below:

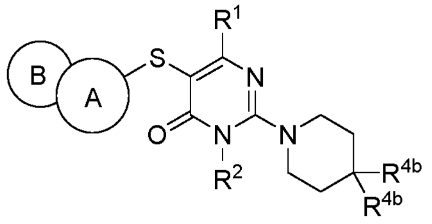

(II)

wherein:

ring A is selected from the group consisting of phenyl ringand 6-membered heteroaryl ring, ring B is selected from the group consisting of a 5-membered heteroaryl ring fused to ring A, wherein the phenyl ringand the heteroaryl ringare optionally substituted with one or more substituents selected from the group consisting of D, halogen, cyano, oxo, $C_{1-6}$ alkyl, $C_{3-6}$ cycloalkyl, 4- to 7-membered heterocyclyl, 5- to 6-membered heteroaryl, $-OR^a$, $-NR^aR^b$, $-C(O)R^a$, $-C(O)NR^aR^b$, $-S(O)_2R^a$, $-S(O)_2NR^aR^b$, and $-NR^aS(O)_2R^b$, wherein the alkyl, the cycloalkyl, the heterocyclyl, and the heteroaryl are optionally substituted with one or more substituents selected from the group consisting of D, halogen, cyano, oxo, $C_{1-6}$ alkyl, $C_{3-6}$ cycloalkyl, 4- to 7-membered heterocyclyl, $-OR^a$, $-NR^aR^b$, $-C(O)R^a$, and $-C(O)NR^aR^b$;
$R^1$ is selected from the group consisting of H, D, cyano, $C_{1-2}$ alkyl, $-OR^a$, and $-NR^aR^b$, wherein one or more hydrogen atoms of the alkyl are optionally substituted with one or more substituents selected from the group consisting of D and fluoro;
$R^2$ is selected from the group consisting of H and $C_{1-2}$ alkyl, wherein one or more hydrogen atoms of the alkyl are optionally substituted with one or more substituents selected from the group consisting of D and fluoro;
$R^{4a}$ and $R^{4b}$ are each independently selected from the group consisting of H, D, halogen, cyano, $-OR^a$, $-NR^aR^b$, $-C(O)NR^aR^b$, $C_{1-6}$ alkyl, $C_{3-6}$ cycloalkyl, 4- to 7-membered heterocyclyl, and 5- to 6-membered heteroaryl, with the proviso that the $R^{4a}$ and the $R^{4b}$ cannot be simultaneously selected from the group consisting of cyano, $-OR^a$, and $-NR^aR^b$, wherein the alkyl, the cycloalkyl, the heterocyclyl, and the heteroaryl are optionally substituted with one or more substituents selected from the group consisting of D, halogen, cyano, oxo, $C_{1-6}$ alkyl, $C_{3-6}$ cycloalkyl, 4- to 7-membered heterocyclyl, 5- to 6-membered heteroaryl, $-OR^a$, $-NR^aR^b$, $-C(O)R^a$, and $-C(O)NR^aR^b$; the $R^{4a}$ and the $R^{4b}$ optionally together with the carbon atom to which the $R^{4a}$ and the $R^{4b}$ are attached form a $C_{3-7}$ carbocyclic ring or 4- to 7-membered heterocyclic ring;
$R^a$ and $R^b$ are each independently selected from the group consisting of H, $C_{1-6}$ alkyl, $C_{3-6}$ cycloalkyl, and 4- to 7-membered heterocyclyl, wherein the alkyl, the cycloalkyl, and the heterocyclyl are optionally substituted with one or more substituents selected from the group consisting of D, fluoro, cyano, oxo, hydroxyl, $-OCH_3$, and $-NH_2$.

**3.** A compound according to claim 1 or 2 or pharmaceutically acceptable salts, prodrugs, stable isotope derivatives, isomers thereof, mixtures thereof, represented by general formula (III) shown below:

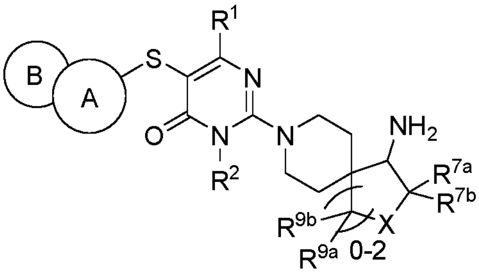

(III)

wherein:

ring A is selected from the group consisting of phenyl ring and 6-membered heteroaryl ring, ring B is selected from the group consisting of a 5-membered heteroaryl ring fused to ring A, wherein phenyl and the heteroaryl are optionally substituted with one or more substituents selected from the group consisting of D, halogen, cyano, oxo, $C_{1-6}$ alkyl, $C_{3-6}$ cycloalkyl, 4- to 7-membered heterocyclyl, 5- to 6-membered heteroaryl, $-OR^a$, $-NR^aR^b$, $-C(O)R^a$, and $-C(O)NR^aR^b$, wherein the alkyl, the cycloalkyl, the heterocyclyl, and the heteroaryl are optionally substituted with one or more substituents selected from the group consisting of D, halogen, cyano, oxo, $C_{1-2}$ alkyl, $-OR^a$, and $-NR^aR^b$;

X is selected from the group consisting of -O- and $-CR^{8a}R^{8b}-$;

$R^1$ is selected from the group consisting of H, D, cyano, $C_{1-2}$ alkyl, and $-NR^aR^b$, wherein one or more hydrogen atoms of the alkyl are optionally substituted with one or more substituents selected from the group consisting of D and fluoro;

$R^2$ is selected from the group consisting of H and $C_{1-2}$ alkyl, wherein one or more hydrogen atoms of the alkyl are optionally substituted with one or more substituents selected from the group consisting of D and fluoro;

$R^{7a}$, $R^{7b}$, $R^{8a}$, $R^{8b}$, $R^{9a}$, and $R^{9b}$ are each independently selected from the group consisting of H, D, halogen, cyano, $C_{1-2}$ alkyl, and $-OR^c$, wherein one or more hydrogen atoms of the alkyl are optionally substituted with one or more substituents selected from the group consisting of D, fluoro, and hydroxyl, with the proviso that the $R^{7a}$ and $R^{7a}$, the $R^{8a}$ and $R^{8b}$, and the $R^{9a}$ and $R^{9b}$ optionally cannot be simultaneously selected from the group consisting of $-OR^c$;

$R^a$ and $R^b$ are each independently selected from the group consisting of H, $C_{1-6}$ alkyl, $C_{3-6}$ cycloalkyl, and 4- to 7-membered heterocyclyl, wherein the alkyl, the cycloalkyl, and the heterocyclyl are optionally substituted with one or more substituents selected from the group consisting of D, fluoro, cyano, oxo, hydroxyl, $-OCH_3$, and $-NH_2$;

$R^c$ is selected from the group consisting of H and $C_{1-2}$ alkyl, wherein one or more hydrogen atoms of the alkyl are optionally substituted with one or more substituents selected from the group consisting of D and fluoro.

**4.** A compound according to any one of the preceding claims or pharmaceutically acceptable salts, prodrugs, stable isotope derivatives, isomers thereof, and mixtures thereof, represented by general formula (IV) shown below:

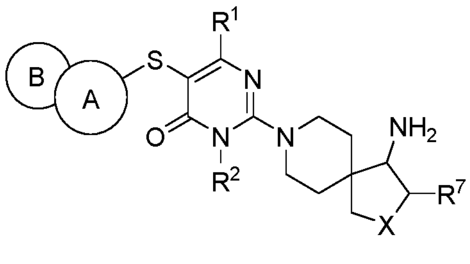

(IV)

wherein:

ring A is selected from the group consisting of phenyl ringand 6-membered heteroaryl ring, ring B is selected from the group consisting ofa 5-membered heteroaryl ring fused to ring A, wherein the phenyl and the heteroaryl are optionally substituted with one or more substituents selected from the group consisting of D, halogen, cyano, oxo, $C_{1-2}$ alkyl, $-OR^a$, and $-NR^aR^b$, wherein one or more hydrogen atoms of the alkyl are optionally substituted with one or more substituents selected from the group consisting of D and fluoro;

X is selected from the group consisting of -O- and $-CH_2-$;

$R^1$ is selected from the group consisting of H, D, $C_{1-2}$ alkyl, and $-NR^aR^b$, wherein one or more hydrogen atoms of the alkyl are optionally substituted with one or more substituents selected from the group consisting of D and fluoro;

$R^2$ is selected from the group consisting of H and $C_{1-2}$ alkyl, wherein one or more hydrogen atoms of the alkyl are optionally substituted with one or more substituents selected from the group consisting of D and fluoro;

$R^7$ is selected from the group consisting of H, D, and $C_{1-2}$ alkyl, wherein one or more hydrogen atoms of the alkyl are optionally substituted with one or more substituents selected from the group consisting of D and fluoro;

$R^a$ and $R^b$ are each independently selected from the group consisting of H and $C_{1-2}$ alkyl, wherein one or more hydrogen atoms of the alkyl are optionally substituted with one or more substituents selected from the group consisting of D and fluoro.

**5.** A compound according to any one of the preceding claims or pharmaceutically acceptable salts, prodrugs, stable isotope derivatives, isomers thereof, and mixtures thereof, wherein the compound is selected from the group consisting of:

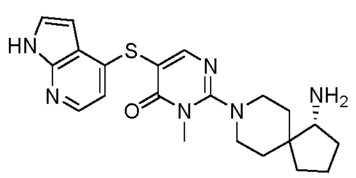,
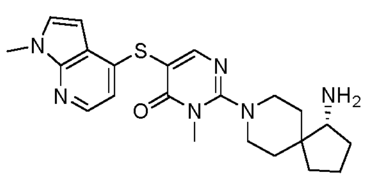,
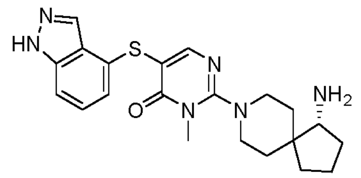,

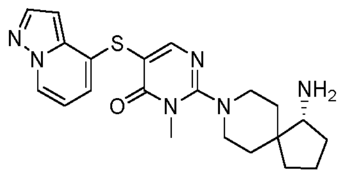,
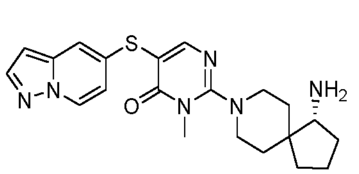,
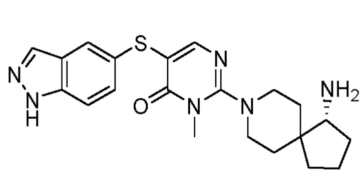,

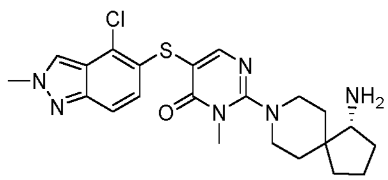,
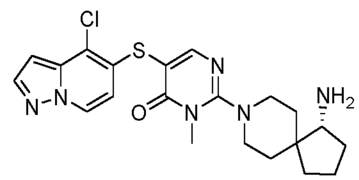,
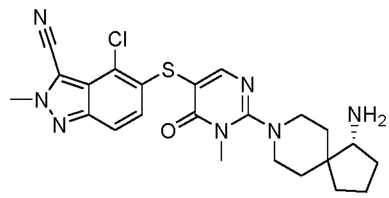,

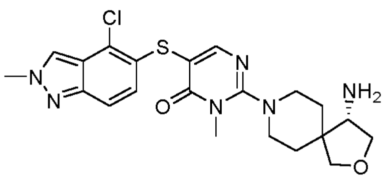,
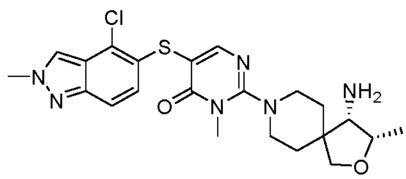,
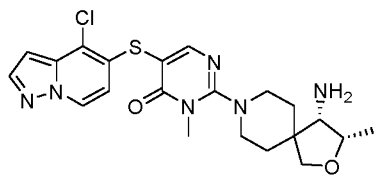,

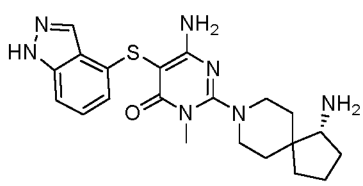,
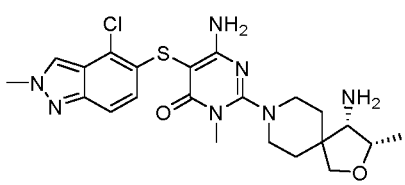,
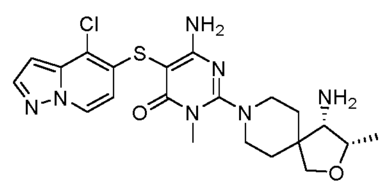,

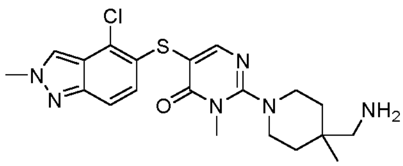, 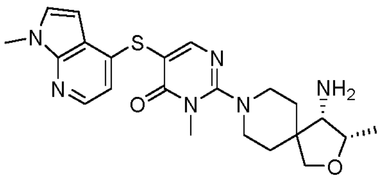, 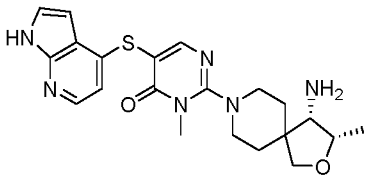,

and

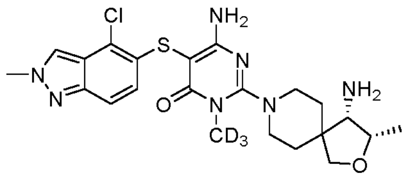.

**6.** A pharmaceutical composition, comprising the compound according to any one of claims 1 to 5 or pharmaceutically acceptable salt, prodrug, stable isotope derivative, and isomer thereof, and mixture thereof, and one or more pharmaceutically acceptable carriers and excipients.

**7.** A pharmaceutical composition, comprising the compound according to any one of claims 1 to 5 or pharmaceutically acceptable salt, prodrug, stable isotope derivative, and isomer thereof, and mixtures thereof, and at least one additional drug, wherein the drug includes but is not limited to chemotherapeutic agents, targeted drugs, DNA synthesis inhibitors, antibody drugs, antibody-drug conjugates, antitumor drugs, immunosuppressants, and the like.

**8.** Use of the compound according to any one of claims 1 to 5 or pharmaceutically acceptable salt, prodrug, stable isotope derivative, and isomer thereof, and mixture thereof, or the pharmaceutical composition according to claims 6 to 7 in the preparation of a medicament for treating and/or preventing diseases associated with abnormal SHP2 activity, wherein the diseases include but are not limited to cancers, comprising leukemia, Noonan syndrome, leopard syndrome, neuroblastoma, melanoma, lung cancer, breast cancer, esophageal cancer, colon cancer, head and neck cancer, and gastric cancer.

**9.** A method for treating and/or preventing SHP2-associated diseases, wherein the method comprises a therapeutically effective dosage of the compound according to any one of claims 1 to 7 or prodrug, stable isotope derivative, pharmaceutically acceptable salt, and isomer thereof, and mixture thereof, or pharmaceutical composition thereof.

**INTERNATIONAL SEARCH REPORT**

International application No.
**PCT/CN2021/071375**

**A. CLASSIFICATION OF SUBJECT MATTER**

C07D 471/04(2006.01)i; C07D 471/10(2006.01)i; C07D 401/14(2006.01)i; A61K 31/506(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D471/-; C07D401/-; A61K31/-; A61P35/-

Documentation searched other than minimum documentation to the extent that such documents are included in the fields searched

Electronic data base consulted during the international search (name of data base and, where practicable, search terms used)

CNKI, CNABS, SIPOABS, DWPI, REGISTRY, CAPLUS (STN), 北京诺诚健华医药科技有限公司, 嘧啶, 吡咯, 吡啶, 吲唑, 吡唑, 癌症, 肿瘤, SHP2, pyrimidin+, pyrrol+, pyrryl+, pyridyl+, indazol+, pyrazolyl+, cancer, tumor, structural search according to compound of claim 1

**C. DOCUMENTS CONSIDERED TO BE RELEVANT**

| Category* | Citation of document, with indication, where appropriate, of the relevant passages | Relevant to claim No. |
|---|---|---|
| Y | CN 107787323 A (NOVARTIS AG) 09 March 2018 (2018-03-09)<br>description, embodiments 1-122, compound, and paragraphs [0016] and [0019] | 1-9 |
| Y | CN 109311848 A (JACOBIO PHARMACEUTICALS GROUP CO., LTD.) 05 February 2019 (2019-02-05)<br>claims 1, 39 and 72-79, and description, embodiments 1-76, compound and paragraph [0211] | 1-9 |
| Y | WO 2018130928 A1 (NOVARTIS AG et al.) 19 July 2018 (2018-07-19)<br>claim 1, and description, pages 36-43, table 1, compound | 1-9 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 April 2021** | **19 April 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/071375**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **9**
because they relate to subject matter not required to be searched by this Authority, namely:

[1] Claim 9 relates to a method of treating and/or preventing SHP2-related diseases, thus belonging to a method for the treatment of the human or animal body (PCT Rule 39.1(iv)). Nevertheless, the search is conducted on the technical subject of claim 9 on the basis of the use of the compound or the pharmaceutical composition in the preparation of a medicament for the treatment and/or prevention of SHP2-related diseases.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/071375**

| Patent document cited in search report | Publication date (day/month/year) | Patent family member(s) | Publication date (day/month/year) |
|---|---|---|---|
| CN 107787323 A | 09 March 2018 | JP 2018517746 A | 05 July 2018 |
| | | CN 107787323 B | 01 September 2020 |
| | | US 10308660 B2 | 04 June 2019 |
| | | EP 3310771 B1 | 22 July 2020 |
| | | WO 2016203405 A1 | 22 December 2016 |
| | | US 2018251471 A1 | 06 September 2018 |
| | | EP 3310771 A1 | 25 April 2018 |
| CN 109311848 A | 05 February 2019 | WO 2017211303 A1 | 14 December 2017 |
| | | SG 11201810983 P A | 30 January 2019 |
| | | PH 12018550202 A1 | 21 October 2019 |
| | | JP 6751203 B2 | 02 September 2020 |
| | | KR 20190015756 A | 14 February 2019 |
| | | AU 2017276457 B2 | 03 October 2019 |
| | | CN 112250670 A | 22 January 2021 |
| | | CA 3026784 A1 | 14 December 2017 |
| | | EP 3464272 A4 | 24 April 2019 |
| | | JP 2019521181 A | 25 July 2019 |
| | | JP 2020189868 A | 26 November 2020 |
| | | EA 201990001 A1 | 31 May 2019 |
| | | US 10858359 B2 | 08 December 2020 |
| | | EP 3464272 A1 | 10 April 2019 |
| | | US 2019127378 A1 | 02 May 2019 |
| | | MX 2018015297 A | 12 August 2019 |
| | | AU 2017276457 A1 | 24 January 2019 |
| WO 2018130928 A1 | 19 July 2018 | CA 3048340 A1 | 19 July 2018 |
| | | JP 2020504136 A | 06 February 2020 |
| | | AU 2018207464 A1 | 20 June 2019 |
| | | EP 3568204 A1 | 20 November 2019 |
| | | IL 267617 D0 | 29 August 2019 |
| | | US 2019343836 A1 | 14 November 2019 |
| | | AU 2018207464 B2 | 14 May 2020 |
| | | CN 110730678 A | 24 January 2020 |
| | | KR 20190104530 A | 10 September 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015107495 A
- WO 2016203405 A
- WO 2018057884 A
- WO 2018013597 A
- WO 2017211303 A


### Non-patent literature cited in the description


- **RUESS DA et al.** *Nat. Med.,* 2018, vol. 24, 954-960
- **LI J et al.** *Cancer Res.,* 2015, vol. 75, 508-518
- **CHEN Y et al.** *Nature,* 2016, vol. 535, 148-52